# EUROPÄISCHE PATENTSCHRIFT

(11) **EP 2 238 150 B1**
(45) Veröffentlichungstag und Bekanntmachung des Hinweises auf die Patenterteilung: **24.10.2012**
(21) Anmeldenummer: 08868567.2
(22) Anmeldetag: 23.12.2008
(51) Int. Cl.: C07J 53/00, A61K 31/58, A61P 5/34, A61P 5/42, A61P 5/28

(54) **15,16-METHYLEN-17-HYDROXY-19-NOR-21-CARBONSÄURE-STEROID y-LACTON-DERIVAT, DESSEN VERWENDUNG UND DAS DERIVAT ENTHALTENDE ARZNEIMITTEL**
15,16-METHYLENE-17-HYDROXY-19-NOR-21-CARBOXYLIC ACID y-LACTONE DERIVATIVE, USE THEREOF, AND MEDICAMENT CONTAINING SAID DERIVATIVE
DÉRIVÉ DE STÉROÏDE y-LACTONE DE L'ACIDE 15,16-MÉTHYLÈNE-17-HYDROXY-19-NOR-21-CARBOXYLIQUE, SON UTILISATION ET MÉDICAMENTS CONTENANT CE DÉRIVÉ

(30) Priorität: 29.12.2007 DE 102007063501
(43) Veröffentlichungstag der Anmeldung: 13.10.2010
(73) Patentinhaber: Bayer Intellectual Property GmbH, 40789 Monheim (DE)
(72) Erfinder: KLAR, Ulrich, 13503 Berlin (DE); KUHNKE, Joachim, 14482 Potsdam (DE); BOHLMANN, Rolf, 14055 Berlin (DE); HÜBNER, Jan, 10317 Berlin (DE); RING, Sven, 07749 Jena (DE); FRENZEL, Thomas, 65719 Hofheim (DE); MENGES, Frederik, 69198 Schriesheim (DE); BORDEN, Steffen, 13355 Berlin (DE); MUHN, Hans, Peter, 13465 Berlin (DE); PRELLE, Katja, 13465 Berlin (DE)
(74) Vertreter: Bayer Intellectual Property GmbH
(86) Internationale Anmeldenummer: PCT/EP2008/011162
(87) Internationale Veröffentlichungsnummer: WO 2009/083269

(56) Entgegenhaltungen:
- EP-A- 0 156 284
- WO-A-2006/072467
- NICKISCH K ET AL: "Aldosterone antagonists. 4. Synthesis and activities of steroidal 6,6-ethylene-15,16-methylene 17-spirolactones" JOURNAL OF MEDICINAL CHEMISTRY, AMERICAN CHEMICAL SOCIETY, WASHINGTON, US, Bd. 34, 1. Januar 1991 (1991-01-01), Seiten 2464-2468, XP002375413 ISSN: 0022-2623

## Beschreibung

Die Erfindung betrifft 15,16-Methylen-17-hydroxy-19-nor-carbonsäure-Steroid γ-Lacton-Derivate mit α,β-ungesättigtem-17,17-Spirolacton, deren Verwendung sowie die Derivate enthaltende Arzneimittel mit gestagener Wirkung, beispielsweise zur Behandlung von prä-, peri- und postmenopausalen sowie von prämenstruellen Beschwerden.

Aus der Literatur sind Verbindungen mit gestagener, antimineralcorticoider, antiandrogener oder antiestrogener Wirkung auf Basis eines Steroidgerüstes bekannt, welche beispielsweise von 19-Nor-androst-4-en-3-on oder einem Derivat davon abgeleitet sind (die Nummerierung des Steroidgerüstes ist beispielsweise Fresenius/Görlitzer 3.Aufl. 1991 "Organisch-chemische Nomenklatur" S. 60 ff. zu entnehmen).

So offenbart WO 2006072467 A1 die als Gestagen wirkende Verbindung 6β,7β;15β,16β-Dimethylen-3-oxo-17-pregn-4-en-21,17β-carbolacton (Drospirenon), welche beispielsweise in einem oralen Kontrazeptivum sowie einem Präparat zur Behandlung postmenopausaler Beschwerden verwendet wurde. Aufgrund seiner vergleichsweise geringen Affinität zum Gestagenrezeptor und seiner vergleichsweise hohen Ovulationshemmdosis ist Drospirenon in dem Kontrazeptivum jedoch in der relativ hohen täglichen Dosis von 3 mg enthalten. Drospirenon zeichnet sich darüber hinaus auch dadurch aus, dass es zusätzlich zur gestagenen Wirkung über aldosteronantagonistische (antimineralcorticoide) sowie antiandrogene Wirkung verfügt. Diese beiden Eigenschaften machen Drospirenon in seinem pharmakologischen Profil dem natürlichen Gestagen Progesteron sehr ähnlich, welches aber anders als Drospirenon nicht ausreichend oral bioverfügbar ist. Um die zu verabreichende Dosis zu senken, werden in WO 2006072467 A1 weiter ein 18-Methyl-19-nor-17-pregn-4-en-21,17-carbolacton sowie diese enthaltende pharmazeutische Präparate vorgeschlagen, welche über eine höhere gestagene Potenz als Drospirenon verfügen.

Daneben offenbart beispielsweise US-A 3,705,179 Steroide, welche eine antiandrogene Aktivität aufweisen und sich zur Behandlung von Krankheiten eignen, die im Zusammenhang mit Androgenen stehen.

Weiterhin werden in US-Patent Nr. 2,918,463 17-carboxyalkylierte 17-Hydroxy-19-nor-androsten-3-one offenbart, u.a.17α-(2-carboxyvinyl)-17β-hydroxy-19-nor-androst-4-en-3-on Lacton. Die beschriebenen Verbindungen sollen die Wirkung von Desoxycorticosteronacetat auf den Gehalt von Natrium und Kalium im Urin blockieren und gleichzeitig in höherer Konzentration eine salzbindende Wirkung haben. Außerdem sollen diese Verbindungen auch gegenüber Bluthochdruck wirksam sein.

Die Aufgabe der vorliegenden Erfindung ist es, Verbindungen zur Verfügung zu stellen, die über eine starke Bindung an den Gestagenrezeptor verfügen. Außerdem sollen die Verbindungen bevorzugt auch eine antimineralcorticoide Wirkung sowie eine im Hinblick auf den Androgenrezeptor neutrale bis leicht androgene Wirkung aufweisen. Ein weiteres wesentliches Ziel der vorliegenden Erfindung besteht auch darin, ein ausgewogenes Wirkungsprofil hinsichtlich der gestagenen Wirkung zur antimineralcorticoiden Wirkung dergestalt zu erreichen, dass das Verhältnis der gestagenen zur antimineralcorticoiden Wirkung geringer ist als bei Drospirenon.

Diese Aufgabe wird durch die erfindungsgemäßen 15,16-Methylen-17-hydroxy-19-norcarbonsäure-Steroid γ-Lacton-Derivate gemäß Anspruch 1, die Verwendung der erfindungsgemäßen Derivate gemäß Anspruch 12 sowie ein mindestens ein erfindungsgemäßes Derivat enthaltendes Arzneimittel gemäß Anspruch 14 gelöst. Vorteilhafte Ausführungsformen der Erfindung sind in den Unteransprüchen angegeben.

Die vorliegende Erfindung betrifft demnach 15,16-Methylen-17-hydroxy-19-norcarbonsäure-Steroid γ-Lacton-Derivate mit der allgemeinen chemischen Formel I, 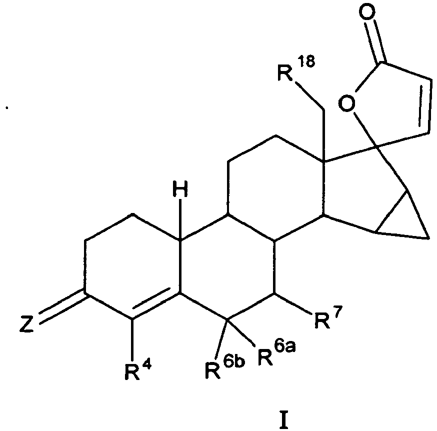 worin
- Z: ausgewählt ist aus der Gruppe, umfassend Sauerstoff, zwei Wasserstoffatome, NOR' und NNHSO₂R', worin R' Wasserstoff, C₁-C₁₀-Alkyl, Aryl oder C₇-C₂₀-Aralkyl ist,
- R⁴: ausgewählt ist aus der Gruppe, umfassend Wasserstoff Fluor, Chlor oder Brom
ferner entweder:
- R^{6a}, R^{6b}: jeweils unabhängig voneinander ausgewählt sind aus der Gruppe, umfassend Wasserstoff, C₁-C₁₀-Alkyl, C₂-C₁₀-Alkenyl und C₂-C₁₀-Alkinyl, oder gemeinsam Methylen oder 1,2-Ethandiyl bilden und
- R⁷: ausgewählt ist aus der Gruppe, umfassend Wasserstoff, C₁-C₁₀-Alkyl, C₃-C₆-Cycloalkyl, C₂-C₁₀-Alkenyl und C₂-C₁₀-Alkinyl,
oder:
- R^{6a}, R⁷: gemeinsam ein Sauerstoffatom oder Methylen bilden oder unter Bildung einer Doppelbindung zwischen C⁶ und C⁷ entfallen und
- R^{6b}: ausgewählt ist aus der Gruppe, umfassend Wasserstoff, C₁-C₁₀-Alkyl, C₂-C₁₀-Alkenyl und C₂-C₁₀-Alkinyl und
- R¹⁸: Wasserstoff oder C₁-C₃-Alkyl ist,
sowie deren Solvate, Hydrate, Stereoisomere und Salze.

Die Nummerierung des C-Gerüstes des erfindungsgemäßen Derivates mit der allgemeinen chemischen Formel I folgt in üblicher Weise der Nummerierung eines Steroidgerüstes, beispielsweise beschrieben in Fresenius, a.a.O. Die Nummerierung der in den Ansprüchen angegebenen Reste entspricht in analoger Weise ihrer Bindungsposition am C-Gerüst des Derivates, soweit dies R⁴, R⁶, R⁷ und R¹⁸ betrifft. So bindet beispielsweise der Rest R⁴ an die C⁴-Position des erfindungsgemäßen Derivates.

Hinsichtlich der zu Z definierten Gruppen binden die Gruppen NOR' und NNHSO₂R' jeweils mit einer Doppelbindung über N an das C-Gerüst des Derivates gemäß =NOR' bzw. =NNH-SO₂R'. OR' in NOR' und NHSO₂R' in NNHSO₂R' können syn- oder antiständig stehen.

Unter Alkyl in R', R^{6a}, R^{6b} und R⁷ sowie in R¹⁹, R²⁰, R^{21a}, R^{21b} und R²² in den weiter unten angegebenen allgemeinen chemischen Formeln sind gerad- oder verzweigtkettige Alkylgruppen mit 1-10 Kohlenstoffatomen zu verstehen, wie beispielsweise Methyl, Ethyl, Propyl, Isopropyl, Butyl, Isobutyl, tert.-Butyl, Pentyl, Isopentyl, Neopentyl, Heptyl, Hexyl, Decyl. Unter Alkyl in R¹⁸ ist Methyl, Ethyl, Propyl oder Isopropyl zu verstehen.

Insbesondere kann Alkyl daher auch für Hydroxymethylen (HO-CH₂), Hydroxyethylen (HO-C₂H₄), Hydroxypropylen (HO-C₃H₆) und Hydroxybutylen (HO-C₄H₈) sowie deren Isomere stehen.

Unter Alkenyl in R^{6a}, R^{6b} und R⁷ sind gerad- oder verzweigtkettige Alkenylgruppen mit 2-10 Kohlenstoffatomen zu verstehen, wie beispielsweise Vinyl, Propenyl, Butenyl, Pentenyl, Isobutenyl, Isopentenyl.

Unter Alkinyl in R^{6a}, R^{6b} und R⁷ sind gerad- oder verzweigtkettige Alkinylgruppen mit 2-10 Kohlenstoffatomen zu verstehen, wie beispielsweise Ethinyl, Propinyl, Butinyl, Pentinyl, Isobutinyl, Isopentinyl.

Unter Cycloalkyl in R⁷ sind Cycloalkylgruppen mit 3-6 Kohlenstoffatomen zu verstehen, beispielsweise Cyclopropyl, Cyclobutyl, Cyclopentyl und Cyclohexyl.

Soweit ansonsten Aryl als Substituent an Alkyl, Alkenyl oder Alkinyl erwähnt wird, handelt es sich insbesondere um Arylgruppen mit 6-12 Ringkohlenstoffatomen.

Als Aralkylreste kommen beispielsweise Benzyl, Phenylethyl, Naphthylmethyl, Naphthylethyl, Furylmethyl, Thienylethyl, Pyridylpropyl in Betracht.

Soweit Alkoxy (O-Alkyl) als Substituent an Alkyl erwähnt wird, handelt es sich um Alkoxygruppen mit 1-4 Kohlenstoffatomen, und, soweit Alkoxy als Substituent an Alkenyl und Alkinyl erwähnt wird, handelt es sich um Alkoxygruppen mit 1-3 Kohlenstoffatomen. Alkoxy kann insbesondere Methoxy, Ethoxy und Propoxy sein.

Soweit Acyl (CO-Alkyl) als Substituent an Cycloalkyl und Aryl erwähnt wird, handelt es sich um Acylgruppen mit 1-10 Kohlenstoffatomen, und, soweit Acyl als Substituent an Aralkyl erwähnt wird, handelt es sich um Acylgruppen mit 1-20 Kohlenstoffatomen. Acyl kann insbesondere Formyl, Acetyl, Propionyl und Butyryl sein.

Soweit Acyloxy (O-CO-Alkyl) als Substituent an Cycloalkyl und Aryl erwähnt wird, handelt es sich um Acyloxygruppen mit 1-10 Kohlenstoffatomen, und, soweit Acyloxy als Substituent an Aralkyl erwähnt wird, handelt es sich um Acyloxygruppen mit 1-20 Kohlenstoffatomen. Acyloxy kann insbesondere Formyloxy, Acetyloxy, Propionyloxy und Butyryloxy sein.

Halogen bedeutet Fluor, Chlor oder Brom.

Gemäß einer bevorzugten Ausführungsform der Erfindung ist Z ausgewählt aus der Gruppe, umfassend Sauerstoff, NOR' und NNHSO₂R'.

Gemäß einer weiter bevorzugten Ausführungsform der Erfindung steht Z für Sauerstoff.

Gemäß einer weiter bevorzugten Ausführungsform der Erfindung ist R⁴ Wasserstoff oder Chlor.

Gemäß einer weiter bevorzugten Ausführungsform der Erfindung bilden R^{6a}, R^{6b} gemeinsam 1,2-Ethandiyl oder sind jeweils Wasserstoff.

Gemäß einer weiter bevorzugten Ausführungsform der Erfindung ist R⁷ ausgewählt aus der Gruppe, umfassend Wasserstoff, Methyl, Ethyl und Vinyl.

Gemäß einer weiter bevorzugten Ausführungsform der Erfindung bilden R^{6a}, R⁷ gemeinsam Methylen.

Gemäß einer weiter bevorzugten Ausführungsform der Erfindung entfallen R^{6a} und R⁷ unter Bildung einer Doppelbindung zwischen C⁶ und C⁷.

Gemäß einer weiter bevorzugten Ausführungsform der Erfindung ist R¹⁸ Wasserstoff oder Methyl.

Bevorzugt sind Verbindungen mit der chemischen Formel I, worin
- Z: Sauerstoff, eine Gruppe NOR', wobei R' Wasserstoff. C₁-C₆-Alkyl, Aryl oder C₇-C₁₂-Aralkyl ist,
- R⁴: Wasserstoff oder Fluor, Chlor oder Brom ist,
und entweder:
- R^{6a}, R^{6b}: unabhängig voneinander Wasserstoff, C₁-C₆-Alkyl, C₂-C₆-Alkenyl oder C₂-C₆-Alkinyl sind oder gemeinsam Methylen oder 1,2-Ethandiyl bilden und
- R⁷: Wasserstoff, C₁-C₆-Alkyl, C₃-C₆-Cycloalkyl, C₂-C₆-Alkenyl oder C₂-C₆-Alkinyl ist,
oder:
- R^{6a}, R⁷: unter Bildung einer Doppelbindung zwischen C⁶ und C⁷ entfallen oder gemeinsam Methylen bilden und
- R^{6b}: ausgewählt ist aus der Gruppe, umfassend Wasserstoff, C₁-C₆-Alkyl, C₂-C₆-Alkenyl und C₂-C₆-Alkinyl,
- R¹⁸: Wasserstoff, Methyl oder Ethyl ist.
Besonders bevorzugt sind Verbindungen mit der Formel I, worin
- Z: Sauerstoff oder eine Gruppe NOR' ist, wobei R' Wasserstoff oder C₁-C₃-Alkyl ist,
- R⁴: Wasserstoff, Chlor oder Brom ist,
und entweder:
- R^{6a}, R^{6b}: unabhängig voneinander Wasserstoff, C₁-C₃-Alkyl oder C₂-C₄-Alkenyl sind oder gemeinsam Methylen oder 1,2-Ethandiyl bilden und
- R⁷: Wasserstoff, C₁-C₄-Alkyl, C₃-C₄-Cycloalkyl oder C₂-C₄-Alkenyl ist,
oder:
- R^{6a}, R⁷: unter Bildung einer Doppelbindung zwischen C⁶ und C⁷ entfallen oder gemeinsam Methylen bilden und

- R^{6a}, R⁷: unter Bildung einer Doppelbindung zwischen C⁶ und C⁷ entfallen oder gemeinsam Methylen bilden und
- R¹⁸: Wasserstoff oder Methyl ist.

Hiermit werden ausdrücklich alle möglichen Stereoisomere sowie Isomerengemische, einschließlich Racemate, der Verbindung mit der allgemeinen chemischen Formel I ausdrücklich mit einbezogen, wobei auch die Stellung des ungesättigten γ-Lactonringes im erfindungsgemäßen Derivat in zwei isomeren Formen vorkommen kann. Jeder der genannten Substituenten am Steroid-Grundgerüst kann sowohl in einer α- als auch in einer β-Stellung vorliegen. Außerdem können auch die Substituenten am Steroid-Grundgerüst, die eine Doppelbindung enthalten und in denen die Doppelbindung an jedem Kohlenstoffatom mindestens einen Substituenten, der nicht Wasserstoff ist, trägt, sowohl E- als auch Z-konfiguriert vorliegen. An zwei benachbarte Kohlenstoffatome des Gerüstes gebundene Gruppen, beispielsweise ein Sauerstoffatom, Methylen oder 1,2-Ethandiyl werden entweder in α,α-Stellung oder in β,β-Stellung gebunden.

Ausdrücklich mit einbezogen sind auch alle Kristallmodifikationen der Verbindung mit der allgemeinen chemischen Formel I.

Ausdrücklich mit einbezogen sind auch erfindungsgemäße Derivate in Form von Solvaten, insbesondere von Hydraten, wobei die erfindungsgemäßen Verbindungen demgemäß polare Lösungsmittel, insbesondere von Wasser, als Strukturelement des Kristallgitters der erfindungsgemäßen Verbindungen enthalten. Das polare Lösungsmittel, insbesondere Wasser, kann in einem stöchiometrischen oder auch unstöchiometrischen Verhältnis vorliegen. Bei stöchiometrischen Solvaten, Hydraten spricht man auch von Hemi-, (Semi-), Mono-, Sesqui-, Di-, Tri-, Tetra-, Penta-, usw. Solvaten oder Hydraten.

Ist eine saure Funktion enthalten, sind als Salze die physiologisch verträglichen Salze organischer und anorganischer Basen geeignet, wie beispielsweise die gut löslichen Alkali- und Erdalkalisalze, sowie die Salze von N-Methyl-glukamin, D-Methyl-glukamin, Ethyl-glukamin, Lysin, 1,6-Hexadiamin, Ethanolamin, Glukosamin, Sarkosin, Serinol, Tris-hydroxymethyl-aminomethan, Aminopropandiol, Sovak-Base, 1-Amino-2,3,4-butantriol. Ist eine basische Funktion enthalten, sind die physiologisch verträglichen Salze organischer und anorganischer Säuren geeignet, wie von Salzsäure, Schwefelsäure, Phosphorsäure, Citronensäure, Weinsäure u.a.

Es wurde gefunden, dass die erfindungsgemäßen Verbindungen bzw. Derivate eine gute gestagene Wirkung aufweisen. Außerdem interagieren einige interessante erfindungsgemäße Verbindungen mit dem Mineralcorticoidrezeptor und sind in der Lage, eine antagonistische Wirkung zu vermitteln. Ferner weisen die erfindungsgemäßen Verbindungen im Hinblick auf den Androgenrezeptor eine neutrale bis leicht androgene Wirkung auf. Eine weitere Eigenschaft der überwiegenden Anzahl der Verbindungen besteht darin, dass die Bindungen dieser Verbindungen an den Progesteronrezeptor und an den Mineralcorticoidrezeptor relativ zueinander ausgewogen sind, und zwar dergestalt, dass bei ihnen das Verhältnis der Bindungsfähigkeit zum Progesteronrezeptor zur Bindungsfähigkeit zum Mineralcorticoidrezeptor geringer ist als bei Drospirenon. Somit ist die antimineralcorticoide Wirkung dieser Verbindungen bei gegebener gestagener Wirkung geringer als bei Drospirenon. Wird die Dosierung einer gegebenen erfindungsgemäßen Verbindung aufgrund von deren gestagener Wirkung festgelegt, so ist die antimineralcorticoide Wirkung dieser Verbindung bei dieser Dosierung somit geringer als bei Drospirenon.

Die nachstehend genannten Verbindungen sind erfindungsgemäß bevorzugt:

| | |
|---|---|
| 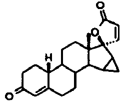 | 17β-Hydroxy-15α,16α-methylen-19-nor-17α-pregna-4,20(Z)-dien-3-on-21-carbonsäure γ-Lacton (Beispiel 12) |
| | 17β-Hydroxy-15β,16β-methylen-19-nor-17α-pregna-4,20(Z)-dien-3-on-21-carbonsäure γ-Lacton (Beispiel 19) |
| | |
| 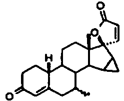 | 17β-Hydroxy-7α-methyl-15α,16α-methylen-19-nor-17α-pregna-4,20(Z)-dien-3-on-21-carbonsäure γ-Lacton (Beispiel 14A) |
| | 17β-Hydroxy-7β-methyl-15α,16α-methylen-19-nor-17α-pregna-4,20(Z)-dien-3-on-21-carbonsäure γ-Lacton (Beispiel 14B) |
| | 17β-Hydroxy-7α-methyl-15β,16β-methylen-19-nor-17α-pregna-4,20(Z)-dien-3-on-21-carbonsäure γ-Lacton (Beispiel 23A) |
| | 17β-Hydroxy-7β-methyl-15β,16β-methylen-19-nor-17α-pregna-4,20(Z)-dien-3-on-21-carbonsäure γ-Lacton (Beispiel 23B) |
| 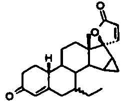 | 17β-Hydroxy-7α-ethyl-15α,16α-methylen-19-nor-17α-pregna-4,20(Z)-dien-3-on-21-carbonsäure γ-Lacton (Beispiel 15A) |
| | 17β-Hydroxy-7β-ethyl-15α,16α-methylen-19-nor-17α-pregna-4,20(Z)-dien-3-on-21-carbonsäure γ-Lacton (Beispiel 15B) |
| | 17β-Hydroxy-7α-ethyl-15β,16β-methylen-19-nor-17α-pregna-4,20(Z)-dien-3-on-21-carbonsäure γ-Lacton (Beispiel 24A) |
| | 17β-Hydroxy-7β-ethyl-15β,16β-methylen-19-nor-17a-pregna-4,20(Z)-dien-3-on-21-carbonsäure γ-Lacton (Beispiel 24B) |
| 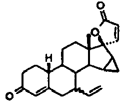 | 17β-Hydroxy-7α-vinyl-15α,16α-methylen-19-nor-17α-pregna-4,20(Z)-dien-3-on-21-carbonsäure γ-Lacton (Beispiel 16A) |
| | 17β-Hydroxy-7β-vinyl-15α,16α-methylen-19-nor-17α-pregna-4,20(Z)-dien-3-on-21-carbonsäure γ-Lacton (Beispiel 16B) |
| | 17β-Hydroxy-7α-vinyl-15β,16β-methylen-19-nor-17α-pregna-4,20(Z)-dien-3-on-21-carbonsäure γ-Lacton (Beispiel 25A) |
| | 17β-Hydroxy-7β-vinyl-15β,16β-methylen-19-nor-17α-pregna-4,20(Z)-dien-3-on-21-carbonsäure γ-Lacton (Beispiel 25B) |
| 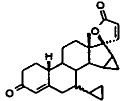 | 17β-Hydroxy-7α-cyclopropyl-15a,16α-methylen-19-nor-17α-pregna-4,20(Z)-dien-3-on-21-carbonsäure γ-Lacton (Beispiel 17A) |
| | 17β-Hydroxy-7β-cyclopropyl-15α,16α-methylen-19-nor-17α-pregna-4,20(Z)-dien-3-on-21-carbonsäure γ-Lacton (Beispiel 17B) |
| | 17β-Hydroxy-7α-cyclopropyl-15β,16β-methylen-19-nor-17α-pregna-4,20(Z)-dien-3-on-21-carbonsäure γ-Lacton (Beispiel 26A) |
| | 17β-Hydroxy-7β-cyclopropyl-15β,16β-methylen-19-nor-17α-pregna-4,20(Z)-dien-3-on-21-carbonsäure γ-Lacton (Beispiel 26B) |
| 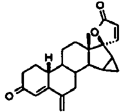 | 17β-Hydroxy-6-methylen-15α,16α-methylen-19-nor-17α-pregna-4,20(Z)-dien-3-on-21-carbonsäure γ-Lacton |
| | 17β-Hydroxy-6-methylen-15β,16β-methylen-19-nor-17α-pregna-4,20(Z)-dien-3-on-21-carbonsäure γ-Lacton |
| 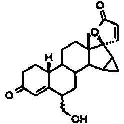 | 17β-Hydroxy-6α-hydroxymethylen-15α,16α-methylen-19-nor-17α-pregna-4,20(Z)-dien-3-on-21-carbonsäure γ-Lacton |
| | 17β-Hydroxy-6β-hydroxymethylen-15α,16α-methylen-19-nor-17α-pregna-4,20(Z)-dien-3-on-21-carbonsäure γ-Lacton |
| | 17β-Hydroxy-6α-hydroxymethylen-15β,16β-methylen-19-nor-17α-pregna- |
| | 4,20(Z)-dien-3-on-21-carbonsäure γ-Lacton |
| | 17β-Hydroxy-6β-hydroxymethylen-15β,16β-methylen-19-nor-17α-pregna-4,20(Z)-dien-3-on-21-carbonsäure γ-Lacton (Beispiel 21) |
| 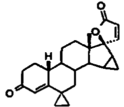 | 6,6-(1,2-Ethandiyl)-17β-hydroxy-15α,16α-methylen-19-nor-17α-pregna-4,20(Z)-dien-3-on-21-carbonsäure γ-Lacton (Beispiel 28) |
| | 6,6-(1,2-Ethandiyl)-17β-hydroxy-15β,16β-methylen-19-nor-17α-pregna-4,20(Z)-dien-3-on-21-carbonsäure γ-Lacton (Beispiel 22) |
| 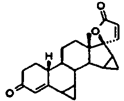 | 17β-Hydroxy-6α,7α;15α,16α-bismethylen-19-nor-17α-pregna-4,20(Z)-dien-3-on-21-carbonsäure γ-Lacton |
| | 17β-Hydroxy-6β,7β;15α,16α-bismethylen-19-nor-17α-pregna-4,20(Z)-dien-3-on-21-carbonsäure γ-Lacton |
| | 17β-Hydroxy-6α,7α;15β,16β-bismethylen-19-nor-17α-pregna-4,20(Z)-dien-3-on-21-carbonsäure γ-Lacton (Beispiel 29B) |
| | 17β-Hydroxy-6β,7β;15β,16β-bismethylen-19-nor-17α-pregna-4,20(Z)-dien-3-on-21-carbonsäure γ-Lacton (Beispiel 29A) |
| 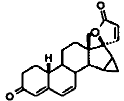 | 17β-Hydroxy-15α,16α-methylen-19-nor-17α-pregna-4,6,20(Z)-trien-3-on-21-carbonsäure γ-Lacton (Beispiel 13) |
| | 17β-Hydroxy-15β,16β-methylen-19-nor-17α-pregna-4,6,20(Z)-trien-3-on-21-carbonsäure γ-Lacton (Beispiel 20) |
| 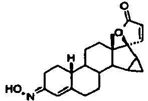 | (E/Z)-3-(Hydroxyimino)-17β-hydroxy-15α,16α-methylen-19-nor-17α-pregna-4,20(Z)-dien-21-carbonsäure γ-Lacton |
| | (E/Z)-3-(Hydroxyimino)-17β-hydroxy-15β,16β-methylen-19-nor-17α-pregna-4,20(Z)-dien-21-carbonsäure γ-Lacton |
| 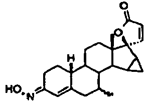 | (E/Z)-3-(Hydroxyimino)-17β-hydroxy-7α-methyl-15α,16α-methylen-19-nor-17α-pregna-4,20(Z)-dien-21-carbonsäure γ-Lacton |
| | (E/Z)-3-(Hydroxyimino)-17β-hydroxy-7β-methyl-15α,16α-methylen-19-nor-17α-pregna-4,20(Z)-dien-21-carbonsäure γ-Lacton |
| | (E/Z)-3-(Hydroxyimino)-17β-hydroxy-7α-methyl-15β,16β-methylen-19-nor-17α-pregna-4,20(Z)-dien-21-carbonsäure γ-Lacton |
| | (E/Z)-3-(Hydroxyimino)-17β-hydroxy-7β-methyl-15β,16β-methylen-19-nor-17α-pregna-4,20(Z)-dien-21-carbonsäure γ-Lacton |
| 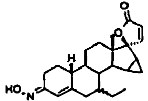 | (E/Z)-3-(Hydroxyimino)-17β-hydroxy-7α-ethyl-15α,16α-methylen-19-nor-17α-pregna-4,20(Z)-dien-21-carbonsäure γ-Lacton |
| | (E/Z)-3-(Hydroxyimino)-17β-hydroxy-7β-ethyl-15α,16α-methylen-19-nor-17α-pregna-4,20(Z)-dien-21-carbonsäure γ-Lacton |
| | (E/Z)-3-(Hydroxyimino)-17β-hydroxy-7α-ethyl-15β,16β-methylen-19-nor-17α-pregna-4,20(Z)-dien-21-carbonsäure γ-Lacton |
| | (E/Z)-3-(Hydroxyimino)-17β-hydroxy-7β-ethyl-15β,16β-methylen-19-nor-17α-pregna-4,20(Z)-dien-21-carbonsäure γ-Lacton |
| 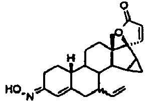 | (E/Z)-3-(Hydroxyimino)-17β-hydroxy-7α-vinyl-15α,16α-methylen-19-nor-17α-pregna-4,20(Z)-dien-21-carbonsäure γ-Lacton |
| | (E/Z)-3-(Hydroxyimino)-17β-hydroxy-7β-vinyl-15α,16α-methylen-19-nor-17α-pregna-4,20(Z)-dien-21-carbonsäure γ-Lacton |
| | (E/Z)-3-(Hydroxyimino)-17β-hydroxy-7α-vinyl-15β,16β-methylen-19-nor-17α-pregna-4,20(Z)-dien-21-carbonsäure γ-Lacton |
| | (E/Z)-3-(Hydroxyimino)-17β-hydroxy-7β-vinyl-15β,16β-methylen-19-nor-17α-pregna-4,20(Z)-dien-21-carbonsäure γ-Lacton |
| 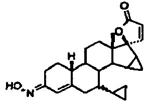 | (E/Z)-3-(Hydroxyimino)-17β-hydroxy-7α-cyclopropyl-15α,16α-methylen-19-nor-17α-pregna-4,20(Z)-dien-21-carbonsäure γ-Lacton |
| | (E/Z)-3-(Hydroxyimino)-17β-hydroxy-7β-cyclopropyl-15α,16α-methylen-19-nor-17α-pregna-4,20(Z)-dien-21-carbonsäure γ-Lacton |
| | (E/Z)-3-(Hydroxyimino)-17β-hydroxy-7α-cyclopropyl-15β,16β-methylen-19-nor-17α-pregna-4,20(Z)-dien-21-carbonsäure γ-Lacton |
| | (E/Z)-3-(Hydroxyimino)-17β-hydroxy-7β-cyclopropyl-15β,16β-methylen-19-nor-17α-pregna-4,20(Z)-dien-21-carbonsäure γ-Lacton |
| 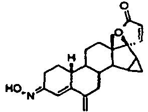 | (E/Z)-3-(Hydroxyimino)-17β-hydroxy-6-methylen-15α,16α-methylen-19-nor-17α-pregna-4,20(Z)-dien-21-carbonsäure γ-Lacton |
| | (E/Z)-3-(Hydroxyimino)-17β-hydroxy-6-methylen-15β,16β-methylen-19-nor-17α-pregna-4,20(Z)-dien-21-carbonsäure γ-Lacton |
| 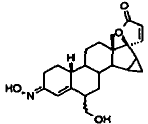 | (E/Z)-3-(Hydroxyimino)-17β-hydroxy-6α-hydroxymethylen-15α,16α-methylen-19-nor-17α-pregna-4,20(Z)-dien-21-carbonsäure γ-Lacton |
| | (E/Z)-3-(Hydroxyimino)-17β-hydroxy-6β-hydroxymethylen-15α,16α-methylen-19-nor-17α-pregna-4,20(Z)-dien-21-carbonsäure γ-Lacton |
| | (E/Z)-3-(Hydroxyimino)-17β-hydroxy-6α-hydroxymethylen-15β,16β- |
| | methylen-19-nor-17α-pregna-4,20(Z)-dien-21-carbonsäure γ-Lacton |
| | (E/Z)-3-(Hydroxyimino)-17β-hydroxy-6β-hydroxymethylen-15β,16β-methylen-19-nor-17α-pregna-4,20(Z)-dien-21-carbonsäure γ-Lacton |
| 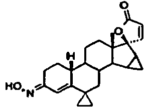 | (E/Z)-3-(Hydroxyimino)-6,6-(1,2-Ethandiyl)-17β-hydroxy-15α,16α-methylen-19-nor-17α-pregna-4,20(Z)-dien-21-carbonsäure γ-Lacton |
| | (E/Z)-3-(Hydroxyimino)-6,6-(1,2-Ethandiyl)-17β-hydroxy-15β,16β-methylen-19-nor-17α-pregna-4,20(Z)-dien-21-carbonsäure γ-Lacton |
| 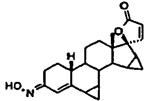 | (E/Z)-3-(Hydroxyimino)-17β-hydroxy-6α,7α;15α,16α-bismethylen-19-nor-17α-pregna-4,20(Z)-dien-21-carbonsäure γ-Lacton |
| | (E/Z)-3-(Hydroxyimino)-17β-hydroxy-6β,7β; 15α,16α-bismethylen-19-nor-17α-pregna-4,20(Z)-dien-21-carbonsäure γ-Lacton |
| | (E/Z)-3-(Hydroxyimino)-17β-hydroxy-6α,7α;15β,16β-bismethylen-19-nor-17α-pregna-4,20(Z)-dien-21-carbonsäure γ-Lacton |
| | (E/Z)-3-(Hydroxyimino)-17β-hydroxy-6β,7β;15β,16β-bismethylen-19-nor-17α-pregna-4,20(Z)-dien-21-carbonsäure γ-Lacton |
| 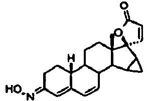 | (E/Z)-3-(Hydroxyimino)-17β-hydroxy-15α,16α-methylen-19-nor-17α-pregna-4,6,20(Z)-trien-21-carbonsäure γ-Lacton |
| | (E/Z)-3-(Hydroxyimino)-17β-hydroxy-15β,16β-methylen-19-nor-17α-pregna-4,6,20(Z)-trien-21-carbonsäure γ-Lacton |
| 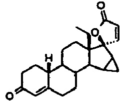 | 17β-Hydroxy-18-methyl-15α,16α-methylen-19-nor-17α-pregna-4,20(Z)-dien-3-on-21-carbonsäure γ-Lacton |
| | 17β-Hydroxy-18-methyl-15β,16β-methylen-19-nor-17a-pregna-4,20(Z)-dien-3-on-21-carbonsäure γ-Lacton (Beispiel 9) |
| 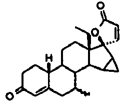 | 17β-Hydroxy-7α-methyl-18-methyl-15α,16α-methylen-19-nor-17α-pregna-4,20(Z)-dien-3-on-21-carbonsäure γ-Lacton |
| | 17β-Hydroxy-7β-methyl-18-methyl-15α,16α-methylen-19-nor-17α-pregna-4,20(Z)-dien-3-on-21-carbonsäure γ-Lacton |
| | 17β-Hydroxy-7α-methyl-18-methyl-15β,16β-methylen-19-nor-17α-pregna-4,20(Z)-dien-3-on-21-carbonsäure γ-Lacton (Beispiel 4A) |
| | 17β-Hydroxy-7β-methyl-18-methyl-15β,16β-methylen-19-nor-17α-pregna-4,20(Z)-dien-3-on-21-carbonsäure γ-Lacton (Beispiel 4B) |
| 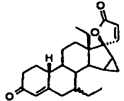 | 17β-Hydroxy-7α-ethyl-18-methyl-15α,16α-methylen-19-nor-17α-pregna-4,20(Z)-dien-3-on-21-carbonsäure γ-Lacton |
| | 17β-Hydroxy-7β-ethyl-18-methyl-15α,16α-methylen-19-nor-17α-pregna-4,20(Z)-dien-3-on-21-carbonsäure γ-Lacton |
| | 17β-Hydroxy-7α-ethyl-18-methyl-15β,16β-methylen-19-nor-17α-pregna-4,20(Z)-dien-3-on-21-carbonsäure γ-Lacton (Beispiel 5A) |
| | 17β-Hydroxy-7β-ethyl-18-methyl-15β,16β-methylen-19-nor-17α-pregna-4,20(Z)-dien-3-on-21-carbonsäure γ-Lacton (Beispiel 5B) |
| 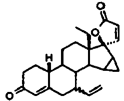 | 17β-Hydroxy-7α-vinyl-18-methyl-15α,16α-methylen-19-nor-17α-pregna-4,20(Z)-dien-3-on-21-carbonsäure γ-Lacton |
| | 17β-Hydroxy-7β-vinyl-18-methyl-15α,16α-methylen-19-nor-17α-pregna-4,20(Z)-dien-3-on-21-carbonsäure γ-Lacton |
| | 17β-Hydroxy-7α-vinyl-18-methyl-15β,16β-methylen-19-nor-17α-pregna-4,20(Z)-dien-3-on-21-carbonsäure γ-Lacton (Beispiel 6A) |
| | 17β-Hydroxy-7β-vinyl-18-methyl-15β,16β-methylen-19-nor-17α-pregna-4,20(Z)-dien-3-on-21-carbonsäure γ-Lacton (Beispiel 6B) |
| 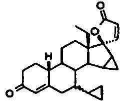 | 17β-Hydroxy-7α-cyclopropyl-18-methyl-15α,16α-methylen-19-nor-17α-pregna-4,20(Z)-dien-3-on-21-carbonsäure γ-Lacton |
| | 17β-Hydroxy-7β-cyclopropyl-18-methyl-15α,16α-methylen-19-nor-17a-pregna-4,20(Z)-dien-3-on-21-carbonsäure γ-Lacton |
| | 17β-Hydroxy-7α-cyclopropyl-18-methyl-15β,16β-methylen-19-nor-17α-pregna-4,20(Z)-dien-3-on-21-carbonsäure γ-Lacton (Beispiel 7A) |
| | 17β-Hydroxy-7β-cyclopropyl-18-methyl-15β,16β-methylen-19-nor-17α-pregna-4,20(Z)-dien-3-on-21-carbonsäure γ-Lacton (Beispiel 7B) |
| 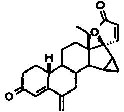 | 17β-Hydroxy-6-methylen-18-methyl-15α,16α-methylen-19-nor-17α-pregna-4,20(Z)-dien-3-on-21-carbonsäure γ-Lacton |
| | 17β-Hydroxy-6-methylen-18-methyl-15β,16β-methylen-19-nor-17α-pregna-4,20(Z)-dien-3-on-21-carbonsäure γ-Lacton |
| 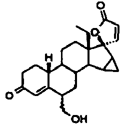 | 17β-Hydroxy-6α-hydroxymethylen-18-methyl-15α,16α-methylen-19-nor-17α-pregna-4,20(Z)-dien-3-on-21-carbonsäure γ-Lacton |
| | 17β-Hydroxy-6β-hydroxymethylen-18-methyl-15α,16α-methylen-19-nor-17α-pregna-4,20(Z)-dien-3-on-21-carbonsäure γ-Lacton |
| | 17β-Hydroxy-6α-hydroxymethylen-18-methyl-15β,16β-methylen-19-nor- |
| | 17α-pregna-4,20(Z)-dien-3-on-21-carbonsäure γ-Lacton |
| | 17β-Hydroxy-6β-hydroxymethylen-18-methyl-15β,16β-methylen-19-nor-17α-pregna-4,20(Z)-dien-3-on-21-carbonsäure γ-Lacton (Beispiel 10) |
| 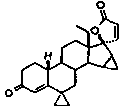 | 6,6-(1,2-Ethandiyl)-17β-hydroxy-18-methyl-15α,16α-methylen-19-nor-17α-pregna-4,20(Z)-dien-3-on-21-carbonsäure γ-Lacton |
| | 6,6-(1,2-Ethandiyl)-17β-hydroxy-18-methyl-15β,16β-methylen-19-nor-17α-pregna-4,20(Z)-dien-3-on-21-carbonsäure γ-Lacton (Beispiel 11) |
| 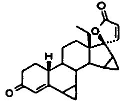 | 17β-Hydroxy-6α,7α; 15α,16α-bismethylen-18-methyl-19-nor-17α-pregna-4,20(Z)-dien-3-on-21-carbonsäure γ-lacton |
| | 17β-Hydroxy-6β,7β;15α,16α-bismethylen-18-methyl-19-nor-17α-pregna-4,20(Z)-dien-3-on-21-carbonsäure γ-Lacton |
| | 17β-Hydroxy-6α,7α; 15β,16β-bismethylen-18-methyl-19-nor-17α-pregna-4,20(Z)-dien-3-on-21-carbonsäure γ-Lacton (Beispiel 2) |
| | 17β-Hydroxy-6β,7β;15β,16β-bismethylen-18-methyl-19-nor-17α-pregna-4,20(Z)-dien-3-on-21-carbonsäure γ-Lacton (Beispiel 1) |
| 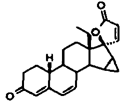 | 17β-Hydroxy-18-methyl-15α,16α-methylen-19-nor-17α-pregna-4,6,20(Z)-trien-3-on-21-carbonsäure γ-Lacton |
| | 17β-Hydroxy-18-methyl-15β,16β-methylen-19-nor-17α-pregna-4,6,20(Z)-trien-3-on-21-carbonsäure γ-Lacton (Beispiel 3) |
| 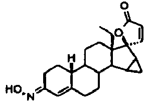 | (E/Z)-3-(Hydroxyimino)-17β-hydroxy-18-methyl-15α,16α-methylen-19-nor-17α-pregna-4,20(Z)-dien-21-carbonsäure γ-Lacton |
| | (E/Z)-3-(Hydroxyimino)-17β-hydroxy-18-methyl-15β,16β-methylen-19-nor-17α-pregna-4,20(Z)-dien-21-carbonsäure γ-Lacton |
| 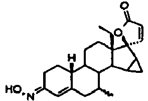 | (E/Z)-3-(Hydroxyimino)-17β-hydroxy-7α-methyl-18-methyl-15α,16α-methylen-19-nor-17α-pregna-4,20(Z)-dien-21-carbonsäure γ-Lacton |
| | (E/Z)-3-(Hydroxyimino)-17β-hydroxy-7β-methyl-18-methyl-15α,16α-methylen-19-nor-17α-pregna-4,20(Z)-dien-21-carbonsäure γ-Lacton |
| | (E/Z)-3-(Hydroxyimino)-17β-hydroxy-7α-methyl-18-methyl-15β,16β-methylen-19-nor-17α-pregna-4,20(Z)-dien-21-carbonsäure γ-Lacton |
| | (E/Z)-3-(Hydroxyimino)-17β-hydroxy-7β-methyl-18-methyl-15β,16β-methylen-19-nor-17α-pregna-4,20(Z)-dien-21-carbonsäure γ-Lacton |
| 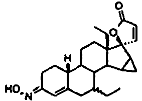 | (E/Z)-3-(Hydroxyimino)-17β-hydroxy-7α-ethyl-18-methyl-15α,16α-methylen-19-nor-17α-pregna-4,20(Z)-dien-21-carbonsäure γ-Lacton |
| | (E/Z)-3-(Hydroxyimino)-17β-hydroxy-7β-ethyl-18-methyl-15α,16α-methylen-19-nor-17α-pregna-4,20(Z)-dien-21-carbonsäure γ-Lacton |
| | (E/Z)-3-(Hydroxyimino)-17β-hydroxy-7α-ethyl-18-methyl-15β,16β-methylen-19-nor-17α-pregna-4,20(Z)-dien-21-carbonsäure γ-Lacton |
| | (E/Z)-3-(Hydroxyimino)-17β-hydroxy-7β-ethyl-18-methyl-15β,16β-methylen-19-nor-17α-pregna-4,20(Z)-dien-21-carbonsäure γ-Lacton |
| 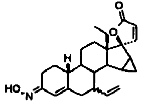 | (E/Z)-3-(Hydroxyimino)-17β-hydroxy-7α-vinyl-18-methyl-15α,16α-methylen-19-nor-17α-pregna-4,20(Z)-dien-21-carbonsäure γ-Lacton |
| | (E/Z)-3-(Hydroxyimino)-17β-hydroxy-7β-vinyl-18-methyl-15α,16α-methylen-19-nor-17α-pregna-4,20(Z)-dien-21-carbonsäure γ-Lacton |
| | (E/Z)-3-(Hydroxyimino)-17β-hydroxy-7α-vinyl-18-methyl-15β,16β-methylen-19-nor-17α-pregna-4,20(Z)-dien-21-carbonsäure γ-Lacton |
| | (E/Z)-3-(Hydroxyimino)-17β-hydroxy-7β-vinyl-18-methyl-15β,16β-methylen-19-nor-17α-pregna-4,20(Z)-dien-21-carbonsäure γ-Lacton |
| 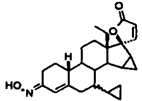 | (E/Z)-3-(Hydroxyimino)-17β-hydroxy-7α-cyclopropyl-18-methyl-15α,16α-methylen-19-nor-17α-pregna-4,20(Z)-dien-21-carbonsäure γ-Lacton |
| | (E/Z)-3-(Hydroxyimino)-17β-hydroxy-7β-cyclopropyl-18-methyl-15α,16α-methylen-19-nor-17α-pregna-4,20(Z)-dien-21-carbonsäure γ-Lacton |
| | (E/Z)-3-(Hydroxyimino)-17β-hydroxy-7α-cyclopropyl-18-methyl-15β,16β-methylen-19-nor-17α-pregna-4,20(Z)-dien-21-carbonsäure γ-Lacton |
| | (E/Z)-3-(Hydroxyimino)-17β-hydroxy-7β-cyclopropyl-18-methyl-15β,16β-methylen-19-nor-17α-pregna-4,20(Z)-dien-21-carbonsäure γ-Lacton |
| 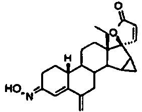 | (E/Z)-3-(Hydroxyimino)-17β-hydroxy-6-methylen-18-methyl-15α,16α-methylen-19-nor-17α-pregna-4,20(Z)-dien-21-carbonsäure γ-Lacton |
| | (E/Z)-3-(Hydroxyimino)-17β-hydroxy-6-methylen-18-methyl-15β,16β-methylen-19-nor-17α-pregna-4,20(Z)-dien-21-carbonsäure γ-Lacton |
| 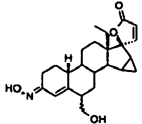 | (E/Z)-3-(Hydroxyimino)-17β-hydroxy-6α-hydroxymethylen-18-methyl-15α,16α-methylen-19-nor-17α-pregna-4,20(Z)-dien-21-carbonsäure γ-Lacton |
| | (E/Z)-3-(Hydroxyimino)-17β-hydroxy-6β-hydroxymethylen-18-methyl-15α,16α-methylen-19-nor-17α-pregna-4,20(Z)-dien-21-carbonsäure γ- |
| | Lacton |
| | (E/Z)-3-(Hydroxyimino)-17β-hydroxy-6α-hydroxymethylen-18-methyl-15β,16β-methylen-19-nor-17α-pregna-4,20(Z)-dien-21-carbonsäure γ-Lacton |
| | (E/Z)-3-(Hydroxyimino)-17β-hydroxy-6β-hydroxymethylen-18-methyl-15β,16β-methylen-19-nor-17α-pregna-4,20(Z)-dien-21-carbonsäure γ-Lacton |
| 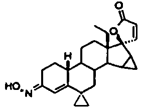 | (E/Z)-3-(Hydroxyimino)-6,6-(1,2-Ethandiyl)-17β-hydroxy-18-methyl-15α,16α-methylen-19-nor-17α-pregna-4,20(Z)-dien-21-carbonsäure γ-Lacton |
| | (E/Z)-3-(Hydroxyimino)-6,6-(1,2-Ethandiyl)-17β-hydroxy-18-methyl-15β,16β-methylen-19-nor-17α-pregna-4,20(Z)-dien-21-carbonsäure γ-Lacton |
| 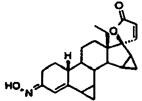 | (E/Z)-3-(Hydroxyimino)-17β-hydroxy-6α,7α,15α,16α-bismethylen-18-methyl-19-nor-17α-pregna-4,20(Z)-dien-21-carbonsäure γ-Lacton |
| | (E/Z)-3-(Hydroxyimino)-17β-hydroxy-6β,7β;15α,16α-bismethylen-18-methyl-19-nor-17α-pregna-4,20(Z)-dien-21-carbonsäure γ-Lacton |
| | (E/Z)-3-(Hydroxyimino)-17β-hydroxy-6α,7a;15β,16β-bismethylen-18-methyl-19-nor-17α-pregna-4,20(Z)-dien-21-carbonsäure γ-Lacton |
| | (E/Z)-3-(Hydroxyimino)-17β-hydroxy-6β,7β;15β,16β-bismethylen-18-methyl-19-nor-17α-pregna-4,20(Z)-dien-21-carbonsäure γ-Lacton |
| | |
| 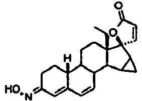 | (E/Z)-3-(Hydroxyimino)-17β-hydroxy-18-methyl-15α,16a-methylen-19-nor-17α-pregna-4,6,20(Z)-trien-21-carbonsäure γ-Lacton |
| | (E/Z)-3-(Hydroxyimino)-17β-hydroxy-18-methyl-15β,16β-methylen-19-nor-17α-pregna-4,6,20(Z)-trien-21-carbonsäure γ-Lacton |
| | 6β,7β;15β,16β-Bismethylen-4-chlor-17β-hydroxy-18-methyl-19-nor-17α-pregna-4,20(Z)-dien-3-on-21-carbonsäure γ-Lacton (Beispiel 8) |
| | 4-Chlor-17β-hydroxy-15α,16α-methylen-19-nor-17α-pregna-4,20(Z)-dien-3-on-21-carbonsäure γ-Lacton (Beispiel 18) |
| | 4-Chlor-17β-hydroxy-15β,16β-methylen-19-nor-17α-pregna-4,20(Z)-dien-3-on-21-carbonsäure γ-Lacton (Beispiel 27) |

Aufgrund ihrer gestagenen Wirksamkeit können die neuen Verbindungen mit der allgemeinen chemischen Formel I allein oder in Kombination mit Estrogen in Arzneimitteln zur Kontrazeption verwendet werden.

Die erfindungsgemäßen Derivate eignen sich daher insbesondere zur Herstellung eines Arzneimittels zur oralen Kontrazeption und zur Behandlung von prä-, peri- und postmenopausalen Beschwerden, einschließlich der Verwendung in Präparaten für die Hormon-Substitutionstherapie (HRT).

Wegen ihres günstigen Wirkungsprofils sind die erfindungsgemäßen Derivate außerdem besonders gut geeignet zur Behandlung prämenstrueller Beschwerden, wie Kopfschmerzen, depressiver Verstimmungen, Wasserretention und Mastodynie.

Besonders bevorzugt ist die Verwendung der erfindungsgemäßen Derivate zur Herstellung eines Arzneimittels mit gestagener und bevorzugt auch antimineralcorticoider und neutraler bis leicht androgener Wirkung.

Eine Behandlung mit den erfindungsgemäßen Derivaten findet bevorzugt am Menschen statt, kann aber auch an verwandten Säugetierspezies, wie beispielsweise an Hund und Katze, durchgeführt werden.

Zur Verwendung der erfindungsgemäßen Derivate als Arzneimittel werden diese mit mindestens einem geeigneten pharmazeutisch unbedenklichen Zusatzstoff, beispielsweise Trägerstoff, kombiniert. Der Zusatzstoff ist beispielsweise für die parenterale, vorzugsweise orale, Applikation geeignet. Es handelt sich dabei um pharmazeutisch geeignete organische oder anorganische inerte Zusatzmaterialien, wie zum Beispiel, Wasser, Gelatine, Gummi arabicum, Milchzucker, Stärke, Magnesiumstearat, Talk, pflanzliche Öle, Polyalkylenglykole usw. Die Arzneimittel können in fester Form, zum Beispiel als Tabletten, Dragees, Suppositorien, Kapseln, oder in flüssiger Form, zum Beispiel als Lösungen, Suspensionen oder Emulsionen, vorliegen. Gegebenenfalls enthalten sie darüber hinaus Hilfsstoffe, wie Konservierungs-, Stabilisierungs-, Netzmittel oder Emulgatoren, Salze zur Veränderung des osmotischen Druckes oder Puffer. Für die parenterale Applikation sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylbenzoat oder Benzylalkohol, zugesetzt werden. Es ist auch möglich, die erfindungsgemäßen Derivate in ein transdermales System einzuarbeiten und sie damit transdermal zu applizieren. Für die orale Applikation kommen insbesondere Tabletten, Dragees, Kapseln, Pillen, Suspensionen oder Lösungen in Frage.

Als Applikationswege kommen weiterhin beispielsweise eine intravaginale oder intrauterine Gabe in Frage. Diese kann durch physiologisch verträgliche Lösungen, wie z.B. einer wässrigen oder öligen Lösung mit oder ohne geeigneten Löslichkeitsvermittler, Dispersionsmittel oder Emulgatoren erfolgen. Als geeignete Öle kommen beispielsweise Erdnussöl, Baumwollsamenöl, Rizinusöl oder Sesamöl in Frage. Die Auswahl ist damit keineswegs darauf beschränkt.

Für die intravaginale oder intrauterine Gabe können spezielle Systeme wie ein intravaginales System (z.B. Vaginalring, VRS) oder ein intrauterines System (IUS) verwendet werden, die eine aktive Substanz der vorliegenden Erfindung aus einem Reservoir auch über eine längere Zeit (z. B. 1, 2, 3, 4 oder 5 Jahre) freisetzen.

Als Beispiel für ein intrauterines System sei stellvertretenderweise MIRENA^{®} angeführt. Hierbei handelt es sich um ein T-förmiges, Levonorgestrel freisetzendes intrauterines Sytem der BAYER SCHERING PHARMA AG.

Weiterhin kann eine Applikation über ein implantiertes Depotsystem aus einem inerten Trägermaterial wie z.B. einem biologisch abbaubaren Polymer oder einem synthetischen Silikon-Polymer erfolgen. Diese Depotsysteme setzten den Wirkstoff kontrolliert über einen längeren Zeitraum frei (z.B. 3 Monate bis 3 Jahre) und werden subkutan implantiert.

Die Dosierung der erfindungsgemäßen Derivate in Kontrazeptionspräparaten soll 0,01 bis 10 mg pro Tag betragen. Die Tagesdosis bei der Behandlung prämenstrueller Beschwerden liegt bei etwa 0,1 bis 20 mg. Die erfindungsgemäßen gestagenen Derivate werden in Kontrazeptionspräparaten sowie in den Arzneimitteln zur Behandlung prämenstrueller Beschwerden vorzugsweise oral appliziert. Die tägliche Dosis wird vorzugsweise einmalig verabreicht. Die vorstehend genannten Dosierungen beziehen sich auf orale Verabreichungsformen.

Bei Verwendung einer Depotformulierung wird die entsprechende, zu den vorstehend genannten oralen Dosierungen equivalente Dosierung kontinuierlich pro Tag aus den längerfristig eingesetzten oben beschriebenen Depotsystemen freigesetzt.

Aus einer Depotformulierung, zum Beispiel aus einem IUS, wird täglich eine Menge von 0,005 bis 10 mg einer Verbindung der allgemeinen Formel 1 freigesetzt.

Die gestagenen und estrogenen Wirkstoffkomponenten werden in Kontrazeptionspräparaten vorzugsweise zusammen oral appliziert. Die tägliche Dosis wird vorzugsweise einmalig verabreicht.

Als Estrogene kömmen synthetische Estrogene, vorzugsweise Ethinylestradiol, aber auch Mestranol, sowie natürliche Estrogene, einschließlich Phytoestrogene, in Betracht.

Das Estrogen wird in einer täglichen Menge verabreicht, die der pharmakologischen Wirkung von 0,01 bis 0,04 mg Ethinylestradiol entspricht. Diese Menge bezieht sich auf eine orale Verabreichungsform. Wird eine anderer Verabreichungsweg gewählt ist eine entsprechende, zur vorstehend genannten oralen Dosierung equivalente Dosierungsmenge zu verwenden.

Als Estrogene in den Arzneimitteln zur Behandlung von prä-, peri- und postmenopausalen Beschwerden sowie für die Hormon-Substitutionstherapie kommen in erster Linie natürliche Estrogene zur Anwendung, vor allem das Estradiol, aber auch die Ester von Estradiol, beispielsweise Estradiolvalerat, oder auch konjugierte Estrogene (CEEs = Conjugated Equine Estrogens).

Die gestagene, antimineralcorticoide und androgene bzw. antiandrogene Wirkung der erfindungsgemaßen Verbindungen wurde mit den folgenden Methoden untersucht:

### 1. Progesteronrezeptor-Bindungstest:

Unter Verwendung von Cytosol aus Progesteronrezeptor-exprimierenden Insektenzellen (Hi5) wurde die kompetitiive Bindefähigkeit an den Progesteronrezeptor ermittelt über die Fähigkeit, ³H-Progesteron als Bezugssubstanz vom Rezeptor zu verdrängen. Verfügt eine Verbindung über eine Progesteron entsprechende Affinität, entspricht das dem Kompetitionsfaktor (KF) von 1. KF-Werte größer als 1 zeichnen sich durch eine geringere, KF-Werte kleiner als 1 durch eine höhere Affinität zum Progesteronrezeptor aus.

### 2. Mineralocorticoidrezeptor-Bindungstest:

Der Test erfolgte analog zu 1., mit folgenden Modifikationen: Zum Einsatz kam Cytosol aus Mineralocorticoidrezeptor-exprimierenden Insektenzellen (Hi5), die Bezugssubstanz war ³H-Aldosteron.

### 3. Androgenrezeptor-Bindungstest:

Der Test erfolgte analog zu 1., mit folgenden Modifikationen: Zum Einsatz kam Cytosol aus Androgenrezeptor-exprimierenden Insektenzellen (Hi5), die Bezugssubstanz war ³H-Testosteron.

Die Ergebnisse der Bindungstests sowie das Verhältnis der Kompetitionsfaktoren KF(PR) und KR(MR) sind in Tabelle 1 wiedergegeben, wobei zum Vergleich Rezeptorbindungswerte auch von Drospirenon als Bezugssubstanz A angegeben sind.

### 4. Bestimmung der gestagenen Wirkung mithilfe von Transaktivierungstests:

Zur Kultivierung der für den Assay verwendeten Zellen wurde als Kultivierungsmedium DMEM (Dulbecco's Modified Eagle Medium: 4500 mg/ml Glukose; PAA, #E15-009) mit 10% FCS (Biochrom, S0115, Charge #615B), 4 mM L-Glutamin, 1 % Penicillin/Streptomycin, 1 mg/ml G418 und 0,5 µg/ml Puromycin verwendet.

Reporter-Zelllinien (CHO K1 Zellen stabil transfiziert mit einem Fusionsprotein aus der PR-Ligandenbindungsdomäne und einer Gal4-Transaktivierungsdomäne sowie einem Reporterkonstrukt, das die Luciferase unter der Kontrolle eines Gal4-responsiven Promotors enthielt) wurden in einer Dichte von 4 x 10⁴ Zellen pro Vertiefung in weißen, undurchsichtigen Gewebekulturplatten mit jeweils 96 Vertiefungen angezüchtet (PerkinElmer, #P12-106-017) und in Kultivierungsmedium mit 3 % DCC-FCS (Aktivkohle behandeltes Serum, zur Entfernung im Serum enthaltener störender Komponenten) gehalten. Die zu untersuchenden Verbindungen wurden acht Stunden später zugegeben, und die Zellen wurden mit den Verbindungen 16 Stunden lang inkubiert. Die Versuche wurden dreifach ausgeführt. Am Ende der Inkubation wurde das Effektor enthaltende Medium entfernt und durch Lysis-Puffer ersetzt. Nachdem Luciferase-Assay-Substrat (Promega, #E1501) zugegeben worden war, wurden die Platten mit den 96 Vertiefungen dann in ein Mikroplatten-Luminometer (Pherastar, BMG labtech) eingeführt, und die Lumineszenz wurde gemessen. Die IC₅₀-Werte wurden unter Verwendung einer Software zur Berechnung von Dosis-Wirkungsbeziehungen ausgewertet. In Tabelle 2 sind Versuchsergebnisse und zum Vergleich entsprechende Ergebnisse von Drospirenon als Bezugssubstanz A wiedergegeben.

### 5. Bestimmung der antimineralocorticoiden Wirkung mithilfe von Transaktivierungstests:

Die Bestimmung der antimineralocorticoiden Aktivität der Testsubstanzen erfolgte analog zu den oben beschriebenen Transaktivierungstests.

Folgende Modifikationen wurden vorgenommen: Hier kamen Reporterzelllinien zum Einsatz (MDCK Zellen), die den humanen Mineralocorticoidrezeptor exprimieren, sowie transient ein Reporterkonstrukt enthalten, das Luciferase unter der Kontrolle eines steroidhormon-responsiven Promoters enthält.

Zur Kultivierung der für den Assay verwendeten Zellen wurde als Kultivierungsmedium DMEM EARLE'S MEM (PAA, Cat.: E15-025) versehen mit 100U Penicillin / 0,1mg/ml Streptomycin (PAA, Cat: P11-010), 4mM L-Glutamin (PAA, Cat: M11-004) sowie foetalem Kälberserum (BIO Witthaker, Cat: DE14-801 F) verwendet.

Zur Bestimmung der antimineralocorticoiden Wirksamkeit wurde den Zellen 1 nM Aldosteron (SIGMA A-6628, Lot 22H4033) zugesetzt, um eine fastmaximale Stimulation des Reportergens zu erreichen. Eine Inhibition des Effektes zeigte eine mineralcorticoidantagonistische Wirkung der Substanzen an (Tabelle 2; zum Vergleich entsprechende Werte für Drospirenon (A)).

### 6. Bestimmung der androgenen / antiandrogenen Wirkung mithilfe von Transaktivierungstests:

Die Bestimmung der androgenen / antiandrogenen Wirkung der Testsubstanzen erfolgte analog zu den oben beschriebenen Transaktivierungstests.

Folgende Modifikationen wurden vorgenommen: Hier kamen Reporterzelllinien zum Einsatz (PC3 Zellen), die den Androgenrezeptor exprimieren, sowie ein Reporterkonstrukt, das Luciferase unter der Kontrolle eines steroidhormon-responsiven Promoters enthält.

Zur Kultivierung der für den Assay verwendeten Zellen wurde als Kultivierungsmedium RPMI Medium ohne Phenolrot (PAA, #E15-49), versehen mit 100U Penicillin / 0,1mg/ml Streptomycin (PAA, Cat: P11-010), 4 mM L-Glutamin (PAA, Cat: M11-004) sowie foetalem Kälberserum (BIO Witthaker, Cat: DE14-801 F), verwendet.

Zur Bestimmung der antiandrogenen Wirksamkeit wurde den Zellen 0,05 nM R1881 zugesetzt, um eine fastmaximale Stimulation des Reportergens zu erreichen. Eine Inhibition des Effektes zeigte eine androgen-antagonistische Wirkung der Substanzen an (Tabelle 2; zum Vergleich entsprechende Werte für Drospirenon (A)).

Soweit die Herstellung der Ausgangsverbindungen hier nicht beschrieben ist, sind diese dem Fachmann bekannt oder analog zu bekannten Verbindungen oder hier beschriebenen Verfahren herstellbar. Die Isomerengemische können nach üblichen Methoden, wie beispielsweise Kristallisation, Chromatographie oder Salzbildung, in die einzelnen Verbindungen aufgetrennt werden. Die Herstellung der Salze erfolgt in üblicher Weise, indem man eine Lösung der Verbindung mit der allgemeinen chemischen Formel I mit der äquivalenten Menge oder einem Überschuss einer Base oder Säure, die sich gegebenenfalls in Lösung befindet, versetzt, gegebenenfalls den Niederschlag abtrennt oder in üblicher Weise die Lösung aufarbeitet.

Die Verbindungen mit der allgemeinen chemischen Formel I werden, ausgehend von Verbindungen mit der allgemeinen chemischen Formel **1** (Schema 2) nach den in Schema 1 angegebenen Verfahren dargestellt, worin R⁴, R^{6a}, R^{6b}, R⁷, R¹⁸ und Z die vorgenannten Bedeutungen haben und worin
- R⁶, R⁷: in **5** und **6** gemeinsam ein Sauerstoffatom oder eine Methylengruppe sind,
- U: ein Sauerstoffatom, zwei Alkoxygruppen OR¹⁹, eine C₂-C₁₀-Alkylen-α,ω-dioxygruppe, die geradkettig oder verzweigt sein kann, ist, wobei R¹⁹ für einen C₁-C₂₀-Alkylrest steht,
- R²⁰: ein C₁-C₂₀-Alkylrest ist,
- X: eine NR^{21a}R^{21b}-Gruppe, eine Alkoxygruppe OR²² ist,
- R^{21a}, R^{21b}: unabhängig voneinander Wasserstoff, C₁-C₁₀-Alkyl sind oder gemeinsam eine C₄-C₁₀-α,ω-Alkylengruppe bilden, die geradkettig oder verzweigt sein kann,
- R²²: ein C₁-C₂₀-Alkylrest ist.

Die Verbindungen **2** und **3** in Schema 1 tragen jeweils eine Doppelbindung zwischen C⁵ und C⁶ oder zwischen C⁵ und C¹⁰ sowie eine weitere Doppelbindung zwischen C² und C³ oder zwischen C³ und C⁴.

Die Verbindungen **7** bis **9** in Schema 1 tragen jeweils eine Doppelbindung zwischen C⁴ und C⁵ oder zwischen C⁵ und C⁶ oder zwischen C⁵ und C¹⁰.

Für den Fachmann ist es selbstverständlich, dass bei den Beschreibungen der synthetischen Transformationen immer vorausgesetzt wird, dass gegebenenfalls am Steroidgerüst vorhandene sonstige funktionelle Gruppen in geeigneter Form geschützt sind.

Die Einführung einer 6,7-Doppelbindung unter Bildung von Verbindungen mit den allgemeinen chemischen Formeln **4**, **13** oder **18** erfolgt über Bromierung der jeweiligen 3,5-Dienolether **3**, **12** oder **17** sowie anschließende Bromwasserstoffabspaltung (siehe z. B. J. Fried, J.A. Edwards, Organic Reactions in Steroid Chemistry, von Nostrand Reinhold Company 1972, S. 265-374).

Die Dienoletherbromierung der Verbindungen **3**, **12** oder **17** kann beispielsweise analog zur Vorschrift aus Steroids 1, 233 (1963) erfolgen. Die Bromwasserstoffabspaltung unter Bildung der Verbindungen mit den allgemeinen chemischen Formeln **4**, **13** oder **18** gelingt durch Erhitzen der 6-Bromverbindung mit basischen Reagenzien, wie beispielsweise mit LiBr oder Li₂CO₃, in aprotischen Lösungsmitteln, wie Dimethylformamid, bei Temperaturen von 50-120°C oder aber indem die 6-Bromverbindungen in einem Lösungsmittel, wie Collidin oder Lutidin, erhitzt werden.

Die Einführung eines Substituenten R⁴ kann zum Beispiel, ausgehend von einer Verbindung mit einer der allgemeinen chemischen Formeln **6**, **11**, **13**, **14**, **16** oder **18**, durch Epoxidierung der 4,5-Doppelbindung mit Wasserstoffperoxid unter alkalischen Bedingungen und Umsetzung der entstandenen Epoxide in einem geeigneten Lösungsmittel mit Säuren mit der allgemeinen chemischen Formel H-R⁴ erfolgen, wobei R⁴ ein Halogenatom, vorzugsweise Chlor oder Brom, sein kann. Verbindungen, in denen R⁴ die Bedeutung von Brom besitzt, lassen sich beispielsweise mit 2,2-Difluor-2-(fluorsulfonyl)essigsäuremethylester in Dimethylformamid in Gegenwart von Kupfer(I)iodid zu Verbindungen umsetzen, in denen R⁴ die Bedeutung Fluor besitzt. Alternativ kann Halogen, ausgehend von einer Verbindung mit einer der allgemeinen chemischen Formeln **6**, **11**, **13**, **14**, **16** oder **18**, durch Umsetzung mit Sulfurylchlorid oder Sulfurylbromid in Gegenwart einer geeigneten Base wie beispielsweise Pyridin, mit R⁴ in der Bedeutung Chlor oder Brom direkt eingeführt werden.

Verbindung **4** wird durch Methenylierung der 6,7-Doppelbindung nach bekannten Verfahren, beispielsweise mit Dimethylsulfoxoniummethylid (siehe zum Beispiel DE-A 11 83 500, DE-A 29 22 500, EP-A 0 019 690, US-A 4,291,029; J. Am. Chem. Soc. 84, 867 (1962)) in eine Verbindung **5** (R⁶, R⁷ bilden gemeinsam eine Methylengruppe) umgewandelt, wobei ein Gemisch der α- und β-Isomeren erhalten wird, das beispielsweise durch Chromatographie in die einzelnen Isomeren getrennt werden kann.

Verbindungen vom Typ **5** können, wie in den Beispielen beschrieben oder analog zu diesen Vorschriften, unter Verwendung analoger zu den dort beschriebenen Reagenzien erhalten werden.

Die Synthese der spirocyclischen Verbindung **18** (R^{6a}, R^{6b} bilden gemeinsam 1,2-Ethandiyl) geht von den Verbindungen **11** oder **14** aus, welche zunächst in ein 3-Amino-3,5-dien-Derivat **15** (X= NR^{21a}R^{21b}) überführt werden. Durch Umsetzung mit Formalin in alkoholischer Lösung wird das 6-Hydroxymethylen-Derivat **16** (R⁶ = Hydroxymethylen) erhalten. Nach Überführung der Hydroxygruppe in eine Fluchtgruppe, wie etwa ein Mesylat, Tosylat oder auch Benzoat, lässt sich Verbindung **18** durch Umsetzung mit Trimethylsulfoxoniumiodid unter Verwendung von Basen, wie etwa Alkalihydroxiden, Alkalialkoholaten, in geeigneten Lösemitteln, wie etwa Dimethylsulfoxid, darstellen.

Zur Einführung einer 6-Methylengruppe kann Verbindung **16** (R⁶ = Hydroxymethylen) mit zum Beispiel Salzsäure in Dioxan/Wasser dehydratisiert werden. Auch nach Überführung der Hydroxygruppe in eine Fluchtgruppe, wie etwa ein Mesylat, Tosylat oder auch Benzoat, lässt sich Verbindung **18** (R^{6a}, R^{6b} bilden gemeinsam Methylen) erzeugen (siehe DE-A 34 02 329, EP-A 0 150 157, US-A 4,584,288; J. Med. Chem. 34, 2464 (1991)).

Eine weitere Möglichkeit zur Herstellung von 6-Methylenverbindungen **18** besteht in der direkten Umsetzung der 4(5)-ungesättigten 3-Ketone, wie beispielsweise von Verbindung **16** (R⁶ = Wasserstoff), mit Acetalen des Formaldehyds in Gegenwart von Natriumacetat mit zum Beispiel Phosphoroxychlorid oder Phosphorpentachlorid in geeigneten Lösungsmitteln, wie Chloroform (siehe zum Beispiel K. Annen, H. Hofmeister, H. Laurent und R. Wiechert, Synthesis 34 (1982)).

Die 6-Methylenverbindungen können zur Darstellung von Verbindungen mit der allgemeinen chemischen Formel **18**, in denen R^{6a} gleich Methyl ist und R^{6b} und R⁷ gemeinsam eine zusätzliche Bindung bilden, genutzt werden.

Hierzu kann man beispielsweise ein in Tetrahedron 21, 1619 (1965) beschriebenes Verfahren anwenden, bei dem eine Isomerisierung der Doppelbindung durch Erwärmen der 6-Methylenverbindungen in Ethanol mit 5% Palladium-Kohle-Katalysator, der entweder mit Wasserstoff oder durch Erwärmen mit einer geringen Menge Cyclohexen vorbehandelt wurde, erzielt werden. Die Isomerisierung kann auch mit einem nicht vorbehandelten Katalysator erfolgen, wenn zur Reaktionsmischung eine geringe Menge Cyclohexen zugesetzt wird. Das Auftreten geringer Anteile hydrierter Produkte kann durch Zugabe eines Überschusses an Natriumacetat verhindert werden.

Alternativ kann die Verbindung **17** (X= OR²²) als Vorstufe verwendet werden. Die direkte Darstellung von 6-Methyl-4,6-dien-3-on-Derivaten ist beschrieben (siehe K. Annen, H. Hofmeister, H. Laurent und R. Wiechert, Lieb. Ann. 712 (1983)).

Verbindungen **18**, in denen R^{6b} eine α-Methylfunktion darstellt, können unter geeigneten Bedingungen aus den 6-Methylenverbindungen (**18**: R^{6a}, R^{6b} bilden gemeinsam Methylen) durch Hydrierung dargestellt werden. Die besten Ergebnisse (selektive Hydrierung der exo-Methylenfunktion) werden durch Transfer-Hydrierung erreicht (J. Chem. Soc. 3578 (1954)). Werden die 6-Methylenderivate **18** in einem geeigneten Lösungsmittel, wie beispielsweise Ethanol, in Gegenwart eines Hydriddonators, wie beispielsweise Cyclohexen, erhitzt, so werden 6α-Methylderivate in sehr guten Ausbeuten erhalten. Geringe Anteile an 6β-Methylverbindung können sauer isomerisiert werden (Tetrahedron 1619 (1965)).

Auch die gezielte Darstellung von 6β-Methylverbindungen ist möglich. Hierfür werden die 4-En-3-one, wie etwa Verbindung **16**, zum Beispiel mit Ethylenglykol, Trimethylorthoformiat in Dichlormethan in Gegenwart katalytischer Mengen einer Säure, zum Beispiel p-Toluolsulfonsäure, zu den entsprechenden 3-Ketalen umgesetzt. Während dieser Ketalisierung isomerisiert die Doppelbindung in die Position C⁵. Eine selektive Epoxidierung dieser 5-Doppelbindung gelingt beispielsweise durch Verwendung organischer Persäuren, zum Beispiel von m-Chlorperbenzoesäure, in einem geeigneten Lösungsmittel, wie Dichlormethan. Alternativ hierzu kann die Epoxidierung auch mit Wasserstoffperoxid in Gegenwart beispielsweise von Hexachloraceton oder 3-Nitrotrifluoracetophenon erfolgen. Die gebildeten 5,6α-Epoxide können dann unter Verwendung entsprechender Alkylmagnesiumhalogenide oder Alkyllithiumverbindungen axial geöffnet werden. Auf diese Weise werden 5α-Hydroxy-6β-Alkylverbindungen erhalten. Die Spaltung der 3-Ketoschutzgruppe kann unter Erhalt der 5α-Hydroxyfunktion durch Behandeln unter milden sauren Bedingungen (Essigsäure oder 4n Salzsäure bei 0°C) erfolgen. Basische Eliminierung der 5α-Hydroxyfunktion mit zum Beispiel verdünnter wässriger Natronlauge ergibt die 3-Keto-4-en-Verbindungen mit einer β-ständigen 6-Alkylgruppe. Alternativ hierzu ergibt die Ketalspaltung unter drastischeren Bedingungen (mit wässriger Salzsäure oder einer anderen starken Säure) die entsprechenden 6α-Alkylverbindungen.

Die Einführung einer 7-Alkyl, 7-Alkenyl- oder 7-Alkinylgruppe zur Bildung von Verbindungen mit der allgemeinen chemischen Formel **14** erfolgt durch 1,6-Addition einer entsprechenden metallorganischen Verbindung an die Vorstufe mit der allgemeinen chemischen Formel **13** unter der Einwirkung von Kupfersalzen. Bevorzugt sind zweiwertige Metalle, wie Magnesium und Zink, als Gegenionen sind Chlor, Brom und Iod bevorzugt. Als Kupfersalze eignen sich ein- oder zweiwertige Kupferverbindungen, wie beispielsweise Kupferchlorid, Kupferbromid oder Kupferacetat. Die Reaktion erfolgt in einem inerten Lösungsmittel, wie beispielsweise Tetrahydrofuran, Diethylether oder Dichlormethan.

Die erhaltenen Verbindungen **6**, **11**, **13**, **14**, **16**, **18** oder **20**, in denen Z für ein Sauerstoffatom steht, können durch Umsetzung mit Hydroxylaminhydrochlorid Alkyloxyaminhydrochloriden oder Sulfonylhydrazinen in Gegenwart eines tertiären Amins bei Temperaturen von -20 bis +40°C in ihre entsprechenden E/Z-konfigurierten Oxime oder Sulfonylhydrazone überführt werden (allgemeine chemische Formel **I** mit Z in der Bedeutung von NOR¹, NNHSO₂R¹)). Geeignete tertiäre Basen sind beispielsweise Trimethylamin, Triethylamin, Pyridin, N,N-Dimethylaminopyridin, 1,5- Diazabicyclo[4.3.0]non-5-en (DBN) und 1,5- Diazabicyclo[5.4.0]undec-5-en (DBU), wobei Pyridin bevorzugt ist. Ein analoges Verfahren ist beispielsweise in WO-A 98/24801 für die Herstellung entsprechender 3-Oxyimino-Derivate des Drospirenons beschrieben.

Zur Herstellung eines Endprodukts mit der allgemeinen chemischen Formel **I** mit Z in der Bedeutung von zwei Wasserstoffatomen kann die 3-Oxogruppe beispielsweise nach der in DE-A 28 05 490 angegebenen Vorschrift durch reduktive Spaltung eines Thioketals der 3-Ketoverbindung auf einer geeigneten Vorstufe, wie beispielsweise von Verbindungen mit einer der allgemeinen chemischen Formeln **6**, **11**, **13**, **14**, **16**, **18** oder **20**, entfernt werden.

Die Bildung von Spirolactonen zu Verbindungen mit einer der allgemeinen chemischen Formeln **6** oder **11** erfolgt, ausgehend von den entsprechenden 17-Hydroxypropenylverbindungen **5** oder **10**, durch Oxidation. Als Oxidationsverfahren seien beispielsweise die Oxidation nach Jones, die Oxidation mit Kaliumpermanganat beispielsweise in einem wässrigen System aus tert.-Butanol und Natriumdihydrogenphosphat, die Oxidation mit Natriumchlorit in wässrigem tert.-Butanol, gegebenenfalls in Gegenwart eines Chlorfängers, wie zum Beispiel in Gegenwart von 2-Methyl-2-buten oder durch Oxidation mit Braunstein genannt.

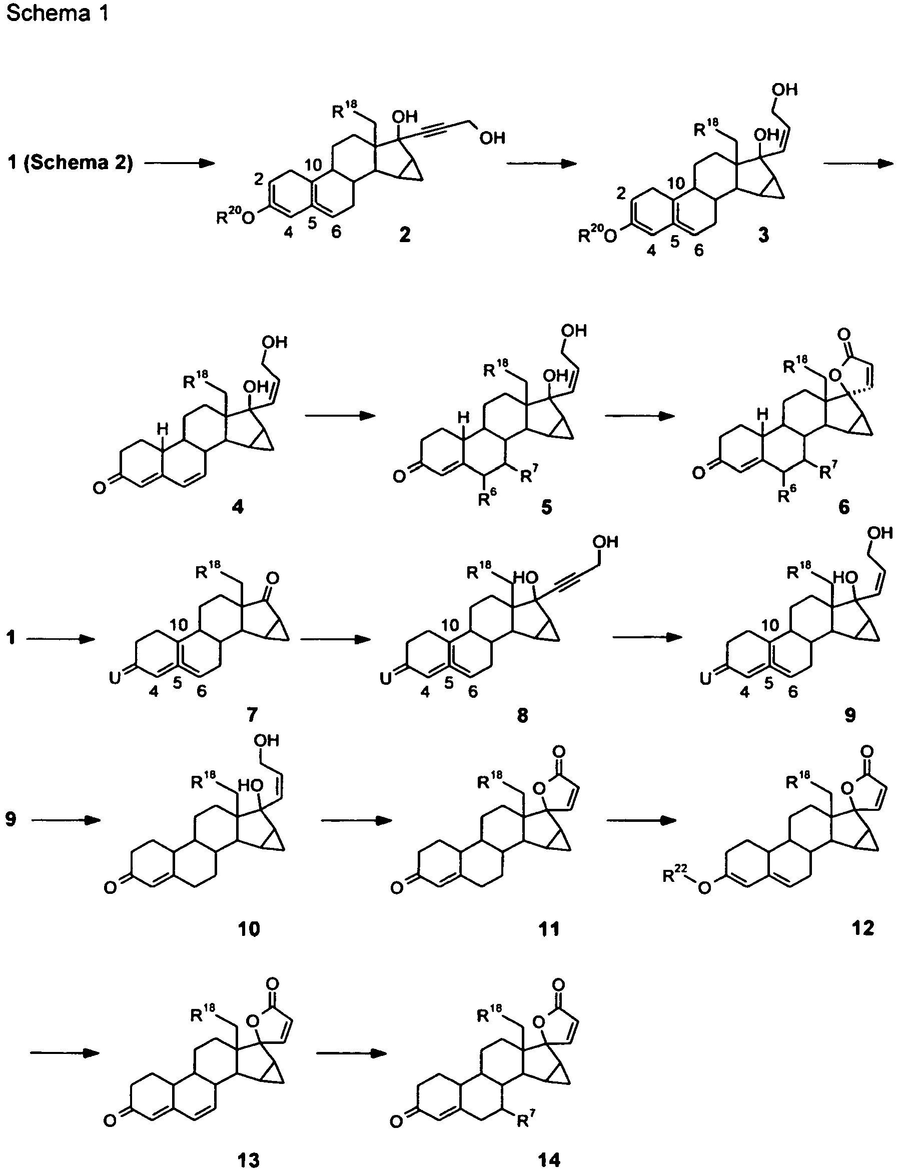

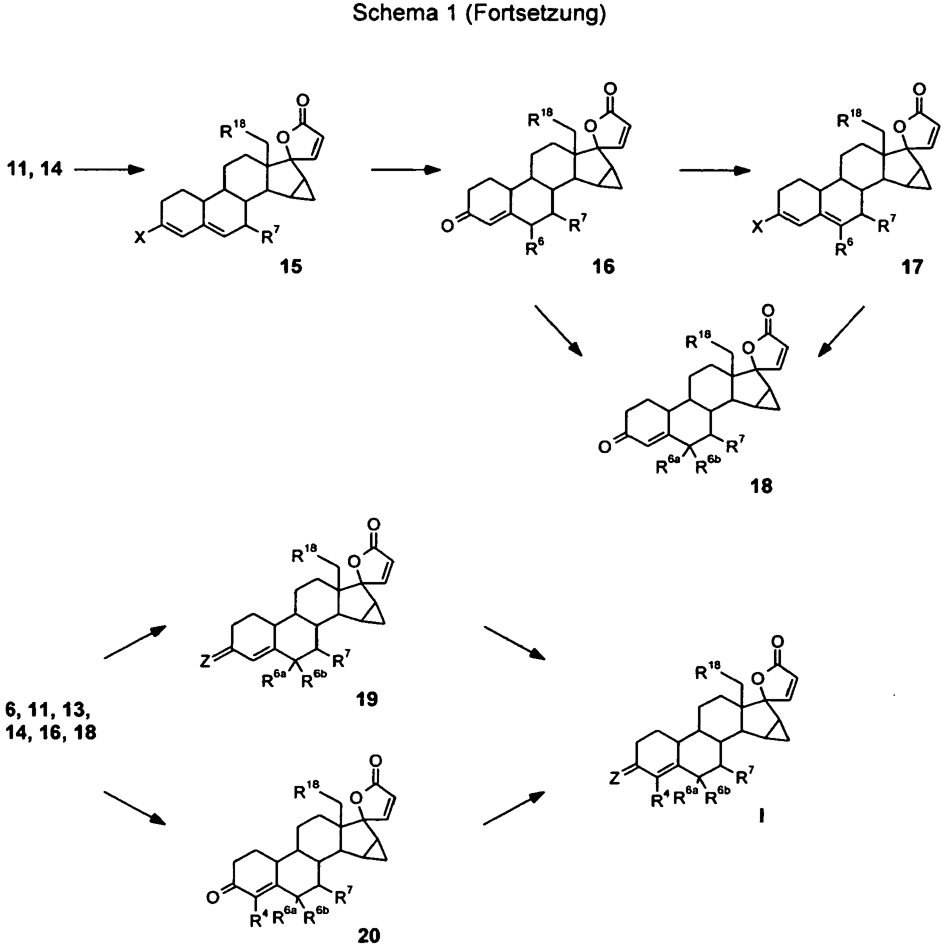

Die Verbindung **1** in Schema 2 trägt eine Doppelbindung zwischen C⁵ und C⁶ oder zwischen C⁵ und C¹⁰ sowie eine weitere Doppelbindung zwischen C² und C³ oder zwischen C³ und C⁴.

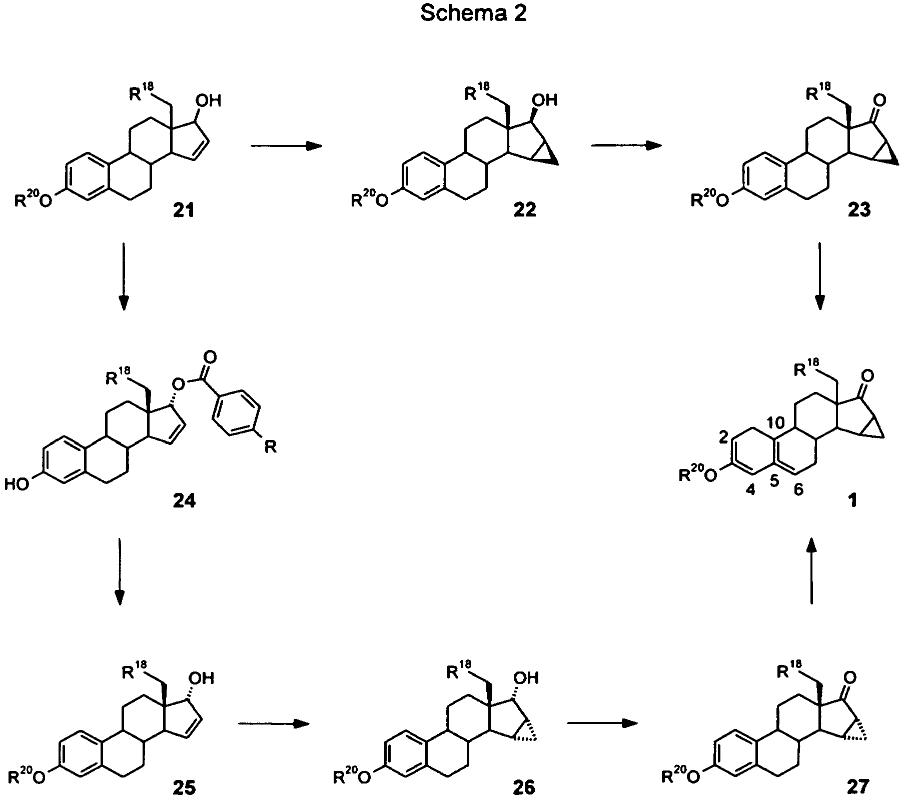

Die nachfolgenden Beispiele dienen der näheren Erläuterung der Erfindung, ohne diese auf die angeführten Beispiele einzugrenzen:

### Beispiel 1: (17-Spirolactonisierung mit Braunstein

### 6β,7β;15β,16β-Bismethylen-17β-hydroxy-18-methyl-19-nor-17α-pregna-4,20(Z)-dien-3-on-21-carbonsäure γ-Lacton

Die Lösung von 865 mg der nach Beispiel 1a dargestellten Verbindung A in 61 ml Dichlormethan wurde mit 5,87 g Braunstein versetzt und ca. 16 Stunden bei 23°C gerührt. Filtriert wurde über Celite, und isoliert wurden nach Einengen und Chromatographie 834 mg der Titelverbindung als kristalliner Feststoff.

¹H-NMR (CDCl₃): δ= 0,6-0,71 (2H), 0,88 (3H), 0,92-1,12 (3H), 1,29-1,99 (13H), 2,12-2,52 (4H), 6,09 (1H), 6,17 (1H), 7,51 (1H) ppm.

### Beispiel 1a: (Corey-Cyclopropanierung)

### 6β,7β;15β,16β-Bismethylen-17α(Z)-(3'-hydroxypropen-1'-yl)-18-methyl-17β-hydroxyestra-4-en-3-on (A) und 6α,7α;15β,16β-Bismethylen-17α(Z)-(3'-hydroxypropen-1'-yl)-18-methyl-17β-hydroxyestra-4-en-3-on (B)

Eine Lösung von 16,8 g Sulfoxoniumiodid in 373 ml Dimethylsulfoxid wurde portionsweise bei 23°C mit 3,33 g einer 55%igen Suspension von Natriumhydrid in Weissöl versetzt. Die Mischung wurde noch 2 Stunden nachgerührt, mit 10,6 g der nach Beispiel 1 b dargestellten Verbindung versetzt und weitere 2,5 Stunden reagieren gelassen. Die Mischung wurde in Wasser gegossen, mehrfach mit Ethylacetat extrahiert, die vereinigten organischen Extrakte wurden mit gesättigter Natriumchloridlösung gewaschen und über Natriumsulfat getrocknet. Der nach Filtration und Lösungsmittelabzug erhaltene Rückstand wurde gereinigt und durch Chromatographie getrennt. Isoliert wurden 1,51 g der Titelverbindung A sowie 1,01 g der Titelverbindung B.

### Beispiel 1b: (Dienonbildung aus Dienolether)

### 17α(Z)-(3'-hydroxypropen-1'-yl)-18-methyl-15β,16β-methylen-17β-hydroxyestra-4,6-dien-3-on

Die Lösung von 10,4 g der nach Beispiel 1 c dargestellten Verbindung in 125 ml Dimethylformamid wurde bei 3°C mit 1,03 g Natriumacetat, 10 ml Wasser und portionsweise mit insgesamt 4,23 g Dibromhydantoin versetzt. Nach 30 Minuten wurde die Mischung mit 3,91 g Lithiumbromid, 3,42 g Lithiumcarbonat versetzt und 3 Stunden lang bei einer Badtemperatur von 100°C erhitzt. Die Mischung wurde in Wasser gegossen und mehrfach mit Ethylacetat extrahiert. Die vereinigten organischen Extrakte wurden mit gesättigter Natriumchloridlösung gewaschen und über Natriumsulfat getrocknet. Der nach Filtration und Lösungsmittelabzug erhaltene Rückstand wurde durch Chromatographie gereinigt. Isoliert wurden 6,35 g der Titelverbindung.

### Beispiel 1c: (Lindlarhydrierung)

### 17α(Z)-(3'-hydroxypropen-1'-yl)-3-methoxy-18-methyl-15β,16β-methylen-17β-hydroxyestra-3,5-dien

Die Lösung von 75 g der nach Beispiel 1d dargestellten Verbindung in 1,5 l Tetrahydrofuran wurde mit 100 ml Pyridin, 5 g Palladium auf Bariumsulfat versetzt und bei einer Atmosphäre Wasserstoff hydriert. Filtriert wurde über Celite, und isoliert wurden nach Einengen 75,7 g der Titelverbindung, die ohne Reinigung weiter umgesetzt wurden.

### Beispiel 1d: (Hydroxypropinaddition)

### 17α(Z)-(3'-hydroxypropin-1'-yl)-3-methoxy-18-methyl-15β,16β-methylen-17β-hydroxyestra-3,5-dien

Die Lösung von 83 ml 2-Propin-1-ol in 1 l Tetrahydrofuran wurde bei -78°C mit 1 l einer 2,5 molaren Lösung von Buthyllithium in Hexan versetzt. Nach 30 Minuten wurde die Lösung von 90 g 3-Methoxy-18-methyl-15β,16β-methylen-estra-3,5-dien-17-on in 0,5 l Tetrahydrofuran zugetropft, auf 23°C erwärmen gelassen und noch 3 Stunden lang gerührt. Die Mischung wurde in gesättigte, eiskalte Ammoniumchloridlösung gegossen, mehrfach mit Ethylacetat extrahiert, die vereinigten organischen Extrakte wurden mit gesättigter Natriumchloridlösung gewaschen und über Natriumsulfat getrocknet. Der nach Filtration und Lösungsmittelabzug erhaltene Rückstand wurde durch Kristallisation gereinigt. Isoliert wurden 90,3 g der Titelverbindung.

### Beispiel 2:

### 6α,7α;15β,16β-Bismethylen-17β-hydroxy-18-methyl-19-nor-17α-pregna-4,20(Z)-dien-3-on-21-carbonsäure γ-Lacton

In Analogie zu Beispiel 1 wurden 114 mg der nach Beispiel 1a dargestellten Verbindung B umgesetzt, und nach Aufarbeitung und Reinigung wurden 68 mg der Titelverbindung isoliert.

¹H-NMR (CDCl₃): δ= 0,58 (1H), 0,77-1,04 (5H), 0,85 (3H), 1,25 (1H), 1,39 (1H), 1,49 (1H), 1,54-1,91 (7H), 1,99-2,18 (4H), 2,26 (1H), 2,49 (1H), 1,99 (2H), 7,48 (1H) ppm.

### Beispiel 3:

### 17β-Hydroxy-18-methyl-15β,16β-methylen-19-nor-17α-pregna-4,6,20(Z)-trien-3-on-21-carbonsäure γ-Lacton

In Analogie zu Beispiel 1 wurden 5 g der nach Beispiel 1a dargestellten Verbindung umgesetzt, und nach Aufarbeitung und Reinigung wurden 4,12 g der Titelverbindung isoliert. ¹H-NMR (CDCl₃): δ= 0,63 (1H), 0,88 (3H), 0,94 (1H), 1,05-1,34 (3H), 1,45-1,93 (7H), 2,16-2,60 (6H), 5,81 (1H), 6,05 (1H), 6,29 (1H), 6,45 (1H), 7,45 (1H) ppm.

### Beispiel 4: (1,6-Addition)

### 7α,18-Bismethyl-17β-hydroxy-15β,16β-methylen-19-nor-17α-pregna-4,20(Z)-dien-3-on-21-carbonsäure γ-Lacton (A) und 7β,18-Bismethyl-17β-hydroxy-15β,16β-methylen-19-nor-17α-pregna-4,20(Z)-dien-3-on-21-carbonsäure γ-Lacton (B)

Zu der auf -30°C gekühlten Suspension von 87 mg Kupfer-(I)-chlorid in 15 ml Tetrahydrofuran wurden 3,6 ml einer 3 molaren Lösung von Methylmagnesiumchlorid in Tetrahydrofuran getropft, und die Lösung wurde noch 10 Minuten gerührt. Die Lösung wurde auf -25°C gekühlt und zu 1,5 g der nach Beispiel 3 dargestellten Verbindung in 70 ml Tetrahydrofuran zugetropft. Nach 1 Minute wurde auf 1 N Salzsäure gegossen, mehrfach mit Ethylacetat extrahiert, die vereinigten organischen Extrakte wurden mit gesättigter Natriumchloridlösung gewaschen und über Natriumsulfat getrocknet. Der nach Filtration und Lösungsmittelabzug erhaltene Rückstand wurde durch Chromatographie gereinigt. Isoliert wurden 20,8 mg der Titelverbindung A sowie 109 mg der Titelverbindung B.

¹H-NMR (CDCl₃) von A: δ= 0,55 (1H), 0,86 (3H), 0,91 (3H), 0,83-0,94 (1H), 1,04 (1H), 1,14-1,28 (2H), 1,41-1,75 (5H), 1,78-1,95 (3H), 2,03-2,15 (2H), 2,19-2,45 (5H), 2,57 (1H), 5,87 (1H), 6,04 (1H), 7,47 (1H) ppm.

¹H-NMR (CDCl₃) von B: δ= 0,62 (1H), 0,86 (3H), 0,90-1,12 (3H), 1,24 (3H), 1,28 (1H), 1,41 (1H), 1,45-1,88 (8H), 2,01-2,30 (5H), 2,37 (1H), 2,49 (1H), 5,83 (1H), 6,02 (1H), 7,44 (1H) ppm.

### Beispiel 5:

### 17β-Hydroxy-7α-ethyl-18-methyl-15β,16β-methylen-19-nor-17α-pregna-4,20(Z)-dien-3-on-21-carbonsäure γ-Lacton (A) und 17β-Hydroxy-7β-ethyl-18-methyl-15β,16β-methylen-19-nor-17α-pregna-4,20(Z)-dien-3-on-21-carbonsäure γ-Lacton (B)

In Analogie zu Beispiel 4 wurden 200 mg der nach Beispiel 3 dargestellten Verbindung unter Verwendung von Ethylmagnesiumchlorid umgesetzt, und nach Aufarbeitung und Reinigung wurden 59 mg der Titelverbindung A neben einem noch verunreinigten Gemisch isoliert, das Anteile der Titelverbindung B enthielt.

¹H-NMR (CDCl₃) von A: δ= 0,63 (1H), 0,86 (3H), 0,96 (3H), 0,89-1,10 (3H), 1,30 (1H), 1,34-1,89 (11H), 2,01-2,42 (6H), 2,62 (1H), 5,85 (1H), 6,02 (1H), 7,43 (1H) ppm.

### Beispiel 6:

### 17β-Hydroxy-7α-vinyl-18-methyl-15β,16β-methylen-19-nor-17α-pregna-4,20(Z)-dien-3-on-21-carbonsäure γ-Lacton (A) und 17β-Hydroxy-7β-vinyl-18-methyl-15β,16β-methylen-19-nor-17α-pregna-4,20(Z)-dien-3-on-21-carbonsäure γ-Lacton (B)

In Analogie zu Beispiel 4 wurden 200 mg der nach Beispiel 3 dargestellten Verbindung unter Verwendung von Vinylmagnesiumchlorid umgesetzt, und nach Aufarbeitung und Reinigung wurden 41 mg der Titelverbindung A neben einem noch verunreinigten Gemisch isoliert, das Anteile der Titelverbindung B enthielt.

¹H-NMR (CDCl₃) von A: δ= 0,61 (1H), 0,90 (3H), 0,94 (1H), 1,10 (1H), 1,24 (1H), 1,33 (1H), 1,46-1,69 (4H), 1,74 (1H), 1,82-2,05 (3H), 2,07-2,38 (4H), 2,48 (1H), 2,56 (1H), 2,70 (1H), 2,87 (1H), 5,22 (1H), 5,27 (1H), 5,92 (2H), 6,07 (1H), 7,49 (1H) ppm.

### Beispiel 7:

### 17β-Hydroxy-7α-cyclopropyl-18-methyl-15β,16β-methylen-19-nor-17α-pregna-4,20(Z)-dien-3-on-21-carbonsäure γ-Lacton (A) und 17β-Hydroxy-7β-cyclopropyl-18-methyl-15β,16β-methylen-19-nor-17α-pregna-4,20(Z)-dien-3-on-21-carbonsäure γ-Lacton (B)

In Analogie zu Beispiel 4 wurden 200 mg der nach Beispiel 3 dargestellten Verbindung unter Verwendung von Cyclopropylmagnesiumbromid umgesetzt, und nach Aufarbeitung und Reinigung wurden 49,2 mg der Titelverbindung A neben einem noch verunreinigten Gemisch isoliert, das Anteile der Titelverbindung B enthielt.

¹H-NMR (CDCl₃) von A: δ= 0,09 (1H), 0,38 (1H), 0,49-0,68 (4H), 0,87 (3H), 0,93 (1H), 1,08 (1H), 1,23 (1H), 1,42-1,63 (6H), 1,71 (1H), 1,83 (1H), 1,88 (1H), 1,99 (1H), 2,13 (1H), 2,24-2,32 (2H), 2,37 (1H), 2,40-2,49 (2H), 2,53 (1H), 5,91 (1H), 6,05 (1H), 7,50 (1H) ppm.

### Beispiel 8: (4-Chlorierung)

### 6β,7β;15β,16β-Bismethylen-4-chlor-17β-hydroxy-18-methyl-19-nor-17α-pregna-4,20(Z)-dien-3-on-21-carbonsäure γ-Lacton

Die Lösung von 50 mg der nach Beispiel 1 dargestellten Verbindung in 0,5 ml Pyridin wurde bei 3°C mit 19 µl Sufurylchlorid versetzt und noch 1,5 Stunden lang bei 3°C gerührt. Die Lösung wurde in gesättigte Natriumhydrogencarbonatlösung gegossen, mehrfach mit Ethylacetat extrahiert, die vereinigten organischen Extrakte wurden mit gesättigter Natriumchloridlösung gewaschen und über Natriumsulfat getrocknet. Der nach Filtration und Lösungsmittelabzug erhaltene Rückstand wurde durch Chromatographie gereinigt. Isoliert wurden 38 mg der Titelverbindung.

¹H-NMR (CDCl₃): δ= 0,67 (1H), 0,88 (3H), 0,95-1,21 (3H), 1,35 (1H), 1,46-1,80 (10H), 1,88 (1H), 2,05 (1H), 2,14-2,51 (4H), 2,73 (1H), 6,10 (1H), 7,52 (1H) ppm.

### Beispiel 9:

### 17β-Hydroxy-18-methyl-15β,16β-methylen-19-nor-17α-pregna-4,20(Z)-dien-3-on-21-carbonsäure γ-Lacton

In Analogie zu Beispiel 1 wurden 200 mg der nach Beispiel 9a dargestellten Verbindung umgesetzt, und nach Aufarbeitung und Reinigung wurden 186 mg der Titelverbindung isoliert.

¹H-NMR (CDCl₃): δ= 0,55 (1H), 0,86 (3H), 0,85-0,99 (2H), 1,06 (1H), 1,17-1,33 (2H), 1,40-1,89 (8H), 2,02-2,46 (7H), 2,57 (1H), 5,86 (1H), 6,03 (1H), 7,45 (1H) ppm.

### Beispiel 9a: (Enonbildung aus Dienolether mit Oxalsäure)

### 17α(Z)-(3'-hydroxypropen-1'-yl)-18-methyl-15β,16β-methylen-17β-hydroxyestra-4-en-3-on

Die Suspension von 5,0 g der nach Beispiel 1c dargestellten Verbindung in 30 ml Aceton und 30 ml Wasser wurde mit 50 ml einer gesättigten wässrigen Lösung von Oxalsäure versetzt, 30 ml Methanol und 50 ml Tetrahydrofuran wurden zugegeben, und die Suspension wurde 5 Stunden lang bei 23°C gerührt. Die Suspension wurde in gesättigte Natriumhydrogencarbonatlösung gegossen, mehrfach mit Ethylacetat extrahiert, die vereinigten organischen Extrakte wurden mit gesättigter Natriumchloridlösung extrahiert und über Natriumsulfat getrocknet. Der nach Filtration und Lösungsmittelabzug erhaltene Rückstand wurde durch Chromatographie gereinigt. Isoliert wurden 4,26 g der Titelverbindung.

### Beispiel 10: (6-Hydroxymethyl-Einführung)

### 17β-Hydroxy-6β-hydroxymethylen-18-methyl-15β,16β-methylen-19-nor-17α-pregna-4,20(Z)-dien-3-on-21-carbonsäure γ-Lacton

Die Lösung von 382 mg der nach Beispiel 10a dargestellten Verbindung in einem Gemisch aus 3,5 ml Toluol und 7,8 ml Ethanol wurde mit 370 µl einer 37%igen wässrigen Formaldehydlösung versetzt und 6 Stunden lang bei 23°C gerührt. Die Lösung wurde eingeengt, und der Rückstand wurde durch Chromatographie gereinigt. Isoliert wurden 119 mg der Titelverbindung.

¹H-NMR (CDCl₃): δ= 0,61 (1H), 0,90 (3H), 0,86-1,18 (3H), 1,29 (1H), 1,45-1,97 (10H), 2,06 (1H), 2,18 (1H), 2,22-2,37 (3H), 2,47 (1H), 2,77 (1H), 3,80 (2H), 5,99 (1H), 6,08 (1H), 7,49 (1H) ppm.

### Beispiel 10a: (Dienamin-Bildung)

### 17β-Hydroxy-18-methyl-15β,16β-methylen-3-pyrrolidinyl-19-nor-17α-pregna-3,5,20(Z)-tri-en-21-carbonsäure γ-Lacton

Die Lösung von 500 mg der nach Beispiel 9 dargestellten Verbindung in 5 ml Methanol wurde mit 271 µl Pyrrolidin versetzt und 2 Stunden lang unter Rückfluss erhitzt. Die Lösung wurde abgekühlt, der Niederschlag wurde abgesaugt, mit wenig kaltem Methanol nachgewaschen, und es wurden 390 mg der Titelverbindung erhalten, die ohne zusätzliche Reinigung weiter umgesetzt wurden.

### Beispiel 11: (6-Spirocyclopropanierung)

### 6,6-(1,2-Ethandiyl)-17β-hydroxy-18-methyl-15β,16β-methylen-19-nor-17α-pregna-4,20(Z)-dien-3-on-21-carbonsäure γ-Lacton

100 mg Trimethylsulfoxoniumiodid wurden in 2,0 ml Dimethylsulfoxid gelöst, das Gemisch wurde mit 18 mg einer 60%igen Natriumhydrid-Dispersion versetzt und 2 Stunden lang bei 23°C gerührt. Anschließend wurde die Lösung von 58 mg der nach Beispiel 11a dargestellten Verbindung in 1,0 ml Dimethylsulfoxid zugetropft und weitere 2,5 Stunden lang bei 23°C gerührt. Das Gemisch wurde in Wasser gegossen, mehrfach mit Ethylacetat extrahiert, die vereinigten organischen Extrakte wurden mit Wasser und gesättigter Natriumchloridlösung gewaschen und über Natriumsulfat getrocknet. Der nach Filtration und Lösungsmittelabzug erhaltene Rückstand wurde durch Chromatographie gereinigt. Isoliert wurden 25,4 mg der Titelverbindung.

¹H-NMR (CDCl₃): δ= 0,44 (1H), 0,53 (1H), 0,62 (1H), 0,80 (1H), 0,88 (3H), 0,90-1,03 (2H), 1,10 (1H), 1,17-1,89 (11H), 1,92-2,30 (5H), 2,42 (1H), 5,72 (1H), 6,04 (1H), 7,45 (1H) ppm.

### Beispiel 11 a: (6-Tosyloxymethyl-Bildung)

### 17β-Hydroxy-18-methyl-15β,16β-methylen-6β-(p-tolylsulfonyloxymethyl)-19-nor-17α-pregna-4,20(Z)-dien-3-on-21-carbonsäure γ-Lacton

Die Lösung von 150 mg der nach Beispiel 10 dargestellten Verbindung in 7 ml Dichlormethan wurde mit 0,65 ml Triethylamin, 175 mg p-Toluolsulfonsäurechlorid versetzt und 6 Stunden lang bei 23°C gerührt. Die Lösung wurde in gesättigte Natriumcarbonatlösung gegossen, mehrfach mit Ethylacetat extrahiert, die vereinigten organischen Extrakte wurden mit Wasser und gesättigter Natriumchloridlösung gewaschen und über Natriumsulfat getrocknet. Der nach Filtration und Lösungsmittelabzug erhaltene Rückstand wurde durch Chromatographie gereinigt. Isoliert wurden 134 mg der Titelverbindung.

### Beispiel 12:

### 17β-Hydroxy-15α, 16α-methylen-19-nor-17α-pregna-4,20(Z)-dien-3-on-21-carbonsäure γ-Lacton

In Analogie zu Beispiel 1 wurden 750 mg der nach Beispiel 12a dargestellten Verbindung umgesetzt, und nach Aufarbeitung und Reinigung wurden 484 mg der Titelverbindung isoliert.

¹H-NMR (CDCl₃): δ= 0,76-0,89 (3H), 1,03 (1H), 1,14 (1H), 1,21-1,33 (2H), 1,37 (3H), 1,39 (1H), 1,45-1,62 (2H), 1,69 (1H), 1,75-1,91 (2H), 2,11-2,49 (6H), 2,58 (1H), 5,89 (1H), 6,06 (1H), 7,40 (1H) ppm.

### Beispiel 12a:

### 17α(Z)-(3'-Hydroxypropen-1'-yl)-15α,16α-methylen-17β-hydroxyestra-4-en-3-on

Die Lösung von 300 mg der nach Beispiel 12b dargestellten Verbindungen in 15 ml Aceton wurde mit 0,83 ml einer 4N Salzsäure versetzt und 1 Stunde lang bei 23°C gerührt. Die Lösung wurde in gesättigte Natriumhydrogencarbonatlösung gegossen, mehrfach mit Ethylacetat extrahiert, die vereinigten organischen Extrakte wurden mit gesättigter Natriumchloridlösung gewaschen und über Natriumsulfat getrocknet. Der nach Filtration und Lösungsmittelabzug erhaltene Rückstand wurde durch Chromatographie gereinigt. Isoliert wurden 135 mg der Titelverbindung.

### Beispiel 12b:

### 17α(Z)-(3'-Hydroxypropen-1'-yl)-15α,16α-methylen-17β-hydroxyestra-5-en-3-on-3-ethylenketal und 17α(Z)-(3'-Hydroxypropen-1'-yl)-15α,16α-methylen-17β-hydroxyestra-5(10)-en-3-on-3-ethylenketal

In Analogie zu Beispiel 1 c wurden 300 mg der nach Beispiel 12c dargestellten Verbindungen umgesetzt, und nach Aufarbeitung wurden 300 mg der Titelverbindungen isoliert, die ohne Reinigung weiter umgesetzt wurden.

### Beispiel 12c:

### 17α(Z)-(3'-Hydroxypropin-1'-yl)-15α,16α-methylen-17β-hydroxyestra-5-en-3-on-3-ethylenketal und 17α(Z)-(3'-Hydroxypropin-1'-yl)-15α,16α-methylen-17β-hydroxyestra-5(10)-en-3-on-3-ethylenketal

In Analogie zu Beispiel 1d wurden 278 mg der nach Beispiel 12d dargestellten Verbindungen umgesetzt und nach Aufarbeitung wurden 347 mg der Titelverbindungen isoliert, die ohne Reinigung weiter umgesetzt wurden.

### Beispiel 12d:

### 15α,16α-Methylen-estra-5-en-3,17-dion-3-ethylenketal und 15α,16α-methylen-estra-5(10)-en-3,17-dion-3-ethylenketal

Die Lösung von 1,06 g der nach Beispiel 12e dargestellten Verbindungen in 32 ml Dichlormethan wurde mit einer Spatelspitze Molekularsieb 4Å, 700 mg N-Methylmorpholino-N-oxid, 90 mg Tetrabutylammoniumperruthenat versetzt und bei 23°C ca. 16 Stunden lang gerührt. Das Gemisch wurde eingeengt, und der Rückstand wurde durch Chromatographie gereinigt. Isoliert wurden 878 mg der Titelverbindungen.

### Beispiel 12e: (Umwandlung von 3-Enolether zu Ethylenketal)

### 15α,16α-Methylen-17α-hydroxyestra-5-en-3-on-3-ethylenketal und 15α,16α-Methylen-17α-hydroxyestra-5(10)-en-3-on-3-ethylenketal

Die Lösung von 500 mg der nach Beispiel 12f dargestellten Verbindung in 10 ml Tetrahydrofuran wurde mit 10 ml Ethylenglykol, 4,4 mg p-Toluolsulfonsäure Hydrat versetzt und bei 23°C 2 Stunden lang gerührt. Die Lösung wurde in gesättigte Natriumhydrogencarbonatlösung gegossen, mehrfach mit Ethylacetat extrahiert, die vereinigten organischen Extrakte wurden mit gesättigter Natriumchloridlösung gewaschen und über Natriumsulfat getrocknet. Der nach Filtration und Lösungsmittelabzug erhaltene Rückstand wurde durch Chromatographie gereinigt. Isoliert wurden 359 mg der Titelverbindung.

### Beispiel 12f: (Birch-Reduktion)3-Methoxy-15α,16α-methylen-17α-hydroxyestra-2,5(10)-dien

597 ml Ammoniak wurden bei -75°C mit 9,91 g Lithium versetzt, und innerhalb von 1 Stunde wurde die Lösung von 24,6 g der nach Beispiel 12g dargestellten Verbindung in 1,2 I Tetrahydrofuran zugetropft. Das Gemisch wurde mit 720 ml Ethanol versetzt, nach 1 Stunde auf -50°C erwärmen gelassen und weitere 2 Stunden lang gerührt. Anschließend wurde mit 600 ml Wasser versetzt, auf 23°C erwärmen gelassen, mehrfach mit Ethylacetat extrahiert, die vereinigten organischen Extrakte wurden mit gesättigter Natriumchloridlösung gewaschen und über Natriumsulfat getrocknet. Nach Filtration und Lösungsmittelabzug wurden 27,1 g der Titelverbindung isoliert, die ohne Reinigung weiter umgesetzt wurde.

### Beispiel 12g:

### 3-Methoxy-15α,16α-methylen-17α-hydroxyestra-1,3,5(10)-trien

Eine Suspension aus 1,5 g Kupfer(II)acetat in 900 ml Diethylether wurde mit 86,6 g Zinkstaub versetzt und 10 Minuten lang unter Rückfluss erhitzt. Anschließend wurde mit 11,7 ml Diiodmethan versetzt und weitere 30 Minuten lang unter Rückfluss erhitzt. Die Lösung von 37,6 g der nach Beispiel 12h dargestellten Verbindung wurde in 100 ml Tetrahydrofuran gegeben, sowie über insgesamt 40 Stunden verteilt wurden insgesamt weitere 35 ml Diiodmethan zugegeben. Das erkaltete Gemisch wurde über Celite filtriert, das Filtrat wurde mit gesättigter Natriumchloridlösung gewaschen und über Natriumsulfat getrocknet. Der nach Filtration und Lösungsmittelabzug erhaltene Rückstand wurde durch Umkristallisation gereinigt. Isoliert wurden 24,6 g der Titelverbindung.

### Beispiel 12h: (Benzoatverseifung)

### 3-Methoxy-17α-hydroxyestra-1,3,5(10),15-tetraen

Die Lösung von 96,3 g der nach Beispiel 12i dargestellten Verbindung in 1,1 I Methanol wurde mit 75,5 g Kaliumcarbonat versetzt, und bei 50°C wurde 2 Stunden lang gerührt. Die Lösung wurde eingeengt, mit Wasser versetzt, mehrfach mit Ethylacetat extrahiert, die vereinigten organischen Extrakte wurden mit Wasser gewaschen und über Natriumsulfat getrocknet. Der nach Filtration und Lösungsmittelabzug erhaltene Rückstand wurde durch Umkristallisation gereinigt. Isoliert wurden 46 g der Titelverbindung.

### Beispiel 12i: (Mitsunobu)

### 4-Nitro-benzoesäure 3-Methoxy-estra-1,3,5(10),15-tetraen-17-yl ester

Die Lösung von 43,9 g 3-Methoxy-17β-hydroxyestra-1,3,5(10),15-tetraen in 1,6 I Tetrahydrofuran wurde mit 121 g Triphenylphosphin, 27,1 g 4-Nitrobenzoesäure, 30,9 ml Azodicarbonsäuredüsopropylester versetzt und bei 23°C 2 Stunden lang gerührt. Die Lösung wurde mit gesättigter Natriumchloridlösung versetzt, mit Ethylacetat extrahiert, die vereinigten organischen Extrakte wurden mit gesättigter Natriumchloridlösung gewaschen und über Natriumsulfat getrocknet. Der nach Filtration und Lösungsmittelabzug erhaltene Rückstand wurde in 1,2 I Aceton aufgenommen, unter Kühlung mit 80 ml einer 30%igen Wasserstoffperoxidlösung versetzt und nach 20 Minuten unter Kühlung in 600 ml einer halbkonzentrierten Natriumthiosulfatlösung eingegossen. Das Gemisch wurde mit Ethylacetat extrahiert, die vereinigten organischen Extrakte wurden mit gesättigter Natriumchloridlösung gewaschen und über Natriumsulfat getrocknet. Der nach Filtration und Lösungsmittelabzug erhaltene Rückstand wurde durch Umkristallisation gereinigt. Isoliert wurden 52,5 g der Titelverbindung.

Beispiel 13:

### 17β-Hydroxy-15α,16α-methylen-19-nor-17α-pregna-4,6,20(Z)-trien-3-on-21-carbonsäure γ-Lacton

In Analogie zu Beispiel 1 b wurden 1,75 g der nach Beispiel 13a dargestellten Verbindung umgesetzt, und nach Aufarbeitung und Reinigung wurden 1,52 g der Titelverbindung isoliert.

¹H-NMR (CDCl₃): δ= 0,82 (1H), 0,92-1,09 (3H), 1,16 (1H), 1,26-1,42 (2H), 1,35 (3H), 1,43-1,63 (2H), 1,72 (1H), 1,89 (1H), 2,19-2,59 (5H), 5,80 (1H), 6,03 (1H), 6,26 (1H), 6,40 (1H), 7,36 (1H) ppm.

### Beispiel 13a: (Dienoletherbildung)

### 17β-Hydroxy-3-methoxy-15α,16α-methylen-19-nor-17α-pregna-3,5,20(Z)-trien-21-carbonsäure γ-Lacton

Die Lösung von 1,84 g der nach Beispiel 12 dargestellten Verbindung in 20 ml 2,2-Dimethoxypropan wurde mit 205 mg Pyridinium-p-toluolsulfonat versetzt und 4 Stunden lang unter Rückfluss erhitzt. Die Lösung wurde in gesättigte Natriumhydrogencarbonatlösung gegossen, mehrfach mit Ethylacetat extrahiert, die vereinigten organischen Extrakte wurden mit gesättigter Natriumchloridlösung extrahiert und über Natriumsulfat getrocknet. Der nach Filtration und Lösungsmittelabzug erhaltene Rückstand wurde durch Umkristallisation gereinigt. Isoliert wurden 1,75 g der Titelverbindung.

### Beispiel 14:

### 17β-Hydroxy-7α-methyl-15α,16α-methylen-19-nor-17α-pregna-4,20(Z)-dien-3-on-21-carbonsäure γ-Lacton (A) und 17β-Hydroxy-7β-methyl-15α,16α-methylen-19-nor-17α-pregna-4,20(Z)-dien-3-on-21-carbonsäure γ-Lacton (B)

In Analogie zu Beispiel 4 wurden 250 mg der nach Beispiel 13 dargestellten Verbindung umgesetzt und nach Aufarbeitung und Reinigung wurden 97 mg der Titelverbindung A neben einem noch verunreinigten Gemisch isoliert, das Anteile der Titelverbindung B enthielt.

¹H-NMR (CDCl₃) von A: δ= 0,74 (1H), 0,85 (3H), 0,88-1,36 (6H), 1,33 (3H), 1,41-1,59 (2H), 1,75-1,92 (3H), 2,05 (1H), 2,19-2,45 (5H), 2,56 (1H), 5,85 (1H), 6,02 (1H), 7,39 (1H) ppm.

### Beispiel 15:

### 17β-Hydroxy-7α-ethyl-15α,16α-methylen-19-nor-17α-pregna-4,20(Z)-dien-3-on-21-carbonsäure γ-Lacton (A) und 17β-Hydroxy-7β-ethyl-15α,16α-methylen-19-nor-17α-pregna-4,20(Z)-dien-3-on-21-carbonsäure γ-Lacton (B)

In Analogie zu Beispiel 4 wurden 250 mg der nach Beispiel 13 dargestellten Verbindung unter Verwendung von Ethylmagnesiumchlorid umgesetzt, und nach Aufarbeitung und Reinigung wurden 105 mg der Titelverbindung A neben einem noch verunreinigten Gemisch isoliert, das Anteile der Titelverbindung B enthielt.

¹H-NMR (CDCl₃) von A: δ= 0,78 (1H), 0,93-1,61 (10H), 0,97 (3H), 1,38 (3H), 1,79-2,04 (4H), 2,11 (1H), 2,24-2,49 (4H), 2,66 (1H), 5,91 (1H), 6,06 (1H), 7,43 (1H) ppm.

### Beispiel 16:

### 17β-Hydroxy-7α-vinyl-15α, 16α-methylen-19-nor-17α-pregna-4,20(Z)-dien-3-on-21-carbonsäure γ-Lacton (A) und 17β-Hydroxy-7β-vinyl-15α,16α-methylen-19-nor-17α-pregna-4,20(Z)-dien-3-on-21-carbonsäure γ-Lacton (B)

In Analogie zu Beispiel 4 wurden 250 mg der nach Beispiel 13 dargestellten Verbindung unter Verwendung von Vinylmagnesiumchlorid umgesetzt, und nach Aufarbeitung und Reinigung wurden 37 mg der Titelverbindung A sowie 6 mg der Titelverbindung B isoliert. ¹H-NMR (CDCl₃) von A: δ= 0,66 (1H), 0,86 (1H), 0,93 (1H), 1,02 (1H), 1,09-1,34 (3H), 1,33 (3H), 1,45-1,57 (2H), 1,75-1,88 (2H), 1,91 (1H), 2,12 (1H), 2,22-2,33 (2H), 2,42 (1H), 2,51 (1H), 2,61 (1H), 2,81 (1H), 5,13 (1H), 5,21 (1H), 5,71 (1H), 5,88 (1H), 6,00 (1H), 7,36 (1H) ppm.

¹H-NMR (CDCl₃) von B: δ= 0,66 (1H), 0,76-0,88 (2H), 0,90-1,03 (2H), 1,17-1,36 (2H), 1,31 (3H), 1,48 (1H), 1,57-1,86 (4H), 2,03-2,44 (7H), 4,98 (1H), 5,10 (1H), 5,84 (1H), 5,88 (1H), 6,01 (1H), 7,35 (1H) ppm.

### Beispiel 17:

### 17β-Hydroxy-7α-cyclopropyl-15α,16α-methylen-19-nor-17α-pregna-4,20(Z)-dien-3-on-21-carbonsäure γ-Lacton (A) und 17β-Hydroxy-7β-cyclopropyl-15α,16α-methylen-19-nor-17α-pregna-4,20(Z)-dien-3-on-21-carbonsäure γ-Lacton (B)

In Analogie zu Beispiel 4 wurden 250 mg der nach Beispiel 13 dargestellten Verbindung unter Verwendung von Cyclopropylmagnesiumbromid umgesetzt, und nach Aufarbeitung und Reinigung wurden 57 mg der Titelverbindung A sowie 3 mg der Titelverbindung B isoliert.

¹H-NMR (CDCl₃) von A: δ= -0,03 (1H), 0,43-0,57 (4H), 0,78 (1H), 0,99 (1H), 1,13-1,40 (7H), 1,34 (3H), 1,50 (1H), 1,80-1,96 (3H), 2,12 (1H), 2,21-2,34 (2H), 2,36-2,51 (2H), 2,56 (1H), 5,90 (1 H), 6,02 (1H), 7,42 (1H) ppm.

¹H-NMR (CDCl₃) von B: δ= 0,06 (1H), 0,44 (1H), 0,49-1,00 (5H), 1,13-1,48 (7H), 1,35 (3H), 1,65-1,98 (4H), 2,09-2,45 (5H), 2,61 (1H), 5,81 (1H), 6,02 (1H), 7,37 (1H) ppm.

### Beispiel 18:

### 4-Chlor-17β-hydroxy-15α, 16α-methylen-19-nor-17α-pregna-4,20(Z)-dien-3-on-21-carbonsäure γ-Lacton

In Analogie zu Beispiel 8 wurden 80 mg der nach Beispiel 12 dargestellten Verbindung umgesetzt, und nach Aufarbeitung und Reinigung wurden 28 mg der Titelverbindung isoliert.

¹H-NMR (CDCl₃): δ= 0,72-0,91 (3H), 0,99 (1H), 1,11 (1H), 1,17-1,40 (3H), 1,33 (3H), 1,48 (1H), 1,52-1,79 (3H), 1,83 (1H), 2,01-2,47 (5H), 2,63 (1H), 3,41 (1H), 6,02 (1H), 7,35 (1H) ppm.

### Beispiel 19:

### 17β-Hydroxy-15β, 16β-methylen-19-nor-17α-pregna-4,20(Z)-dien-3-on-21-carbonsäure γ-Lacton

In Analogie zu Beispiel 1 wurden 2,9 g der nach Beispiel 19a dargestellten Verbindung umgesetzt, und nach Aufarbeitung und Reinigung wurden 1,73 g der Titelverbindung isoliert.

¹H-NMR (CDCl₃): δ= 0,54 (1H), 0,88 (1H), 1,04-1,68 (9H), 1,12 (3H), 1,79 (1H), 1,90 (1H), 2,05-2,46 (6H), 2,56 (1H), 5,86 (1H), 6,02 (1H), 7,43 (1H) ppm.

### Beispiel 19a:

### 17α(Z)-(3'-hydroxypropen-1'-yl)-15β, 16β-methylen-17β-hydroxyestra-4-en-3-on

In Analogie zu Beispiel 9a wurden 14,2 g der nach Beispiel 19b dargestellten Verbindung umgesetzt, und nach Aufarbeitung und Reinigung wurden 11,9 g der Titelverbindung isoliert.

### Beispiel 19b: 17α(Z)-(3'-hydroxypropen-1'-yl)-3-methoxy-15β,16β-methylen-17β-hydroxyestra-3,5-dien

In Analogie zu Beispiel 1 c wurden 23,8 g der nach Beispiel 19c dargestellten Verbindung umgesetzt, und nach Aufarbeitung und Reinigung wurden 23,7 g der Titelverbindung isoliert.

### Beispiel 19c: 17α(Z)-(3'-hydroxypropin-1'-yl)-3-methoxy-15β,16β-methylen-17β-hydroxyestra-3,5-dien

In Analogie zu Beispiel 1 d wurden 38 g 3-Methoxy-15β,16β-methylen-estra-3,5-dien-17-on umgesetzt, und nach Aufarbeitung und Reinigung 39,2 g der Titelverbindung isoliert.

### Beispiel 20:

### 17β-Hydroxy-15β,16β-methylen-19-nor-17a-pregna-4,6,20(Z)-trien-3-on-21-carbonsäure γ-Lacton

In Analogie zu Beispiel 1b wurden 6,9 g der nach Beispiel 20a dargestellten Verbindung umgesetzt, und nach Aufarbeitung und Reinigung wurden 3,2 g der Titelverbindung isoliert.

¹H-NMR (CDCl₃): δ= 0,66 (1H), 1,11-1,75 (8H), 1,19 (3H), 1,83 (1H), 2,10 (1H), 2,23-2,48 (4H), 2,59 (1H), 5,86 (1H), 6,09 (1H), 6,33 (1H), 6,48 (1H), 7,47 (1H) ppm.

### Beispiel 20a:

### 17β-Hydroxy-3-methoxy-15β,16β-methylen-19-nor-17α-pregna-3,5,20(Z)-trien-21-carbonsäure γ-Lacton

In Analogie zu Beispiel 13a wurden 6,5 g der nach Beispiel 19 dargestellten Verbindung umgesetzt und 6,9 g der Titelverbindung isoliert, die ohne Reinigung weiter umgesetzt wurde.

### Beispiel 21:

### 17β-Hydroxy-6β-hydroxymethylen-15β,16β-methylen-19-nor-17α-pregna-4,20(Z)-dien-3-on-21-carbonsäure γ-Lacton

In Analogie zu Beispiel 10 wurden 727 mg der nach Beispiel 21a dargestellten Verbindung umgesetzt, und nach Aufarbeitung und Reinigung wurden 266 mg der Titelverbindung isoliert.

¹H-NMR (CDCl₃): δ= 0,60 (1H), 0,93 (1H), 1,09-1,95 (12H), 1,16 (3H), 2,13-2,53 (5H), 2,77 (1H), 3,80 (2H), 5,99 (1H), 6,07 (1H), 7,47 (1H) ppm.

### Beispiel 21a:

### 17β-Hydroxy-15β,16β-methylen-3-pyrrolidinyl-19-nor-17α-pregna-3,5,20(Z)-trien-21-carbonsäure γ-Lacton

In Analogie zu Beispiel 10a wurden 783 mg der nach Beispiel 19 dargestellten Verbindung umgesetzt, und nach Aufarbeitung und Reinigung wurden 734 mg der Titelverbindung isoliert.

### Beispiel 22:

### 6,6-(1,2-Ethandiyl)-17β-hydroxy-15β,16β-methylen-19-nor-17α-pregna-4,20(Z)-dien-3-on-21-carbonsäure γ-Lacton

In Analogie zu Beispiel 11 wurden 136 mg der nach Beispiel 22a dargestellten Verbindung umgesetzt, und nach Aufarbeitung und Reinigung wurden 40 mg der Titelverbindung isoliert.

¹H-NMR (CDCl₃): δ= 0,44 (1H), 0,52 (1H), 0,61 (1H), 0,80 (1H), 0,98 (1H), 1,11-1,52 (9H), 1,14 (3H), 1,71-1,93 (4H), 2,15-2,28 (3H), 2,41 (1H), 5,71 (1H), 6,02 (1H), 7,43 (1H) ppm.

### Beispiel 22a:

### 17β-Hydroxy-15β,16β-methylen-6β-(p-tolylsulfonyloxymethyl)-19-nor-17α-pregna-4,20(Z)-dien-3-on-21-carbonsäure γ-Lacton

In Analogie zu Beispiel 11a wurden 730 mg der nach Beispiel 21 dargestellten Verbindung umgesetzt, und nach Aufarbeitung und Reinigung wurden 143 mg der Titelverbindung isoliert.

### Beispiel 23:

### 17β-Hydroxy-7α-methyl-15β,16β-methylen-19-nor-17α-pregna-4,20(Z)-dien-3-on-21-carbonsäure γ-Lacton (A) und 17β-Hydroxy-7β-methyl-15β,16β-methylen-19-nor-17α-pregna-4,20(Z)-dien-3-on-21-carbonsäure γ-Lacton (B)

In Analogie zu Beispiel 4 wurden 250 mg der nach Beispiel 20 dargestellten Verbindung umgesetzt, und nach Aufarbeitung und Reinigung wurden 76 mg der Titelverbindung A neben einem noch verunreinigten Gemisch isoliert, das Anteile der Titelverbindung B enthielt.

¹H-NMR (CDCl₃) von A: δ= 0,55 (1H), 0,91 (3H), 1,02-1,58 (8H), 1,13 (3H), 1,73-1,87 (2H), 1,97 (1H), 2,06 (1H), 2,20-2,45 (5H), 2,57 (1H), 5,87 (1H), 6,03 (1H), 7,45 (1H) ppm.

### Beispiel 24:

### 7α-Ethyl-17β-hydroxy-15β,16β-methylen-19-nor-17α-pregna-4,20(Z)-dien-3-on-21-carbonsäure γ-Lacton (A) und 7β-Ethyl-17β-hydroxy-15β,16β-methylen-19-nor-17α-pregna-4,20(Z)-dien-3-on-21-carbonsäure γ-Lacton (B)

In Analogie zu Beispiel 4 wurden 250 mg der nach Beispiel 20 dargestellten Verbindung unter Verwendung von Ethylmagnesiumchlorid umgesetzt, und nach Aufarbeitung und Reinigung wurden 57 mg der Titelverbindung A sowie 23 mg der Titelverbindung B isoliert.

¹H-NMR (CDCl₃) von A: δ= 0,56 (1H), 0,95 (3H), 1,02-1,55 (10H), 1,12 (3H), 1,76-1,86 (2H), 1,93 (1H), 2,02-2,10 (2H), 2,20-2,44 (4H), 2,62 (1H), 5,87 (1H), 6,02 (1H), 7,44 (1H) ppm.

¹H-NMR (CDCl₃) von B: δ= 0,63 (1H), 0,96 (3H), 1,00 (1H), 1,06-1,68 (11H), 1,14 (3H), 1,84 (1H), 1,99-2,30 (5H), 2,37 (1H), 2,60 (1H), 5,84 (1H), 6,01 (1H), 7,41 (1H) ppm.

### Beispiel 25:

### 17β-Hydroxy-15β,16β-methylen-7α-vinyl-19-nor-17α-pregna-4,20(Z)-dien-3-on-21-carbonsäure γ-Lacton (A) und 17β-Hydroxy-15β,16β-methylen-7β-vinyl-19-nor-17α-pregna-4,20(Z)-dien-3-on-21-carbonsäure γ-Lacton (B)

In Analogie zu Beispiel 4 wurden 250 mg der nach Beispiel 20 dargestellten Verbindung unter Verwendung von Vinylmagnesiumchlorid umgesetzt, und nach Aufarbeitung und Reinigung wurden 29 mg der Titelverbindung A neben einem noch verunreinigten Gemisch isoliert, das Anteile der Titelverbindung B enthielt.

¹H-NMR (CDCl₃) von A: δ= 0,56 (1H), 1,07 (1H), 1,12 (3H), 1,18-1,31 (4H), 1,39 (1H), 1,49-1,60 (2H), 1,79-1,87 (2H), 1,92 (1H), 2,12 (1H), 2,21-2,33 (2H), 2,43 (1H), 2,51 (1H), 2,66 (1H), 2,83 (1H), 5,17 (1H), 5,22 (1H), 5,85 (1H), 5,87 (1H), 6,01 (1H), 7,43 (1H) ppm.

### Beispiel 26:

### 7α-Cyclopropyl-17β-hydroxy-15β,16β-methylen-19-nor-17α-pregna-4,20(Z)-dien-3-on-21-carbonsäure γ-Lacton (A) und 7β-Cyclopropyl-17β-hydroxy-15β,16β-methylen-19-nor-17α-pregna-4,20(Z)-dien-3-on-21-carbonsäure γ-Lacton (B)

In Analogie zu Beispiel 4 wurden 250 mg der nach Beispiel 20 dargestellten Verbindung umgesetzt, und nach Aufarbeitung und Reinigung wurden 91 mg der Titelverbindung A sowie 25 mg der Titelverbindung B isoliert.

¹H-NMR (CDCl₃) von A: δ= 0,07 (1H), 0,39 (1H), 0,47-0,68 (4H), 1,12 (4H), 1,21-1,32 (3H), 1,35-1,61 (5H), 1,83-1,92 (2H), 2,11 (1H), 2,20-2,33 (3H), 2,39-2,49 (2H), 2,53 (1H), 5,90 (1H), 6,03 (1H), 7,48 (1H) ppm.

¹H-NMR (CDCl₃) von B: δ= 0,28 (1H), 0,35 (1H), 0,53-0,67 (3H), 0,78-1,12 (4H), 1,14 (3H), 1,21-1,51 (5H), 1,59-1,67 (2H), 1,83 (1H), 1,99-2,30 (5H), 2,39 (1H), 2,57 (1H), 5,82 (1H), 6,02 (1H), 7,43 (1H) ppm.

### Beispiel 27:

### 4-Chlor-17β-hydroxy-15β, 16β-methylen-19-nor-17α-pregna-4,20(Z)-dien-3-on-21-carbonsäure γ-Lacton

In Analogie zu Beispiel 8 wurden 100 mg der nach Beispiel 19 dargestellten Verbindung umgesetzt, und nach Aufarbeitung und Reinigung wurden 70 mg der Titelverbindung isoliert.

¹H-NMR (CDCl₃): δ= 0,61 (1H), 1,01 (1H), 1,11-1,73 (9H), 1,17 (3H), 1,83 (1H), 1,94 (1H), 2,17-2,50 (5H), 2,69 (1H), 3,48 (1H), 6,07 (1H), 7,48 (1H) ppm.

### Beispiel 28:

### 6,6-(1,2-Ethandiyl)-17β-hydroxy-15α,16α-methylen-19-nor-17α-pregna-4,20(Z)-dien-3-on-21-carbonsäure γ-Lacton

In Analogie zu Beispiel 11 wurden 115 mg der nach Beispiel 28a dargestellten Verbindung umgesetzt, und nach Aufarbeitung und Reinigung wurden 25 mg der Titelverbindung isoliert.

¹H-NMR (CDCl₃): δ= 0,44 (1H), 0,60 (1H), 0,69-1,11 (6H), 1,20-1,53 (6H), 1,35 (3H), 1,65-1,97 (4H), 2,15-2,31 (3H), 2,41 (1H), 5,70 (1H), 6,02 (1H), 7,36 (1H) ppm.

### Beispiel 28a:

### 17β-Hydroxy-15α, 16α-methylen-6β-(p-tolylsulfonyloxymethyl)-19-nor-17α-pregna-4,20(Z)-dien-3-on-21-carbonsäure γ-Lacton

In Analogie zu Beispiel 11 a wurden 890 mg der nach Beispiel 28b dargestellten Verbindung umgesetzt, und nach Aufarbeitung und Reinigung wurden 115 mg der Titelverbindung isoliert.

### Beispiel 28b:

### 17β-Hydroxy-6-hydroxymethylen-15α,16α-methylen-19-nor-17α-pregna-4,20(Z)-dien-3-on-21-carbonsäure γ-Lacton

In Analogie zu Beispiel 10 wurden 895 mg der nach Beispiel 28c dargestellten Verbindung umgesetzt, und nach Aufarbeitung wurden 1,1 g der Titelverbindung als Rohprodukt isoliert, das ohne Reinigung weiter umgesetzt wurde.

### Beispiel 28c:

### 17β-Hydroxy-15α,16α-methylen-3-pyrrolidinyl-19-nor-17α-pregna-3,5,20(Z)-trien-21-carbonsäure γ-Lacton

In Analogie zu Beispiel 10a wurden 858 mg der nach Beispiel 12 dargestellten Verbindung umgesetzt, und nach Aufarbeitung und Reinigung wurden 895 mg der Titelverbindung isoliert.

### Beispiel 29:

### 6β,7β;15β,16β-Bismethylen-17β-hydroxy-19-nor-17α-pregna-4,20(Z)-dien-3-on-21-carbonsäure γ-Lacton (A) und 6α,7α;15β,16β-Bismethylen-17β-hydroxy-19-nor-17α-pregna-4,20(Z)-dien-3-on-21-carbonsäure γ-Lacton (B)

In Analogie zu Beispiel 1a wurden 1,0 g der nach Beispiel 20 dargestellten Verbindung umgesetzt, und nach Aufarbeitung und Reinigung wurden 143 mg der Titelverbindung A sowie 182 mg der Titelverbindung B isoliert.

¹H-NMR (CDCl₃) von A: δ= 0,57-0,63 (2H), 0,98 (1H), 1,04-1,17 (2H), 1,09 (3H), 1,26-1,35 (3H), 1,42-1,77 (7H), 1,88 (1H), 2,10-2,21 (2H), 2,27 (1H), 2,42 (1H), 6,04 (1H), 6,12 (1H), 7,44 (1H) ppm.

¹H-NMR (CDCl₃) von B: δ= 0,58 (1H), 0,76-0,86 (2H), 1,00 (1H), 1,04-1,21 (2H), 1,15 (3H), 1,25-1,45 (4H), 1,62-1,69 (2H), 1,76 (1H), 1,87 (1H), 1,98-2,14 (4H), 2,26 (1H), 2,50 (1H), 6,03 (1H), 6,05 (1H), 7,46 (1H) ppm

### Beispiel 30

Inerte, intrauterin implantierbare Depotsysteme aus einem biologisch abbaubaren Polymer bzw. einem synthetischen Silikon-Polymer, bestehend aus einem wirkstoffhaltigen Kern in entsprechendem Polymer-Wirkstoff-Mischungsverhältnis, umgeben von einer die gewünschte tägliche Freisetzungsrate gewährleistenden Polymermembran, werden in das Uteruslumen von Ratten verbracht. Die weiblichen Tiere werden vorher kastriert und mit Estradiol über drei Tage vorbehandelt. Die Implantate von unterschiedlicher Länge (5-20 mm) und einem begrenzten Durchmesser (1.1 bis 2 mm) verbleiben zwischen 4 und 14 Tage im Rattenuterus, um die lokale wie sy-stemische gestagene Wirkung des freigesetzten Wirkstoffes anhand verschiedener Parameter in unterschiedlichen Gewebe zu untersuchen. Folgende Parameter werden ermittelt: 1) gestagene lokale Wirkung am Uterus anhand des Uterusgewichts, der histologisch erfassbaren Epithelhöhe und der Expression gestagenregulierter Markergene (z.B. IGFBP-1); 2) gestagene systemische Wirkung an der Mamma anhand der Expression gestagenregulierter Markergene (z.B. RankL), 3) gestagene systemische Wirkung an der Hypophyse anhand des LH-Spiegels (Absenkung des estrogen-induziert erhöhten LH-Spiegels).

Die Verbindungen der vorliegenden Erfindung zeigen einen signifikanten gestagenen Effekt im Uterus der vergleichbar mit einer entsprechenden Behandlung mit einem Levonorgestrel enthaltenden Depotsystems wie MIRENA^{®} ist.

**Tabelle 1: Rezeptorbindungswerte**

| | | Rezeptorbindung | | | | | |
|---|---|---|---|---|---|---|---|
| | | Progesteronrezeptor (PR) | | Mineralocorticoidrezeptor (MR) | Androgenrezeptor | | |
| Bsp. | Struktur | IC50 [nM] | Competition factor | Competition factor | IC50 [nM] | Competition factor | KF PR / KF MR |
| A | 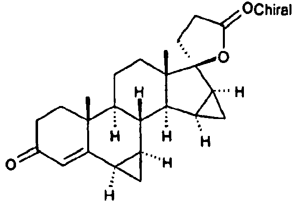 | 43,3 | 2,7 | 0,5 | 630 | 37 | 5,40 |
| 1 | 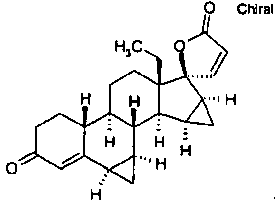 | 41 | 0,76 | 1,6 | 280 | 30,6 | 0,48 |
| 2 | 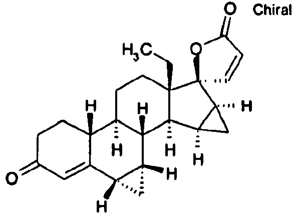 | 630 | 24,37 | 4,4 | 450 | 49,6 | 5,54 |
| 3 | 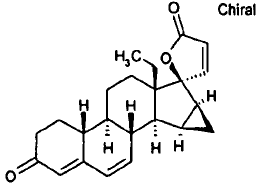 | 72 | 2,72 | 1,5 | 1300 | 62,9 | 1,81 |
| 4A | 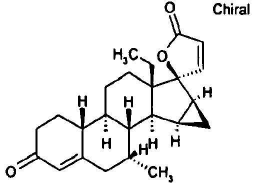 | 48 | 2,66 | 0,6 | 60 | 2,6 | 4,43 |
| 5A | 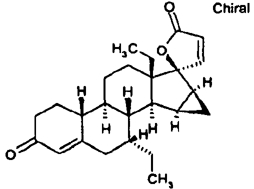 | 300 | 12,00 | 1,0 | 110 | 4,1 | 12,00 |
| 6A | 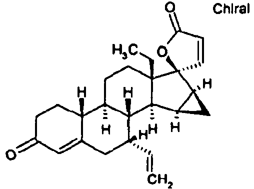 | 120 | 4,85 | 1,5 | 45 | 1,7 | 3,23 |
| 7A | 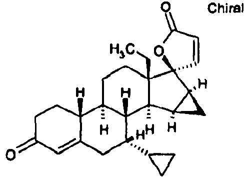 | 380 | 14,22 | 4,7 | 170 | 5,2 | 3,03 |
| 7B | 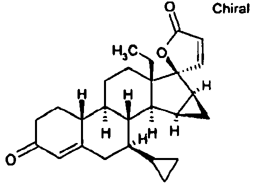 | | | | | | |
| 8 | 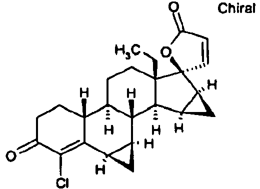 | 740 | 31,12 | 29,0 | 4300 | 240,0 | 1,07 |
| 9 | 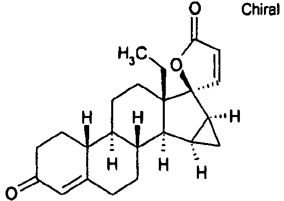 | 78,5 | 1,65 | 1,1 | 0 | 5,6 | 1,50 |
| 10 | 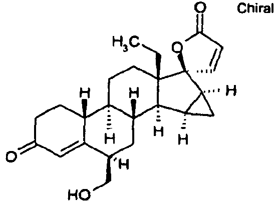 | 167 | 4000,00 | 2,7 | 1000 | 1000,0 | inaktiv |
| 12 | 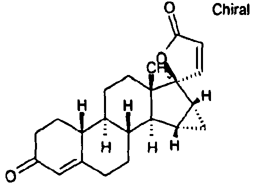 | 49 | 1,50 | 2,9 | 200 | 16,5 | 0,52 |
| 13 | 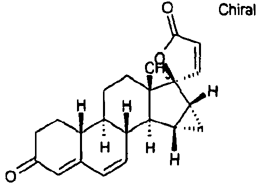 | 190 | 7,89 | 1,6 | 5100 | 163,2 | 4,93 |
| 14A | 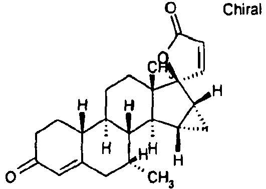 | 49 | 2,04 | 1,3 | 74 | 2,3 | 1,57 |
| 15A | 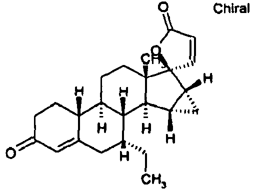 | 200 | 8,36 | 0,8 | 60 | 2,2 | 10,45 |
| 16A | 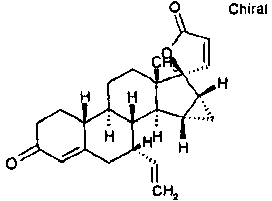 | 85 | 3,76 | 2,0 | 39 | 2,6 | 1,88 |
| 16B | 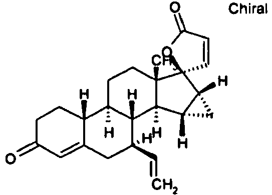 | | | | | | |
| 17A | 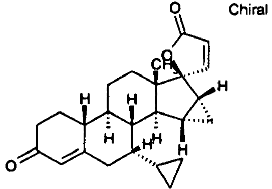 | 280 | 12,24 | 2,6 | 77 | 5,2 | |
| 17B | 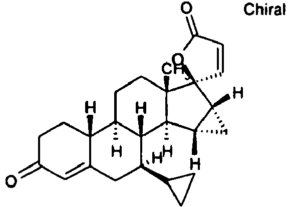 | | | | | | |
| 18 | 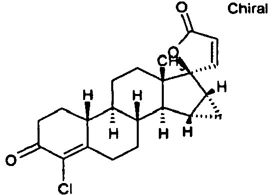 | 400 | 17,24 | 35,0 | 140 | 7,3 | |
| 19 | 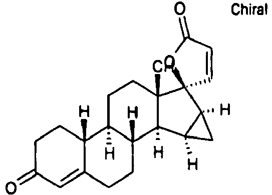 | 25 | 1,70 | 187,0 | 52 | 6,4 | |
| 20 | 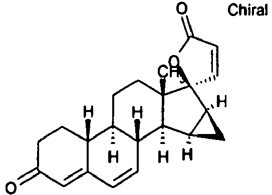 | | | | | | |
| 21 | 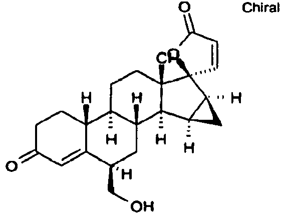 | 4800 | 159,27 | | 1000 | 1000,0 | |
| 22 | 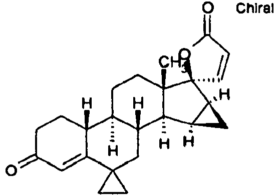 | 30 | 1,04 | 0,8 | | 2,0 | 1,30 |
| 23A | 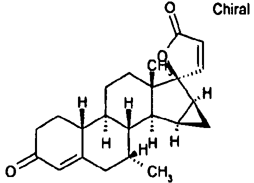 | 120 | 3,79 | 1,9 | 81 | 4,2 | 1,99 |
| 24A | 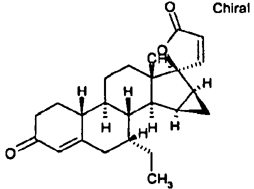 | 1300 | 41,55 | 1,9 | 160 | 8,1 | 21,87 |
| 25A | 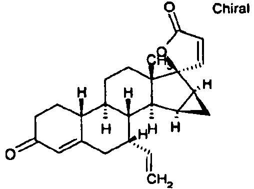 | 150 | 6,14 | 9,4 | 37 | 1,4 | 0,65 |
| 27 | 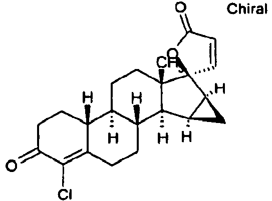 | 2900 | 101,93 | 47,0 | 810 | 36,4 | 2,17 |

**Tabelle 2: Werte zur in vitro-Transaktivierung**

| | | *In vitro*-Transaktivierung | | | | | | | |
|---|---|---|---|---|---|---|---|---|---|
| | | Progesteronrezeptor | | Mineralocorticoidrezeptor | | Androgenrezeptor | | | |
| Bsp. | Struktur | Agonismus EC50 [nM] | Agonismus Wirksamkeit [%] | Antagonismus IC50 [nM] | Antagonismus Wirksamkeit [%] | Agonismus EC50 [nM] | Agonismus Wirksamkeit [%] | Antagonismus IC50 [nM] | Antagonismus Wirksamkeit [%] |
| A | 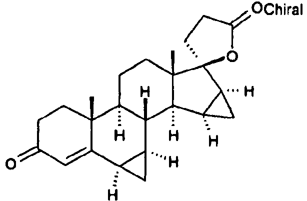 | 88 | 72,2 | 3,3 | 64,1 | 112,5 | 24,26 | 27 | 54,58 |
| 1 | 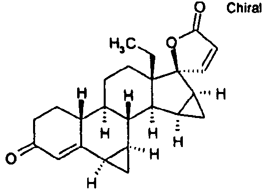 | 1,5 | 62,8 | 10 | 79,5 | 100 | 46,3 | 1000 | 5 |
| 2 | 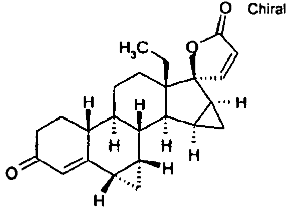 | | | 230 | 111,9 | 130 | 32,6 | 1000 | 5 |
| 3 | 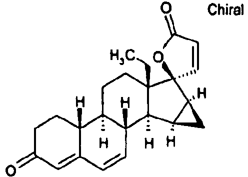 | 1,4 | 34,2 | 22 | 97,6 | 120 | 25,39 | 1000 | 5 |
| 4A | 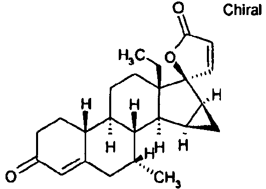 | 39,0 | 71,1 | 3 | 99,9 | 3 | 97,55 | 1000 | 5 |
| 5A | 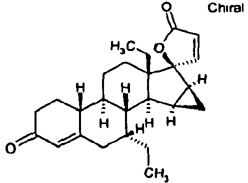 | 99,0 | 35,7 | | | 8,3 | 73 | 1000 | 5 |
| 6A | 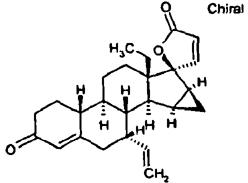 | 140,0 | 35,2 | 9 | 101,8 | 0,98 | 117,61 | 1000 | 5 |
| 7A | 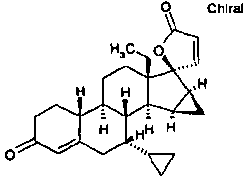 | 1000,0 | 5,0 | 77 | 101,9 | 33 | 62,57 | 1000 | 5 |
| 7B | 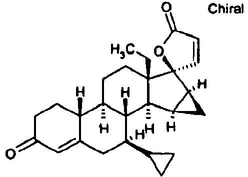 | | | | | | | | |
| 8 | 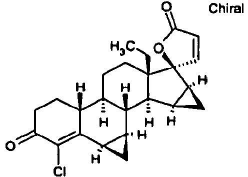 | 1000,0 | 9,8 | 960 | 31,1 | 250 | 32,89 | 130 | 22,88 |
| 9 | 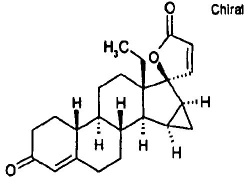 | 1,6 | 56,0 | 3 | 27,8 | 4,7 | 96,19 | 1000 | 5 |
| 10 | 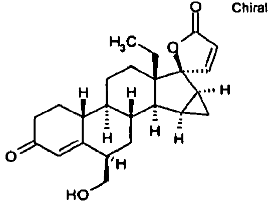 | inaktiv | | 110 | 92,7 | 130 | 17,87 | 160 | 15,58 |
| 12 | 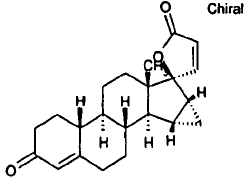 | 0,4 | 75,5 | 100 | 71,4 | 130 | 14,86 | 86 | 61,71 |
| 13 | 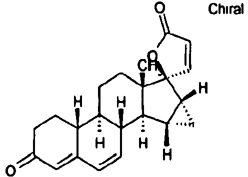 | 150,0 | 37,1 | 12 | 116,2 | 120 | 14,39 | 100 | 47,37 |
| 14A | 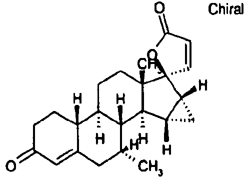 | 16,0 | 86,3 | 3 | 83,0 | 13 | 41,82 | 1,5 | 20,98 |
| 15A | 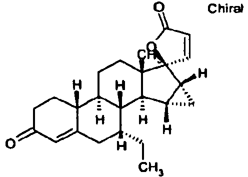 | 92,0 | 39,5 | 12 | 112,3 | 11 | 49,42 | 1000 | 5 |
| 16A | 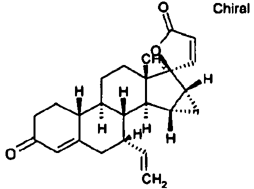 | 140,0 | 59,3 | 95 | 103,1 | 12 | 62,56 | 1000 | 5 |
| 16B | 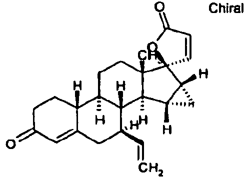 | | | | | | | | |
| 17A | 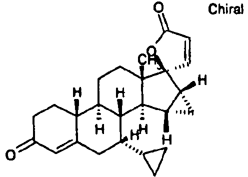 | 970,0 | 71,8 | 89 | 114,8 | 23 | 40,77 | 11 | 33,72 |
| 17B | 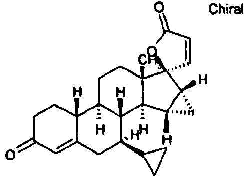 | | | | | | | | |
| 18 | 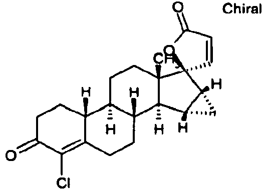 | 810,0 | 25,7 | 830 | 37,3 | 12 | 71,47 | 1000 | 5 |
| 19 | 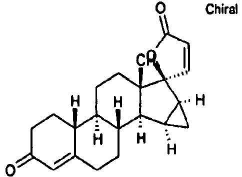 | | | 62 | 92,8 | 5,3 | 87,5 | 1000 | 5 |
| 20 | 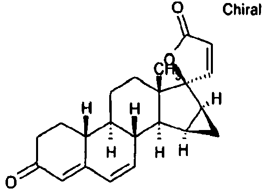 | | | | | | | | |
| 21 | 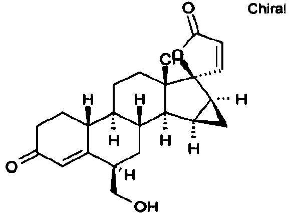 | 780,0 | 21,0 | 160 | 68,0 | 1000 | 5 | 500 | 15,68 |
| 22 | 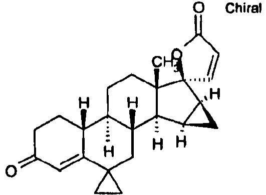 | 1,1 | 126,3 | 49 | 91,2 | 1,7 | 98,61 | 1000 | 5 |
| 23A | 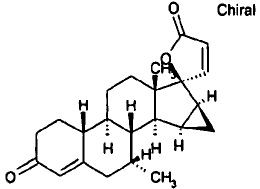 | 21,0 | 140,4 | 290 | 105,3 | 19 | 63,59 | 1000 | 5 |
| 24A | 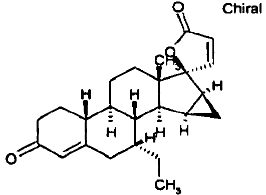 | 140,0 | 42,7 | 160 | 81,1 | 52 | 36,79 | 11 | 22,48 |
| 25A | 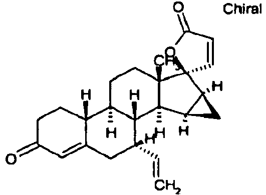 | 560,0 | 46,5 | 910 | 84,1 | 1,83 | 73,32 | *1000* | 5 |
| 27 | 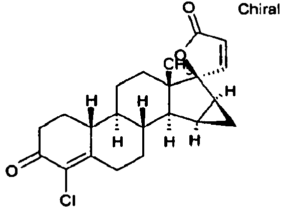 | 330,0 | 49,6 | 1000 | 5,0 | 69,5 | 66,94 | *1000* | 5 |

## Patentansprüche

1. 15,16-Methylen-17-hydroxy-19-nor-21-carbonsäure-Steroid γ-Lacton-Derivat mit der allgemeinen chemischen Formel I: 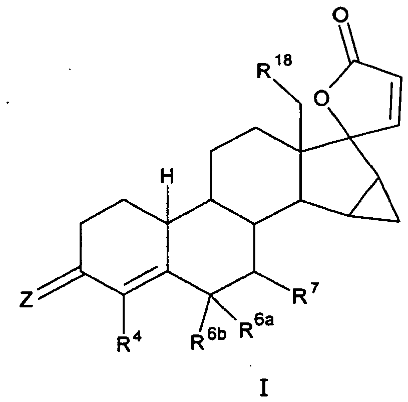 worin
Z ausgewählt ist aus der Gruppe, umfassend Sauerstoff, zwei Wasserstoffatome, NOR' und NNHSO₂R', worin R' Wasserstoff, C₁-C₁₀-Alkyl, Aryl oder C₇-C₂₀-Aralkyl ist,
R⁴ ausgewählt ist aus der Gruppe, umfassend Wasserstoff , Fluor, Chlor oder Brom,
ferner entweder:
R^{6a}, R^{6b} jeweils unabhängig voneinander ausgewählt sind aus der Gruppe, umfassend Wasserstoff, C₁-C₁₀-Alkyl, C₂-C₁₀-Alkenyl und C₂-C₁₀-Alkinyl, oder gemeinsam Methylen oder 1,2-Ethandiyl bilden und
R⁷ ausgewählt ist aus der Gruppe, umfassend Wasserstoff, C₁-C₁₀-Alkyl, C₃-C₆-Cycloalkyl, C₂-C₁₀-Alkenyl und C₂-C₁₀-Alkinyl,
oder:
R^{6a}, R⁷ gemeinsam ein Sauerstoffatom oder Methylen bilden oder unter Bildung einer Doppelbindung zwischen C⁶ und C⁷ entfallen und
R^{6b} ausgewählt ist aus der Gruppe, umfassend Wasserstoff, C₁-C₁₀-Alkyl, C₂-C₁₀-Alkenyl und C₂-C₁₀-Alkinyl und
R¹⁸ Wasserstoff oder C₁-C₃-Alkyl ist,
sowie deren Solvate, Hydrate, Stereoisomere und Salze.

2. 15,16-Methylen-17-hydroxy-19-nor-21-carbonsäure-Steroid γ-Lacton-Derivat nach Anspruch 1, **dadurch gekennzeichnet, dass** Z ausgewählt ist aus der Gruppe, umfassend Sauerstoff, NOR' und NNHSO₂R'.

3. 15,16-Methylen-17-hydroxy-19-nor-21-carbonsäure-Steroid γ-Lacton-Derivat nach Anspruch 1, **dadurch gekennzeichnet, dass** Z für Sauerstoff steht.

4. 15,16-Methylen-17-hydroxy-19-nor-21-carbonsäure-Steroid γ-Lacton-Derivat nach einem der vorstehenden Ansprüche, **dadurch gekennzeichnet, dass** R⁴ Wasserstoff oder Chlor ist.

5. 15,16-Methylen-17-hydroxy-19-nor-21-carbonsäure-Steroid γ-Lacton-Derivat nach einem der vorstehenden Ansprüche, **dadurch gekennzeichnet, dass** R^{6a}, R^{6b} gemeinsam 1,2-Ethandiyl bilden oder jeweils Wasserstoff sind.

6. 15,16-Methylen-17-hydroxy-19-nor-21-carbonsäure-Steroid γ-Lacton-Derivat nach einem der vorstehenden Ansprüche, **dadurch gekennzeichnet, dass** R⁷ ausgewählt ist aus der Gruppe, umfassend Wasserstoff, Methyl, Ethyl und Vinyl.

7. 15,16-Methylen-17-hydroxy-19-nor-21-carbonsäure-Steroid γ-Lacton-Derivat nach einem der Ansprüche 1-4, **dadurch gekennzeichnet, dass** R^{6a}, R⁷ gemeinsam Methylen bilden.

8. 15,16-Methylen-17-hydroxy-19-nor-21-carbonsäure-Steroid γ-Lacton-Derivat nach einem der Ansprüche 1-4, **dadurch gekennzeichnet, dass** R^{6a} und R⁷ unter Bildung einer Doppelbindung zwischen C⁶ und C⁷ entfallen.

9. 15,16-Methylen-17-hydroxy-19-nor-21-carbonsäure-Steroid γ-Lacton-Derivat nach einem der vorstehenden Ansprüche, **dadurch gekennzeichnet, dass** R¹⁸ Wasserstoff oder Methyl ist.

10. 15,16-Methyten-17-hydroxy-19-nor-21-carbonsäure-Steroid γ-Lacton-Derivat einem der vorstehenden Ansprüche, ausgewählt aus der Gruppe
17β-Hydroxy-15α, 16α-methylen-19-nor-17α-pregna-4,20(Z)-dien-3-on-21-carbonsäure γ-Lacton
17β-Hydroxy-15β,16β-methylen-19-nor-17α-pregna-4,20(Z)-dien-3-on-21-carbonsäure γ-Lacton
17β-Hydroxy-7β-methyl-15α,16α-methylen-19-nor-17α-pregna-4,20(Z)-dien-3-on-21-carbonsäure γ-Lacton
17β-Hydroxy-7α-methyl-15β,16β-methylen-19-nor-17α-pregna-4,20(Z)-dien-3-on-21-carbonsäure γ-Lacton
17β-Hydroxy-7β-methyl-15β,16β-methylen-19-nor-17α-pregna-4,20(Z)-dien-3-on-21-carbonsäure γ-Lacton
17β-Hydroxy-7α-ethyl-15α,16α-methylen-19-nor-17α-pregna-4,20(Z)-dien-3-on-21-carbonsäure γ-Lacton
17β-Hydroxy-7β-ethyl-15α,16α-methylen-19-nor-17α-pregna-4,20(Z)-dien-3-on-21-carbonsäure γ-Lacton
7α-Ethyl-17β-hydroxy-15β,16β-methylen-19-nor-17α-pregna-4,20(Z)-dien-3-on-21-carbonsäure γ-Lacton
7β-Ethyl-17β-hydroxy-15β,16β-methylen-19-nor-17α-pregna-4,20(Z)-dien-3-on-21-carbonsäure γ-Lacton
17β-Hydroxy-7α-vinyl-15α,16α-methylen-19-nor-17α-pregna-4, 20(Z)-dien-3-on-21-carbonsäure γ-Lacton
17β-Hydroxy-7β-vinyl-15α,16α-methylen-19-nor-17α-pregna-4,20(Z)-dien-3-on-21-carbonsäure γ-Lacton
17β-Hydroxy-15β,16β-methylen-7α-vinyl-19-nor-17α-pregna-4,20(Z)-dien-3-on-21-carbonsäure γ-Lacton
17β-Hydroxy-15β,16β-methylen-7β-vinyl-19-nor-17α-pregna-4,20(Z)-dien-3-on-21-carbonsäure γ-Lacton
17β-Hydroxy-7α-cyclopropyl-15α,16α-methylen-19-nor-17α-pregna-4,20(Z)-dien-3-on-21-carbonsäure γ-Lacton
17β-Hydroxy-7β-cyclopropyl-15α,16α-methylen-19-nor-17α-pregna-4,20(Z)-dien-3-on-21-carbonsäure γ-Lacton
7α-Cyclopropyl-17β-hydroxy-15β,16β-methylen-19-nor-17α-pregna-4,20(Z)-dien-3-on-21-carbonsäure γ-Lacton
17β-Hydroxy-7β-cyclopropyl-15β, 16β-methylen-19-nor-17α-pregna-4,20(Z)-dien-3-on-21-carbonsäure γ-Lacton
17β-Hydroxy-6-methylen-15α,16α-methylen-19-nor-17α-pregna-4,20(Z)-dien-3-on-21-carbonsäure γ-Lacton
17β-Hydroxy-6-methylen-15β,16β-methylen-19-nor-17α-pregna-4,20(Z)-dien-3-on-21-carbonsäure γ-Lacton
17β-Hydroxy-6α-hydroxymethylen-15α,16α-methylen-19-nor-17α-pregna-4,20(Z)-dien-3-on-21-carbonsäure γ-Lacton
17β-Hydroxy-6β-hydroxymethylen-15α,16α-methylen-19-nor-17α-pregna-4,20(Z)-dien-3-on-21-carbonsäure γ-Lacton
17β-Hydroxy-6α-hydroxymethylen-15β,16β-methylen-19-nor-17α-pregna-4,20(Z)-dien-3-on-21-carbonsäure γ-Lacton
17β-Hydroxy-6β-hydroxymethylen-15β,16β-methylen-19-nor-17α-pregna-4,20(Z)-dien-3-on-21-carbonsäure γ-Lacton
6,6-(1,2-Ethandiyl)-17β-hydroxy-15α,16α-methylen-19-nor-17α-pregna-4,20(Z)-dien-3-on-21-carbonsäure γ-Lacton
6,6-(1,2-Ethandiyl)-17β-hydroxy-15β,16β-methylen-19-nor-17α-pregna-4,20(Z)-dien-3-on-21-carbonsäure γ-Lacton
17β-Hydroxy-6α,7α;15α,16α-bismethylen-19-nor-17α-pregna-4,20(Z)-dien-3-on-21-carbonsäure γ-Lacton
17β-Hydroxy-6β,7β;15α,16α-bismethylen-19-nor-17α-pregna-4,20(Z)-dien-3-on-21 - carbonsäure γ-Lacton
17β-Hydroxy-6α,7α; 15β,16β-bismethylen-19-nor-17α-pregna-4,20(Z)-dien-3-on-21-carbonsäure γ-Lacton
17β-Hydroxy-6β,7β;15β,16β-bismethylen-19-nor-17α-pregna-4,20(Z)-dien-3-on-21-carbonsäure γ-Lacton
17β-Hydroxy-15α,16α-methylen-19-nor-17α-pregna-4,6,20(Z)-trien-3-on-21-carbonsäure γ-Lacton
17β-Hydroxy-15β,16β-methylen-19-nor-17α-pregna-4,6,20(Z)-trien-3-on-21-carbonsäure γ-Lacton
(E/Z)-3-(Hydroxyimino)-17β-hydroxy-15α,16α-methylen-19-nor-17α-pregna-4,20(Z)-dien-21-carbonsäure γ-Lacton
(E/Z)-3-(Hydroxyimino)-17β-hydroxy-15β,16β-methylen-19-nor-17α-pregna-4,20(Z)-dien-21-carbonsäure γ-Lacton
(E/Z)-3-(Hydroxyimino)-17β-hydroxy-7α-methyl-15α,16α-methylen-19-nor-17α-pregna-4,20(Z)-dien-21-carbonsäure γ-Lacton
(E/Z)-3-(Hydroxyimino)-17β-hydroxy-7β-methyl-15α,16α-methylen-19-nor-17α-pregna-4,20(Z)-dien-21-carbonsäure γ-Lacton
(E/Z)-3-(Hydroxyimino)-17β-hydroxy-7α-methyl-15β,16β-methylen-19-nor-17α-pregna-4,20(Z)-dien-21-carbonsäure γ-Lacton
(E/Z)-3-(Hydroxyimino)-17β-hydroxy-7β-methyl-15β,16β-methylen-19-nor-17α-pregna-4,20(Z)-dien-21-carbonsäure γ-Lacton
(E/Z)-3-(Hydroxyimino)-17β-hydroxy-7α-ethyl-15α,16α-methylen-19-nor-17α-pregna-4,20(Z)-dien-21-carbonsäure γ-Lacton
(E/Z)-3-(Hydroxyimino)-17β-hydroxy-7β-ethyl-15α,16α-methylen-19-nor-17α-pregna-4,20(Z)-dien-21-carbonsäure γ-Lacton
(E/Z)-3-(Hydroxyimino)-17β-hydroxy-7α-ethyl-15β,16β-methylen-19-nor-17α-pregna-4,20(Z)-dien-21-carbonsäure γ-Lacton
(E/Z)-3-(Hydroxyimino)-17β-hydroxy-7β-ethyl-15β,16β-methylen-19-nor-17α-pregna-4,20(Z)-dien-21-carbonsäure γ-Lacton
(E/Z)-3-(Hydroxyimino)-17β-hydroxy-7α-vinyl-15α,16α-methylen-19-nor-17α-pregna-4,20(Z)-dien-21-carbonsäure γ-Lacton
(E/Z)-3-(Hydroxyimino)-17β-hydroxy-7β-vinyl-15α,16α-methylen-19-nor-17α-pregna-4,20(Z)-dien-21-carbonsäure γ-Lacton
(E/Z)-3-(Hydroxyimino)-17β-hydroxy-7α-vinyl-15β,16β-methylen-19-nor-17α-pregna-4,20(Z)-dien-21-carbonsäure γ-Lacton
(E/Z)-3-(Hydroxyimino)-17β-hydroxy-7β-vinyl-15β,16β-methylen-19-nor-17α-pregna-4,20(Z)-dien-21-carbonsäure γ-Lacton
(E/Z)-3-(Hydroxyimino)-17β-hydroxy-7α-cyclopropyl-15α,16α-methylen-19-nor-17α-pregna-4,20(Z)-dien-21-carbonsäure γ-Lacton
(E/Z)-3-(Hydroxyimino)-17β-hydroxy-7β-cyclopropyl-15α,16α-methylen-19-nor-17a-pregna-4,20(Z)-dien-21-carbonsäure γ-Lacton
(E/Z)-3-(Hydroxyimino)-17β-hydroxy-7α-cyclopropyl-15β,16β-methylen-19-nor-17α-pregna-4,20(Z)-dien-21-carbonsäure γ-Lacton
(E/Z)-3-(Hydroxyimino)-17β-hydroxy-7β-cyclopropyl-15β,16β-methylen-19-nor-17α-pregna-4,20(Z)-dien-21-carbonsäure γ-Lacton
(E/Z)-3-(Hydroxyimino)-17β-hydroxy-6-methylen-15α,16α-methylen-19-nor-17α-pregna-4,20(Z)-dien-21-carbonsäure γ-Lacton
(E/Z)-3-(Hydroxyimino)-17β-hydroxy-6-methylen-15β,16β-methylen-19-nor-17α-pregna-4,20(Z)-dien-21-carbonsäure γ-Lacton
(E/Z)-3-(Hydroxyimino)-17β-hydroxy-6α-hydroxymethylen-15α,16α-methylen-19-nor-17α-pregna-4,20(Z)-dien-21-carbonsäure γ-Lacton
(E/Z)-3-(Hydroxyimino)-17β-hydroxy-6β-hydroxymethylen-15α,16α-methylen-19-nor-17α-pregna-4,20(Z)-dien-21-carbonsäure γ-Lacton
(E/Z)-3-(Hydroxyimino)-17β-hydroxy-6α-hydroxymethylen-15β,16β-methylen-19-nor-17α-pregna-4,20(Z)-dien-21-carbonsäure γ-Lacton
(E/Z)-3-(Hydroxyimino)-17β-hydroxy-6β-hydroxymethylen-15β,16β-methylen-19-nor-17α-pregna-4,20(Z)-dien-21-carbonsäure γ-Lacton
(E/Z)-3-(Hydroxyimino)-6,6-(1,2-Ethandiyl)-17β-hydroxy-15α,16α-methylen-19-nor-17α-pregna-4,20(Z)-dien-21-carbonsäure γ-Lacton
(E/Z)-3-(Hydroxyimino)-6,6-(1,2-Ethandiyl)-17β-hydroxy-15β,16β-methylen-19-nor-17α-pregna-4,20(Z)-dien-21-carbonsäure γ-Lacton
(E/Z)-3-(Hydroxyimino)-17β-hydroxy-6α,7α;15α,16α-bismethylen-19-nor-17α-pregna-4,20(Z)-dien-21-carbonsäure γ-Lacton
(E/Z)-3-(Hydroxyimino)-17β-hydroxy-6β,7β;15α,16α-bismethylen-19-nor-17α-pregna-4,20(Z)-dien-21-carbonsäure γ-Lacton
(E/Z)-3-(Hydroxyimino)-17β-hydroxy-6α,7α; 15β,16β-bismethylen-19-nor-17α-pregna-4,20(Z)-dien-21-carbonsäure γ-Lacton
(E/Z)-3-(Hydroxyimino)-17β-hydroxy-6β,7β:15β,16β-bismethylen-19-nor-17α-pregna-4,20(Z)-dien-21-carbonsäure γ-Lacton
(E/Z)-3-(Hydroxyimino)-17β-hydroxy-15α,16α-methylen-19-nor-17α-pregna-4,6,20(Z)-trien-21-carbonsäure γ-Lacton
(E/Z)-3-(Hydroxyimino)-17β-hydroxy-15β,16β-methylen-19-nor-17α-pregna-4,6,20(Z)-trien-21-carbonsäure γ-Lacton
17β-Hydroxy-18-methyl-15α,16α-methylen-19-nor-17α-pregna-4,20(Z)-dien-3-on-21-carbonsäure γ-Lacton
17β-Hydroxy-18-methyl-15β,16β-methylen-19-nor-17α-pregna-4,20(Z)-dien-3-on-21-carbonsäure γ-Lacton
17β-Hydroxy-7α-methyl-18-methyl-15α,16α-methylen-19-nor-17α-pregna-4,20(Z)-dien-3-on-21-carbonsäure γ-Lacton
17β-Hydroxy-7β-methyl-18-methyl-15α,16α-methylen-19-nor-17α-pregna-4,20(Z)-dien-3-on-21-carbonsäure γ-Lacton
17β-Hydroxy-7α-methyl-18-methyl-15β,16β-methylen-19-nor-17α-pregna-4,20(Z)-dien-3-on-21-carbonsäure γ-Lacton
17β-Hydroxy-7β-methyl-18-methyl-15β,16β-methylen-19-nor-17α-pregna-4,20(Z)-dien-3-on-21-carbonsäure γ-Lacton
17β-Hydroxy-7α-ethyl-18-methyl-15α,16α-methylen-19-nor-17α-pregna-4,20(Z)-dien-3-on-21-carbonsäure γ-Lacton
17β-Hydroxy-7β-ethyl-18-methyl-15α,16α-methylen-19-nor-17α-pregna-4,20(Z)-dien-3-on-21-carbonsäure γ-Lacton
17β-Hydroxy-7α-ethyl-18-methyl-15β,16β-methylen-19-nor-17α-pregna-4,20(Z)-dien-3-on-21-carbonsäure γ-Lacton
17β-Hydroxy-7β-ethyl-18-methyl-15β,16β-methylen-19-nor-17α-pregna-4,20(Z)-dien-3-on-21-carbonsäure γ-Lacton
17β-Hydroxy-7α-vinyl-18-methyl-15α,16α-methylen-19-nor-17α-pregna-4,20(Z)-dien-3-on-21-carbonsäure γ-Lacton
17β-Hydroxy-7β-vinyl-18-methyl-15α,16α-methylen-19-nor-17α-pregna-4,20(Z)-dien-3-on-21-carbonsäure γ-Lacton
17β-Hydroxy-7α-vinyl-18-methyl-15β,16β-methylen-19-nor-17α-pregna-4,20(Z)-dien-3-on-21-carbonsäure γ-Lacton
17β-Hydroxy-7β-vinyl-18-methyl-15β,16β-methylen-19-nor-17α-pregna-4,20(Z)-dien-3-on-21-carbonsäure γ-Lacton
17β-Hydroxy-7α-cyclopropyl-18-methyl-15α,16α-methylen-19-nor-17α-pregna-4,20(Z)-dien-3-on-21-carbonsäure γ-Lacton
17β-Hydroxy-7β-cyclopropyl-18-methyl-15α,16α-methylen-19-nor-17α-pregna-4,20(Z)-dien-3-on-21-carbonsäure γ-Lacton
7β-Hydroxy-7α-cyclopropyl-18-methyl-15β,16β-methylen-19-nor-17α-pregna-4,20(Z)-dien-3-on-21-carbonsäure γ-Lacton
17β-hydroxy-7β-Cyclopropyl-18-methyl-15β,16β-methylen-19-nor-17α-pregna-4,20(Z)-dien-3-on-21-carbonsäure γ-Lacton
17β-Hydroxy-6-methylen-18-methyl-15α,16α-methylen-19-nor-17α-pregna-4,20(Z)-dien-3-on-21-carbonsäure γ-Lacton
17β-Hydroxy-6-methylen-18-methyl-15β,16β-methylen-19-nor-17α-pregna-4,20(Z)-dien-3-on-21-carbonsäure γ-Lacton
17β-Hydroxy-6α-hydroxymethylen-18-methyl-15α,16α-methylen-19-nor-17α-pregna-4,20(Z)-dien-3-on-21-carbonsäure γ-Lacton
17β-Hydroxy-6β-hydroxymethylen-18-methyl-15α,16α-methylen-19-nor-17α-pregna-4,20(Z)-dien-3-on-21-carbonsäure γ-Lacton
17β-Hydroxy-6α-hydroxymethylen-18-methyl-15β,16β-methylen-19-nor-17α-pregna-4,20(Z)-dien-3-on-21-carbonsäure γ-Lacton
17β-Hydroxy-6β-hydroxymethylen-18-methyl-15β,16β-methylen-19-nor-17α-pregna-4,20(Z)-dien-3-on-21-carbonsäure γ-Lacton
6,6-(1,2-Ethandiyl)-17β-hydroxy-18-methyl-15α,16α-methylen-19-nor-17α-pregna-4,20(Z)-dien-3-on-21-carbonsäure γ-Lacton
6,6-(1,2-Ethandiyl)-17β-hydroxy-18-methyl-15β,16β-methylen-19-nor-17α-pregna-4,20(Z)-dien-3-on-21-carbonsäure γ-Lacton
17β-Hydroxy-6α,7α;15α,16α-bismethylen-18-methyl-19-nor-17α-pregna-4,20(Z)-dien-3-on-21-carbonsäure γ-Lacton
17β-Hydroxy-6β,7β,15α,16α-bismethylen-18-methyl-19-nor-17α-pregna-4,20(Z)-dien-3-on-21-carbonsäure γ-Lacton
17β-Hydroxy-6α,7α;15β,16β-bismethylen-18-methyl-19-nor-17α-pregna-4,20(Z)-dien-3-on-21-carbonsäure γ-Lacton
17β-Hydroxy-6β,7β;15β,16β-bismethylen-18-methyl-19-nor-17α-pregna-4,20(Z)-dien-3-on-21-carbonsäure γ-Lacton
17β-Hydroxy-18-methyl-15α,16α-methylen-19-nor-17α-pregna-4,6,20(Z)-trien-3-on-21-carbonsäure γ-Lacton
17β-Hydroxy-18-methyl-15β,16β-methylen-19-nor-17α-pregna-4,6,20(Z)-trien-3-on-21-carbonsäure γ-Lacton
(E/Z)-3-(Hydroxyimino)-17β-hydroxy-18-methyl-15α,16α-methylen-19-nor-17α-pregna-4,20(Z)-dien-21-carbonsäure γ-Lacton
(E/Z)-3-(Hydroxyimino)-17β-hydroxy-18-methyl-15β,16β-methylen-19-nor-17α-pregna-4,20(Z)-dien-21-carbonsäure γ-Lacton
(E/Z)-3-(Hydroxyimino)-17β-hydroxy-7α-methyl-18-methyl-15α,16α-methylen-19-nor-17α-pregna-4,20(Z)-dien-21-carbonsäure γ-Lacton
(E/Z)-3-(Hydroxyimino)-17β-hydroxy-7β-methyl-18-methyl-15α,16α-methylen-19-nor-17α-pregna-4,20(Z)-dien-21-carbonsäure γ-Lacton
(E/Z)-3-(Hydroxyimino)-17β-hydroxy-7α-methyl-18-methyl-15β,16β-methylen-19-nor-17α-pregna-4,20(Z)-dien-21-carbonsäure γ-Lacton
(E/Z)-3-(Hydroxyimino)-17β-hydroxy-7β-methyl-18-methyl-15β,16β-methylen-19-nor-17α-pregna-4,20(Z)-dien-21-carbonsäure γ-Lacton
(E/Z)-3-(Hydroxyimino)-17β-hydroxy-7α-ethyl-18-methyl-15α,16α-methylen-19-nor-17α-pregna-4,20(Z)-dien-21-carbonsäure γ-Lacton
(E/Z)-3-(Hydroxyimino)-17β-hydroxy-7β-ethyl-18-methyl-15α,16α-methylen-19-nor-17α-pregna-4,20(Z)-dien-21-carbonsäure γ-Lacton
(E/Z)-3-(Hydroxyimino)-17β-hydroxy-7α-ethyl-18-methyl-15β,16β-methylen-19-nor-17α-pregna-4,20(Z)-dien-21-carbonsäure γ-Lacton
(E/Z)-3-(Hydroxyimino)-17β-hydroxy-7β-ethyl-18-methyl-15β,16β-methylen-19-nor-17α-pregna-4,20(Z)-dien-21-carbonsäure γ-Lacton
(E/Z)-3-(Hydroxyimino)-17β-hydroxy-7α-vinyl-18-methyl-15α,16α-methylen-19-nor-17α-pregna-4,20(Z)-dien-21-carbonsäure γ-Lacton
(E/Z)-3-(Hydroxyimino)-17β-hydroxy-7β-vinyl-18-methyl-15α,16α-methylen-19-nor-17α-pregna-4,20(Z)-dien-21-carbonsäure γ-Lacton
(E/Z)-3-(Hydroxyimino)-17β-hydroxy-7α-vinyl-18-methyl-15β,16β-methylen-19-nor-17α-pregna-4,20(Z)-dien-21-carbonsäure γ-Lacton
(E/Z)-3-(Hydroxyimino)-17β-hydroxy-7β-vinyl-18-methyl-15β,16β-methylen-19-nor-17α-pregna-4,20(Z)-dien-21-carbonsäure γ-Lacton
(E/Z)-3-(Hydroxyimino)-17β-hydroxy-7α-cyclopropyl-18-methyl-15α,16α-methylen-19-nor-17α-pregna-4,20(Z)-dien-21-carbonsäure γ-Lacton
(E/Z)-3-(Hydroxyimino)-17β-hydroxy-7β-cyclopropyl-18-methyl-15α,16α-methylen-19-nor-17α-pregna-4,20(Z)-dien-21-carbonsäure γ-Lacton
(E/Z)-3-(Hydroxyimino)-17β-hydroxy-7α-cyclopropyl-18-methyl-15β,16β-methylen-19-nor-17α-pregna-4,20(Z)-dien-21-carbonsäure γ-Lacton
(E/Z)-3-(Hydroxyimino)-17β-hydroxy-7β-cyclopropyl-18-methyl-15β,16β-methylen-19-nor-17α-pregna-4,20(Z)-dien-21-carbonsäure γ-Lacton
(E/Z)-3-(Hydroxyimino)-17β-hydroxy-6-methylen-18-methyl-15α,16α-methylen-19-nor-17α-pregna-4,20(Z)-dien-21-carbonsäure γ-Lacton
(E/Z)-3-(Hydroxyimino)-17β-hydroxy-6-methylen-18-methyl-15β,16β-methylen-19-nor-17α-pregna-4,20(Z)-dien-21-carbonsäure γ-Lacton
(E/Z)-3-(Hydroxyimino)-17β-hydroxy-6α-hydroxymethylen-18-methyl-15α,16α-methylen-19-nor-17α-pregna-4,20(Z)-dien-21-carbonsäure γ-Lacton
(E/Z)-3-(Hydroxyimino)-17β-hydroxy-6β-hydroxymethylen-18-methyl-15α,16α-methylen-19-nor-17α-pregna-4,20(Z)-dien-21-carbonsäure γ-Lacton
(E/Z)-3-(Hydroxyimino)-17β-hydroxy-6α-hydroxymethylen-18-methyl-15β,16β-methylen-19-nor-17α-pregna-4,20(Z)-dien-21-carbonsäure γ-Lacton
(E/Z)-3-(Hydroxyimino)-17β-hydroxy-6β-hydroxymethylen-18-methyl-15β,16β-methylen-19-nor-17α-pregna-4,20(Z)-dien-21-carbonsäure γ-Lacton
(E/Z)-3-(Hydroxyimino)-6,6-(1,2-Ethandiyl)-17β-hydroxy-18-methyl-15α,16α-methylen-19-nor-17α-pregna-4,20(Z)-dien-21-carbonsäure γ-Lacton
(E/Z)-3-(Hydroxyimino)-6,6-(1,2-Ethandiyl)-17β-hydroxy-18-methyl-15β,16β-methylen-19-nor-17α-pregna-4,20(Z)-dien-21-carbonsäure γ-Lacton
(E/Z)-3-(Hydroxyimino)-17β-hydroxy-6α,7α; 15α,16α-bismethylen-18-methyl-19-nor-17α-pregna-4,20(Z)-dien-21-carbonsäure γ-Lacton
(E/Z)-3-(Hydroxyimino)-17β-hydroxy-6β,7β;15α,16β-bismethylen-18-methyl-19-nor-17α-pregna-4,20(Z)-dien-21-carbonsäure γ-Lacton
(E/Z)-3-(Hydroxyimino)-17β-hydroxy-6α,7α;15β,16β-bismethylen-18-methyl-19-nor-17α-pregna-4,20(Z)-dien-21-carbonsäure γ-Lacton
(E/Z)-3-(Hydroxyimino)-17β-hydroxy-6β,7β;15β,16β-bismethylen-18-methyl-19-nor-17α-pregna-4,20(Z)-dien-21-carbonsäure γ-Lacton
(E/Z)-3-(Hydroxyimino)-17β-hydroxy-18-methyl-15α,16α-methylen-19-nor-17α-pregna-4,6,20(Z)-trien-21-carbonsäure γ-Lacton
(E/Z)-3-(Hydroxyimino)-17β-hydroxy-18-methyl-15β,16β-methylen-19-nor-17α-pregna-4,6,20(Z)-trien-21-carbonsäure γ-Lacton
6β,7β;15β,16β-Bismethylen-4-chlor-17β-hydroxy-18-methyl-19-nor-17α-pregna-4,20(Z)-dien-3-on-21-carbonsäure γ-Lacton
4-Chlor-17β-hydroxy-15α,16α-methylen-19-nor-17α-pregna-4,20(Z)-dien-3-on-21-carbonsäure γ-Lacton
4-Chlor-17β-hydroxy-15β,16β-methylen-19-nor-17α-pregna-4,20(Z)-dien-3-on-21-carbonsäure γ-Lacton

11. 15,16-Methylen-17-hydroxy-19-nor-21-carbonsäure-Steroid γ-Lacton-Derivat nach einem der vorstehenden Ansprüche zur oralen Kontrazeption und zur Behandlung von prä-, peri- und postmenopausalen Beschwerden.

12. Verwendung des 15,16-Methylen-17-hydroxy-19-nor-21-carbonsäure-Steroid γ-Lacton-Derivats nach einem der Ansprüche 1 bis 11 zur Herstellung eines Arzneimittels zur oralen Kontrazeption und zur Behandlung von prä-, peri- und postmenopausalen Beschwerden.

13. Verwendung nach Anspruch 12, **dadurch gekennzeichnet, dass** das Arzneimittel gestagene, antimineralcorticoide und neutrale bis leicht androgene Wirkung aufweist.

14. Arzneimittel enthaltend mindestens ein 15,16-Methylen-17-hydroxy-19-nor-21-carbonsäure-Steroid γ-Lacton-Derivat nach einem der Ansprüche 1 bis 11 sowie mindestens einen geeigneten pharmazeutisch unbedenklichen Zusatzstoff.

15. Arzneimittel nach Anspruch 14, enthaltend außerdem mindestens ein Estrogen.

16. Arzneimittel nach Anspruch 15, **dadurch gekennzeichnet, dass** das Estrogen Ethinylestradiol ist.

17. Arzneimittel nach Anspruch 15, **dadurch gekennzeichnet, dass** das natürliche Estrogen Estradiolvalerat ist.

18. Arzneimittel nach Anspruch 15, **dadurch gekennzeichnet, dass** das Estrogen ein natürliches Estrogen ist.

19. Arzneimittel nach Anspruch 18, **dadurch gekennzeichnet, dass** das natürliche Estrogen Estradiol ist.

20. Arzneimittel nach Anspruch 18, **dadurch gekennzeichnet, dass** das natürliche Estrogen ein konjugiertes Estrogen ist.

21. Verwendung des 15,16-Methylen-17-hydroxy-19-nor-21-carbonsäure-Steroid γ-Lacton-Derivats nach einem der Ansprüche 1-9 zur Herstellung eines Arzneimittels zur intrauterinen Anwendung.

22. Verwendung nach Anspruch 21 zur Herstellung eines intrauterinen Systems (IUS).

23. Arzneimittel enthaltend mindestens ein 15,16-Methylen-17-hydroxy-19-nor-21-carbonsäure-Steroid γ-Lacton-Derivat nach einem der Ansprüche 1-9 sowie mindestens einen geeigneten pharmazeutisch unbedenklichen Trägerstoff, **dadurch gekennzeichnet, dass** es zur intrauterinen Anwendung hergerichtet ist.

24. Arzneimittel nach Anspruch 23, **dadurch gekennzeichnet, dass** es ein intrauterines System ist.

## Claims

1. 15,16-Methylene-17-hydroxy-19-nor-21-carboxylic acid-steroid γ-lactone derivative with the general chemical formula I: 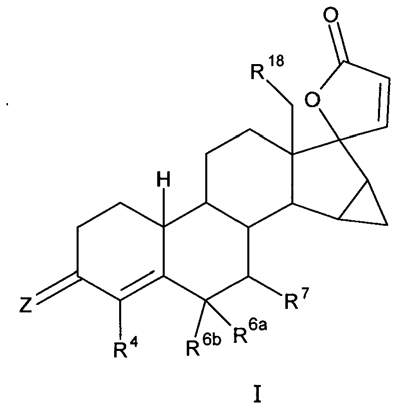 in which
Z is selected from the group comprising oxygen, two hydrogen atoms, NOR' and NNHSO₂R', in which R' is hydrogen, C₁-C₁₀-alkyl, aryl or C₇-C₂₀-aralkyl,
R⁴ is selected from the group comprising hydrogen, fluorine, chlorine or bromine,
furthermore either:
R^{6a}, R^{6b} in each case independently of one another, are selected from the group comprising hydrogen, C₁-C₁₀-alkyl, C₂-C₁₀-alkenyl and C₂-C₁₀-alkynyl, or together form methylene or 1,2-ethanediyl and
R⁷ is selected from the group comprising hydrogen, C₁-C₁₀-alkyl, C₃-C₆-cycloalkyl, C₂-C₁₀-alkenyl and C₂-C₁₀-alkynyl,
or:
R^{6a}, R⁷ together form an oxygen atom or methylene or drop out with formation of a double bond between C⁶ and C⁷ and
R^{6b} is selected from the group comprising hydrogen, C₁-C₁₀-alkyl, C₂-C₁₀-alkenyl and C₂-C₁₀-alkynyl and
R¹⁸ is hydrogen or C₁-C₃-alkyl,
and their solvates, hydrates, stereoisomers and salts.

2. 15,16-Methylene-17-hydroxy-19-nor-21-carboxylic acid-steroid γ-lactone derivative according to Claim 1, **characterized in that** Z is selected from the group comprising oxygen, NOR' and NNHSO₂R'.

3. 15,16-Methylene-17-hydroxy-19-nor-21-carboxylic acid-steroid γ-lactone derivative according to Claim 1, **characterized in that** Z stands for oxygen.

4. 15,16-Methylene-17-hydroxy-19-nor-21-carboxylic acid-steroid γ-lactone derivative according to one of the preceding claims, **characterized in that** R⁴ is hydrogen or chlorine.

5. 15,16-Methylene-17-hydroxy-19-nor-21-carboxylic acid-steroid γ-lactone derivative according to one of the preceding claims, **characterized in that** R^{6a}, R^{6b} together form 1,2-ethanediyl or are each hydrogen.

6. 15,16-Methylene-17-hydroxy-19-nor-21-carboxylic acid-steroid γ-lactone derivative according to one of the preceding claims, **characterized in that** R⁷ is selected from the group comprising hydrogen, methyl, ethyl and vinyl.

7. 15,16-Methylene-17-hydroxy-19-nor-21-carboxylic acid-steroid γ-lactone derivative according to one of Claims 1 - 4, **characterized in that** R^{6a}, R⁷ together form methylene.

8. 15,16-Methylene-17-hydroxy-19-nor-21-carboxylic acid-steroid γ-lactone derivative according to one of Claims 1 - 4, **characterized in that** R^{6a} and R⁷ drop out with formation of a double bond between C⁶ and C⁷.

9. 15,16-Methylene-17-hydroxy-19-nor-21-carboxylic acid-steroid γ-lactone derivative according to one of the preceding claims, **characterized in that** R¹⁸ is hydrogen or methyl.

10. 15,16-Methylene-17-hydroxy-19-nor-21-carboxylic acid-steroid γ-lactone derivative according to one of the preceding claims, selected from the group
17β-Hydroxy-15α,16α-methylene-19-nor-17α-pregna-4,20(Z)-dien-3-one-21-carboxylic acid γ-lactone
17β-Hydroxy-15β,16β-methylene-19-nor-17α-pregna-4,20(Z)-dien-3-one-21-carboxylic acid γ-lactone
17β-Hydroxy-7β-methyl-15α,16α-methylene-19-nor-17α-pregna-4,20(Z)-dien-3-one-21-carboxylic acid γ-lactone
17β-Hydroxy-7α-methyl-15β,16β-methylene-19-nor-17α-pregna-4,20(Z)-dien-3-one-21-carboxylic acid γ-lactone
17β-Hydroxy-7β-methyl-15β,16β-methylene-19-nor-17α-pregna-4,20(Z)-dien-3-one-21-carboxylic acid γ-lactone
17β-Hydroxy-7α-ethyl-15α,16α-methylene-19-nor-17α-pregna-4,20(Z)-dien-3-one-21-carboxylic acid γ-lactone
17β-Hydroxy-7β-ethyl-15α,16α-methylene-19-nor-17α-pregna-4,20(Z)-dien-3-one-21-carboxylic acid γ-lactone
7α-Ethyl-17β-hydroxy-15β,16β-methylene-19-nor-17α-pregna-4,20(Z)-dien-3-one-21-carboxylic acid γ-lactone
7β-Ethyl-17β-hydroxy-15β,16β-methylene-19-nor-17α-pregna-4,20(Z)-dien-3-one-21-carboxylic acid γ-lactone
17β-Hydroxy-7α-vinyl-15α,16α-methylene-19-nor-17α-pregna-4,20(Z)-dien-3-one-21-carboxylic acid γ-lactone
17β-Hydroxy-7β-vinyl-15α,16α-methylene-19-nor-17α-pregna-4,20(Z)-dien-3-one-21-carboxylic acid γ-lactone
17β-Hydroxy-15β,16β-methylene-7α-vinyl-19-nor-17α-pregna-4,20(Z)-dien-3-one-21-carboxylic acid γ-lactone
17β-Hydroxy-15β,16β-methylene-7β-vinyl-19-nor-17α-pregna-4,20(Z)-dien-3-one-21-carboxylic acid γ-lactone
17β-Hydroxy-7α-cyclopropyl-15α,16α-methylene-19-nor-17α-pregna-4,20(Z)-dien-3-one-21-carboxylic acid γ-lactone
17β-Hydroxy-7β-cyclopropyl-15α,16α-methylene-19-nor-17α-pregna-4,20(Z)-dien-3-one-21-carboxylic acid γ-lactone
7α-Cyclopropyl-17β-hydroxy-15β,16β-methylene-19-nor-17α-pregna-4,20(Z)-dien-3-one-21-carboxylic acid γ-lactone
17β-Hydroxy-7β-cyclopropyl-15β,16β-methylene-19-nor-17α-pregna-4,20(Z)-dien-3-one-21-carboxylic acid γ-lactone
17β-Hydroxy-6-methylene-15α,16α-methylene-19-nor-17α-pregna-4,20(Z)-dien-3-one-21-carboxylic acid γ-lactone
17β-Hydroxy-6-methylene-15β,16β-methylene-19-nor-17α-pregna-4,20(Z)-dien-3-one-21-carboxylic acid γ-lactone
17β-Hydroxy-6α-hydroxymethylene-15α,16α-methylene-19-nor-17α-pregna-4,20(Z)-dien-3-one-21-carboxylic acid γ-lactone
17β-Hydroxy-6β-hydroxymethylene-15α,16α-methylene-19-nor-17α-pregna-4,20(Z)-dien-3-one-21-carboxylic acid γ-lactone
17β-Hydroxy-6α-hydroxymethylene-15β,16β-methylene-19-nor-17α-pregna-4,20(Z)-dien-3-one-21-carboxylic acid γ-lactone
17β-Hydroxy-6β-hydroxymethylene-15β,16β-methylene-19-nor-17α-pregna-4,20(Z)-dien-3-one-21-carboxylic acid γ-lactone
6,6-(1,2-Ethanediyl)-17β-hydroxy-15α,16α-methylene-19-nor-17α-pregna-4,20(Z)-dien-3-one-21-carboxylic acid γ-lactone
6,6-(1,2-Ethanediyl)-17β-hydroxy-15β,16β-methylene-19-nor-17α-pregna-4,20(Z)-dien-3-one-21-carboxylic acid γ-lactone
17β-Hydroxy-6α,7α;15α,16α-bismethylene-19-nor-17α-pregna-4,20(Z)-dien-3-one-21-carboxylic acid γ-lactone
17β-Hydroxy-6β,7β;15α,16α-bismethylene-19-nor-17α-pregna-4,20(Z)-dien-3-one-21-carboxylic acid γ-lactone
17β-Hydroxy-6α,7α; 15β,16β-bismethylene-19-nor-17α-pregna-4,20(Z)-dien-3-one-21-carboxylic acid γ-lactone
17β-Hydroxy-6β,7β;15β,16β-bismethylene-19-nor-17α-pregna-4,20(Z)-dien-3-one-21-carboxylic acid γ-lactone
17β-Hydroxy-15α,16α-methylene-19-nor-17α-pregna-4,6,20(Z)-trien-3-one-21-carboxylic acid γ-lactone
17β-Hydroxy-15β,16β-methylene-19-nor-17α-pregna-4,6,20(Z)-trien-3-one-21-carboxylic acid γ-lactone
(E/Z)-3-(Hydroxyimino)-17β-hydroxy-15α,16α-methylene-19-nor-17α-pregna-4,20(Z)-diene-21-carboxylic acid γ-lactone
(E/Z)-3-(Hydroxyimino)-17β-hydroxy-15β,16β-methylene-19-nor-17α-pregna-4,20(Z)-diene-21-carboxylic acid γ-lactone
(E/Z)-3-(Hydroxyimino)-17β-hydroxy-7α-methyl-15α,16α-methylene-19-nor-17α-pregna-4,20(Z)-diene-21-carboxylic acid γ-lactone
(E/Z)-3-(Hydroxyimino)-17α-hydroxy-7α-methyl-15α,16α-methylene-19-nor-17α-pregna-4,20(Z)-diene-21-carboxylic acid γ-lactone
(E/Z)-3-(Hydroxyimino)-17β-hydroxy-7α-methyl-15β,16β-methylene-19-nor-17α-pregna-4,20(Z)-diene-21-carboxylic acid γ-lactone
(E/Z)-3-(Hydroxyimino)-17β-hydroxy-7β-methyl-15β,16β-methylene-19-nor-17α-pregna-4,20(Z)-diene-21-carboxylic acid γ-lactone
(E/Z)-3-(Hydroxyimino)-17β-hydroxy-7α-ethyl-15α,16α-methylene-19-nor-17α-pregna-4,20(Z)-diene-21-carboxylic acid γ-lactone
(E/Z)-3-(Hydroxyimino)-17β-hydroxy-7β-ethyl-15α,16α-methylene-19-nor-17α-pregna-4,20(Z)-diene-21-carboxylic acid γ-lactone
(E/Z)-3-(Hydroxyimino)-17β-hydroxy-7β-ethyl-15β,16β-methylene-19-nor-17α-pregna-4,20(Z)-diene-21-carboxylic acid γ-lactone
(E/Z)-3-(Hydroxyimino)-17β-hydroxy-7β-ethyl-15β,16β-methylene-19-nor-17α-pregna-4,20(Z)-diene-21-carboxylic acid γ-lactone
(E/Z)-3-(Hydroxyimino)-17β-hydroxy-7α-vinyl-15α,16α-methylene-19-nor-17α-pregna-4,20(Z)-diene-21-carboxylic acid γ-lactone
(E/Z)-3-(Hydroxyimino)-17β-hydroxy-7β-vinyl-15α,16α-methylene-19-nor-17α-pregna-4,20(Z)-diene-21-carboxylic acid γ-lactone
(E/Z)-3-(Hydroxyimino)-17β-hydroxy-7α-vinyl-15β,16β-methylene-19-nor-17α-pregna-4,20(Z)-diene-21-carboxylic acid γ-lactone
(E/Z)-3-(Hydroxyimino)-17β-hydroxy-7β-vinyl-15β,16β-methylene-19-nor-17α-pregna-4,20(Z)-diene-21-carboxylic acid γ-lactone
(E/Z)-3-(Hydroxyimino)-17β-hydroxy-7α-cyclopropyl-15α,16α-methylene-19-nor-17α-pregna-4,20(Z)-diene-21-carboxylic acid γ-lactone
(E/Z)-3-(Hydroxyimino)-17β-hydroxy-7β-cyclopropyl-15α,16α-methylene-19-nor-17α-pregna-4,20(Z)-diene-21-carboxylic acid γ-lactone
(E/Z)-3-(Hydroxyimino)-17β-hydroxy-7α-cyclopropyl-15β,16β-methylene-19-nor-17α-pregna-4,20(Z)-diene-21-carboxylic acid γ-lactone
(E/Z)-3-(Hydroxyimino)-17β-hydroxy-7β-cyclopropyl-15β,16β-methylene-19-nor-17α-pregna-4,20(Z)-diene-21-carboxylic acid γ-lactone
(E/Z)-3-(Hydroxyimino)-17β-hydroxy-6-methylene-15α,16α-methylene-19-nor-17α-pregna-4,20(Z)-diene-21-carboxylic acid γ-lactone
(E/Z)-3-(Hydroxyimino)-17α-hydroxy-6-methylene-15β,16β-methylene-19-nor-17α-pregna-4,20(Z)-diene-21-carboxylic acid γ-lactone
(E/Z)-3-(Hydroxyimino)-17β-hydroxy-6α-hydroxymethylene-15α,16α-methylene-19-nor-17α-pregna-4,20(Z)-diene-21-carboxylic acid γ-lactone
(E/Z)-3-(Hydroxyimino)-17β-hydroxy-6β-hydroxymethylene-15α,16α-methylene-19-nor-17α-pregna-4,20(Z)-diene-21-carboxylic acid γ-lactone
(E/Z)-3-(Hydroxyimino)-17α-hydroxy-6α-hydroxymethylene-15α,16α-methylene-19-nor-17α-pregna-4,20(Z)-diene-21-carboxylic acid γ-lactone
(E/Z)-3-(Hydroxyimino)-17β-hydroxy-6β-hydroxymethylene-15β,16β-methylene-19-nor-17α-pregna-4,20(Z)-diene-21-carboxylic acid γ-lactone
(E/Z)-3-(Hydroxyimino)-6,6-(1,2-ethanediyl)-17α-hydroxy-15α,16α-methylene-19-nor-17α-pregna-4,20(Z)-diene-21-carboxylic acid γ-lactone
(E/Z)-3-(Hydroxyimino)-6,6-(1,2-ethanediyl)-17β-hydroxy-15β,16β-methylene-19-nor-17α-pregna-4,20(Z)-diene-21-carboxylic acid γ-lactone
(E/Z)-3-(Hydroxyimino)-17α-hydroxy-6α,7α; 15α,16α-bismethylene-19-nor-17α-pregna-4,20(Z)-diene-21-carboxylic acid γ-lactone
(E/Z)-3-(Hydroxyimino)-17β-hydroxy-6β,7β;15α,16α-bismethylene-19-nor-17α-pregna-4,20(Z)-diene-21-carboxylic acid γ-lactone
(E/Z)-3-(Hydroxyimino)-17β-hydroxy-6α,7α; 15β,16β-bismethylene-19-nor-17α-pregna-4,20(Z)-diene-21-carboxylic acid γ-lactone
(E/Z)-3-(Hydroxyimino)-17β-hydroxy-6β,7β:15β,16β-bismethylene-19-nor-17α-pregna-4,20(Z)-diene-21-carboxylic acid γ-lactone
(E/Z)-3-(Hydroxyimino)-17β-hydroxy-15α,16α-methylene-19-nor-17α-pregna-4,6,20(Z)-triene-21-carboxylic acid γ-lactone
(E/Z)-3-(Hydroxyimino)-17β-hydroxy-15β,16β-methylene-19-nor-17a-pregna-4,6,20(Z)-triene-21-carboxylic acid γ-lactone
17β-Hydroxy-18-methyl-15α,16α-methylene-19-nor-17α-pregna-4,20(Z)-dien-3-one-21-carboxylic acid γ-lactone
17β-Hydroxy-18-methyl-15β,16β-methylene-19-nor-17α-pregna-4,20(Z)-dien-3-one-21-carboxylic acid γ-lactone
17β-Hydroxy-7α-methyl-18-methyl-15α,16α-methylene-19-nor-17α-pregna-4,20(Z)-dien-3-one-21-carboxylic acid γ-lactone
17β-Hydroxy-7β-methyl-18-methyl-15α,16α-methylene-19-nor-17α-pregna-4,20(Z)-dien-3-one-21-carboxylic acid γ-lactone
17β-Hydroxy-7α-methyl-18-methyl-15β,16β-methylene-19-nor-17α-pregna-4,20(Z)-dien-3-one-21-carboxylic acid γ-lactone
17β-Hydroxy-7β-methyl-18-methyl-15β,16β-methylene-19-nor-17α-pregna-4,20(Z)-dien-3-one-21-carboxylic acid γ-lactone
17β-Hydroxy-7α-ethyl-18-methyl-15α,16α-methylene-19-nor-17α-pregna-4,20(Z)-dien-3-one-21-carboxylic acid γ-lactone
17β-Hydroxy-7β-ethyl-18-methyl-15α,16α-methylene-19-nor-17α-pregna-4,20(Z)-dien-3-one-21-carboxylic acid γ-lactone
17β-Hydroxy-7α-ethyl-18-methyl-15β,16β-methylene-19-nor-17α-pregna-4,20(Z)-dien-3-one-21-carboxylic acid γ-lactone
17β-Hydroxy-7β-ethyl-18-methyl-15β,16β-methylene-19-nor-17α-pregna-4,20(Z)-dien-3-one-21-carboxylic acid γ-lactone
17β-Hydroxy-7α-vinyl-18-methyl-15α,16α-methylene-19-nor-17α-pregna-4,20(Z)-dien-3-one-21-carboxylic acid γ-lactone
17β-Hydroxy-7β-vinyl-18-methyl-15α,16α-methylene-19-nor-17a-pregna-4,20(Z)-dien-3-one-21-carboxylic acid γ-lactone
17β-Hydroxy-7α-vinyl-18-methyl-15β,16β-methylene-19-nor-17α-pregna-4,20(Z)-dien-3-one-21-carboxylic acid γ-lactone
17β-Hydroxy-7β-vinyl-18-methyl-15β,16β-methylene-19-nor-17α-pregna-4,20(Z)-dien-3-one-21-carboxylic acid γ-lactone
17β-Hydroxy-7α-cyclopropyl-18-methyl-15α,16α-methylene-19-nor-17α-pregna-4,20(Z)-dien-3-one-21-carboxylic acid γ-lactone
17β-Hydroxy-7β-cyclopropyl-18-methyl-15α,16α-methylene-19-nor-17α-pregna-4,20(Z)-dien-3-one-21-carboxylic acid γ-lactone
7β-Hydroxy-7α-cyclopropyl-18-methyl-15β,16β-methylene-19-nor-17α-pregna-4,20(Z)-dien-3-one-21-carboxylic acid γ-lactone
17β-Hydroxy-7β-cyclopropyl-18-methyl-15β,16β-methylene-19-nor-17α-pregna-4,20(Z)-dien-3-one-21-carboxylic acid γ-lactone
17β-Hydroxy-6-methylene-18-methyl-15α,16α-methylene-19-nor-17α-pregna-4,20(Z)-dien-3-one-21-carboxylic acid γ-lactone
17β-Hydroxy-6-methylene-18-methyl-15β,16β-methylene-19-nor-17α-pregna-4,20(Z)-dien-3-one-21-carboxylic acid γ-lactone
17β-Hydroxy-6α-hydroxymethylene-18-methyl-15α,16α-methylene-19-nor-17α-pregna-4,20(Z)-dien-3-one-21-carboxylic acid γ-lactone
17β-Hydroxy-6β-hydroxymethylene-18-methyl-15α,16α-methylene-19-nor-17α-pregna-4,20(Z)-dien-3-one-21-carboxylic acid γ-lactone
17β-Hydroxy-6α-hydroxymethylene-18-methyl-15β,16β-methylene-19-nor-17α-pregna-4,20(Z)-dien-3-one-21-carboxylic acid γ-lactone
17β-Hydroxy-6β-hydroxymethylene-18-methyl-15β,16β-methylene-19-nor-17α-pregna-4,20(Z)-dien-3-one-21-carboxylic acid γ-lactone
6,6-(1,2-ethanediyl)-17β-Hydroxy-18-methyl-15α,16α-methylene-19-nor-17α-pregna-4,20(Z)-dien-3-one-21-carboxylic acid γ-lactone
6,6-(1,2-ethanediyl)-17β-Hydroxy-18-methyl-15β,16β-methylene-19-nor-17α-pregna-4,20(Z)-dien-3-one-21-carboxylic acid γ-lactone
17β-Hydroxy-6α,7α;15α,16α-bismethylene-18-methyl-19-nor-17α-pregna-4,20(Z)-dien-3-one-21-carboxylic acid γ-lactone
17β-Hydroxy-6β,7β,15α,16α-bismethylene-18-methyl-19-nor-17α-pregna-4,20(Z)-dien-3-one-21-carboxylic acid γ-lactone
17β-Hydroxy-6α,7α;15β,16β-bismethylene-18-methyl-19-nor-17α-pregna-4,20(Z)-dien-3-one-21-carboxylic acid γ-lactone
17β-Hydroxy-6β,7β;15β,16β-bismethylene-18-methyl-19-nor-17α-pregna-4,20(Z)-dien-3-one-21-carboxylic acid γ-lactone
17β-Hydroxy-18-methyl-15α,16α-methylene-19-nor-17α-pregna-4,6,20(Z)-trien-3-one-21-carboxylic acid γ-lactone
17β-Hydroxy-18-methyl-15β,16β-methylene-19-nor-17α-pregna-4,6,20(Z)-trien-3-one-21-carboxylic acid γ-lactone
(E/Z)-3-(Hydroxyimino)-17β-hydroxy-18-methyl-15α,16α-methylene-19-nor-17α-pregna-4,20(Z)-diene-21-carboxylic acid γ-lactone
(E/Z)-3-(Hydroxyimino)-17β-hydroxy-18-methyl-15β,16β-methylene-19-nor-17a-pregna-4,20(Z)-diene-21-carboxylic acid γ-lactone
(E/Z)-3-(Hydroxyimino)-17β-hydroxy-7α-methyl-18-methyl-15α,16α-methylene-19-nor-17α-pregna-4,20(Z)-diene-21-carboxylic acid γ-lactone
(E/Z)-3-(Hydroxyimino)-17β-hydroxy-7β-methyl-18-methyl-15α,16α-methylene-19-nor-17α-pregna-4,20(Z)-diene-21-carboxylic acid γ-lactone
(E/Z)-3-(Hydroxyimino)-17β-hydroxy-7α-methyl-18-methyl-15β,16β-methylene-19-nor-17α**-**pregna-4,20(Z)-diene-21-carboxylic acid γ-lactone
(E/Z)-3-(Hydroxyimino)-17β-hydroxy-7β-methyl-18-methyl-15β,16β-methylene-19-nor-17α-pregna-4,20(Z)-diene-21-carboxylic acid γ-lactone
(E/Z)-3-(Hydroxyimino)-17β-hydroxy-7α-ethyl-18-methyl-15α,16α-methylene-19-nor-17α-pregna-4,20(Z)-diene-21-carboxylic acid γ-lactone
(E/Z)-3-(Hydroxyimino)-17β-hydroxy-7β-ethyl-18-methyl-15α,16α-methylene-19-nor-17α-pregna-4,20(Z)-diene-21-carboxylic acid γ-lactone
(E/Z)-3-(Hydroxyimino)-17α-hydroxy-7α-ethyl-18-methyl-15β,16β-methylene-19-nor-17α-pregna-4,20(Z)-diene-21-carboxylic acid γ-lactone
(E/Z)-3-(Hydroxyimino)-17β-hydroxy-7β-ethyl-18-methyl-15β,16β-methylene-19-nor-17a-pregna-4,20(Z)-diene-21-carboxylic acid γ-lactone
(E/Z)-3-(Hydroxyimino)-17β-hydroxy-7α-vinyl-18-methyl-15α,16α-methylene-19-nor-17α-pregna-4,20(Z)-diene-21-carboxylic acid γ-lactone
(E/Z)-3-(Hydroxyimino)-17β-hydroxy-7β-vinyl-18-methyl-15α,16α-methylene-19-nor-17α-pregna-4,20(Z)-diene-21-carboxylic acid γ-lactone
(E/Z)-3-(Hydroxyimino)-17α-hydroxy-7α-vinyl-18-methyl-15β,16β-methylene-19-nor-17α-pregna-4,20(Z)-diene-21-carboxylic acid γ-lactone
(E/Z)-3-(Hydroxyimino)-17β-hydroxy-7β-vinyl-18-methyl-15β,16β-methylene-19-nor-17a-pregna-4,20(Z)-diene-21-carboxylic acid γ-lactone
(E/Z)-3-(Hydroxyimino)-17β-hydroxy-7α-cyclopropyl-18-methyl-15α,16α-methylene-19-nor-17α-pregna-4,20(Z)-diene-21-carboxylic acid γ-lactone
(E/Z)-3-(Hydroxyimino)-17β-hydroxy-7β-cyclopropyl-18-methyl-15α,16α-methylene-19-nor-17α-pregna-4,20(Z)-diene-21-carboxylic acid γ-lactone
(E/Z)-3-(Hydroxyimino)-17β-hydroxy-7α-cyclopropyl-18-methyl-15β,16β-methylene-19-nor-17α-pregna-4,20(Z)-diene-21-carboxylic acid γ-lactone
(E/Z)-3-(Hydroxyimino)-17β-hydroxy-7β-cyclopropyl-18-methyl-15β,16β-methylene-19-nor-17α-pregna-4,20(Z)-diene-21-carboxylic acid γ-lactone
(E/Z)-3-(Hydroxyimino)-17β-hydroxy-6-methylene-18-methyl-15α,16α-methylene-19-nor-17α-pregna-4,20(Z)-diene-21-carboxylic acid γ-lactone
(E/Z)-3-(Hydroxyimino)-17β-hydroxy-6-methylene-18-methyl-15β,16β-methylene-19-nor-17α-pregna-4,20(Z)-diene-21-carboxylic acid γ-lactone
(E/Z)-3-(Hydroxyimino)-17β-hydroxy-6α-hydroxymethylene-18-methyl-15α,16α-methylene-19-nor-17α-pregna-4,20(Z)-diene-21-carboxylic acid γ-lactone
(E/Z)-3-(Hydroxyimino)-17β-hydroxy-6β-hydroxymethylene-18-methyl-15α,16α-methylene-19-nor-17α-pregna-4,20(Z)-diene-21-carboxylic acid γ-lactone
(E/Z)-3-(Hydroxyimino)-17α-hydroxy-6α-hydroxymethylene-18-methyl-15β,16β-methylene-19-nor-17α-pregna-4,20(Z)-diene-21-carboxylic acid γ-lactone
(E/Z)-3-(Hydroxyimino)-17α-hydroxy-6α-hydroxymethylene-18-methyl-15β,16β-methylene-19-nor-17α-pregna-4,20(Z)-diene-21-carboxylic acid γ-lactone
(E/Z)-3-(Hydroxyimino)-6,6-(1,2-ethanediyl)-17β-hydroxy-18-methyl-15α,16α-methylene-19-nor-17α-pregna-4,20(Z)-diene-21-carboxylic acid γ-lactone
(E/Z)-3-(Hydroxyimino)-6,6-(1,2-ethanediyl)-17β-hydroxy-18-methyl-15β,16β-methylene-19-nor-17α-pregna-4,20(Z)-diene-21-carboxylic acid γ-lactone
(E/Z)-3-(Hydroxyimino)-17β-hydroxy-6α,7α;15α,16α-bismethylene-18-methyl-19-nor-17α-pregna-4,20(Z)-diene-21-carboxylic acid γ-lactone
(E/Z)-3-(Hydroxyimino)-17β-hydroxy-6β,7β;15α,16α-bismethylene-18-methyl-19-nor-17α-pregna-4,20(Z)-diene-21-carboxylic acid γ-lactone
(E/Z)-3-(Hydroxyimino)-17β-hydroxy-6α,7α;15β,16β-bismethylene-18-methyl-19-nor-17α-pregna-4,20(Z)-diene-21-carboxylic acid γ-lactone
(E/Z)-3-(Hydroxyimino)-17β-hydroxy-6β,7β;15β,16β-bismethylene-18-methyl-19-nor-17a-pregna-4,20(Z)-diene-21-carboxylic acid γ-lactone
(E/Z)-3-(Hydroxyimino)-17β-hydroxy-18-methyl-15α,16α-methylene-19-nor-17α-pregna-4,6,20(Z)-triene-21-carboxylic acid γ-lactone
(E/Z)-3-(Hydroxyimino)-17α-hydroxy-18-methyl-15β,16β-methylene-19-nor-17α-pregna-4,6,20(Z)-triene-21-carboxylic acid γ-lactone
6β,7β;15β,16β-Bismethylene-4-chloro-17β-hydroxy-18-methyl-19-nor-17α-pregna-4,20(Z)-dien-3-one-21-carboxylic acid γ-lactone
4-Chloro-17β-hydroxy-15α,16α-methylene-19-nor-17α-pregna-4,20(Z)-dien-3-one-21-carboxylic acid γ-lactone
4-Chloro-17β-hydroxy-15β,16β-methylene-19-nor-17α-pregna-4,20(Z)-dien-3-one-21-carboxylic acid γ-lactone

11. 15,16-Methylene-17-hydroxy-19-nor-21-carboxylic acid-steroid γ-lactone derivative according to one of the preceding claims for oral contraception and for the treatment of pre-, peri- and postmenopausal complaints.

12. Use of the 15,16-methylene-17-hydroxy-19-nor-21-carboxylic acid-steroid γ-lactone derivative according to one of Claims 1 to 11 for the production of a medicinal product for oral contraception and for the treatment of pre-, peri-and postmenopausal complaints.

13. Use according to Claim 12, **characterized in that** the medicinal product has progestational, antimineralocorticoid and neutral to slightly androgenic action.

14. Medicinal product containing at least one 15,16-methylene-17-hydroxy-19-nor-21-carboxylic acid-steroid γ-lactone derivative according to one of Claims 1 to 11 and at least one suitable pharmaceutically harmless additive.

15. Medicinal product according to Claim 14, additionally containing at least one estrogen.

16. Medicinal product according to Claim 15, **characterized in that** the estrogen is ethinylestradiol.

17. Medicinal product according to Claim 15, **characterized in that** the natural estrogen is estradiol valerate.

18. Medicinal product according to Claim 15, **characterized in that** the estrogen is a natural estrogen.

19. Medicinal product according to Claim 18, **characterized in that** the natural estrogen is estradiol.

20. Medicinal product according to Claim 18, **characterized in that** the natural estrogen is a conjugated estrogen.

21. Use of the 15,16-methylene-17-hydroxy-19-nor-21-carboxylic acid-steroid γ-lactone derivative according to any of Claims 1-9 for the production of a medicinal product for intrauterine use.

22. Use according to Claim 21 for the production of an intrauterine system (IUS).

23. Medicinal product containing at least one 15,16-methylene-17-hydroxy-19-nor-21-carboxylic acidsteroid γ-lactone derivative according to any of Claims 1-9 and at least one suitable pharmaceutically harmless additive, **characterized in that** it is designed for intrauterine use.

24. Medicinal product according to Claim 23, **characterized in that** it is an intrauterine system.

## Revendications

1. Dérivé de γ-lactone de 15,16-méthylène-17-hydroxy-19-nor-21-carboxy-stéroïde correspondant à la formule chimique générale I suivante : 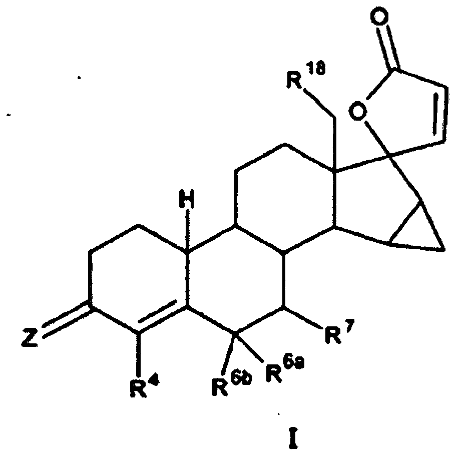 dans laquelle
Z est choisi dans l'ensemble comprenant un atome d'oxygène, deux atomes d'hydrogène, NOR' et NNHSO₂R', où R' est un atome d'hydrogène, un groupe alkyle en C₁-C₁₀, aryle ou aralkyle en C₇-C₂₀,
R⁴ est choisi dans le groupe comprenant un atome d'hydrogène, de fluor, chlore ou brome,
en outre soit :
R^{6a}, R^{6b} sont choisis chacun, indépendamment l'un de l'autre, dans l'ensemble comprenant un atome d'hydrogène, des groupes alkyle en C₁-C₁₀, alcényle en C₂-C₁₀ et alcynyle en C₂-C₁₀, ou forment ensemble un groupe méthylène ou 1,2-éthanediyle et
R⁷ est choisi dans l'ensemble comprenant un atome d'hydrogène, des groupes alkyle en C₁-C₁₀, cycloalkyle en C₃-C₆, alcényle en C₂-C₁₀ et alcynyle en C₂-C₁₀,
soit :
R^{6a}, R⁷ ensemble représentent un atome d'oxygène ou forment un groupe méthylène ou sont absents avec formation d'une double liaison entre C⁶ et C⁷ et
R^{6b} est choisi dans l'ensemble comprenant un atome d'hydrogène, des groupes alkyle en C₁-C₁₀, alcényle en C₂-C₁₀ et alcynyle en C₂-C₁₀ et
R¹⁸ est un atome d'hydrogène ou un groupe alkyle en C₁-C₃,
ainsi que produits de solvatation, hydrates, stéréoisomères et sels d'un tel dérivé.

2. Dérivé de γ-lactone de 15,16-méthylène-17-hydroxy-19-nor-21-carboxy-stéroïde selon la revendication 1, **caractérisé en ce que** Z est choisi dans l'ensemble comprenant un atome d'oxygène, NOR' et NNHSO₂R'.

3. Dérivé de γ-lactone de 15,16-méthylène-17-hydroxy-19-nor-21-carboxy-stéroïde selon la revendication 1, **caractérisé en ce que** Z représente un atome d'oxygène.

4. Dérivé de γ-lactone de 15,16-méthylène-17-hydroxy-19-nor-21-carboxy-stéroïde selon l'une quelconque des revendications précédentes, **caractérisé en ce que** R⁴ est un atome d'hydrogène ou de chlore.

5. Dérivé de γ-lactone de 15,16-méthylène-17-hydroxy-19-nor-21-carboxy-stéroïde selon l'une quelconque des revendications précédentes, **caractérisé en ce que** R^{6a}, R^{6b} forment ensemble le groupe 1,2-éthanediyle ou représentent chacun un atome d'hydrogène.

6. Dérivé de γ-lactone de 15,16-méthylène-17-hydroxy-19-nor-21-carboxy-stéroïde selon l'une quelconque des revendications précédentes, **caractérisé en ce que** R⁷ est choisi dans l'ensemble comprenant un atome d'hydrogène, les groupes méthyle, éthyle et vinyle.

7. Dérivé de γ-lactone de 15,16-méthylène-17-hydroxy-19-nor-21-carboxy-stéroïde selon l'une quelconque des revendications 1 à 4, **caractérisé en ce que** R^{6a} R⁷ forment ensemble un groupe méthylène.

8. Dérivé de γ-lactone de 15,16-méthylène-17-hydroxy-19-nor-21-carboxy-stéroïde selon l'une quelconque des revendications 1 à 4, **caractérisé en ce que** R^{6a} et R⁷ sont absents avec formation d'une double liaison entre C⁶ et C⁷.

9. Dérivé de γ-lactone de 15,16-méthylène-17-hydroxy-19-nor-21-carboxy-stéroïde selon l'une quelconque des revendications précédentes, **caractérisé en ce que** R¹⁸ est un atome d'hydrogène ou le groupe méthyle.

10. Dérivé de γ-lactone de 15,16-méthylène-17-hydroxy-19-nor-21-carboxy-stéroïde selon l'une quelconque des revendications précédentes, choisi dans le groupe
γ-lactone d'acide 17β-hydroxy-15α,16α-méthylène-19-nor-17α-prégna-4,20(Z)-dién-3-one-21-carboxylique
γ-lactone d'acide 17β-hydroxy-15β,16β-méthylène-19-nor-17α-prégna-4,20(Z)-dién-3-one-21-carboxylique
γ-lactone d'acide 17β-hydroxy-7β-méthyl-15α,16α-méthylène-19-nor-17α-prégna-4,20(Z)-dién-3-one-21-carboxylique
γ-lactone d'acide 17β-hydroxy-7α-méthyl-15β,16β-méthylène-19-nor-17α-prégna-4,20(Z)-dién-3-one-21-carboxylique
γ-lactone d'acide 17β-hydroxy-7β-méthyl-15β,16β-méthylène-19-nor-17α-prégna-4,20(Z)-dién-3-one-21-carboxylique
γ-lactone d'acide 17β-hydroxy-7α-éthyl-15α,16α-méthylène-19-nor-17α-prégna-4,20(Z)-dién-3-one-21-carboxylique
γ-lactone d'acide 17β-hydroxy-7β-éthyl-15α,16α-méthylène-19-nor-17α-prégna-4,20(Z)-dién-3-one-21-carboxylique
γ-lactone d'acide 7α-éthyl-17β-hydroxy-15β,16β-méthylène-19-nor-17α-prégna-4,20(Z)-dién-3-one-21-carboxylique
γ-lactone d'acide 7β-éthyl-17β-hydroxy-15β,16β-méthylène-19-nor-17α-prégna-4,20(Z)-dién-3-one-21-carboxylique
γ-lactone d'acide 17β-hydroxy-7α-vinyl-15α,16α-méthylène-19-nor-17α-prégna-4,20(Z)-dién-3-one-21-carboxylique
γ-lactone d'acide 17β-hydroxy-7β-vinyl-15α,16α-méthylène-19-nor-17α-prégna-4,20(Z)-dién-3-one-21-carboxylique
γ-lactone d'acide 17 β-hydroxy-15β,16β-méthylène-7α-vinyl-19-nor-17α-prégna-4,20(Z)-dién-3-one-21-carboxylique
γ-lactone d'acide 17β-hydroxy-15β,16β-méthylène-7β-vinyl-19-nor-17α-prégna-4,20(Z)-dién-3-one-21-carboxylique
γ-lactone d'acide 17β-hydroxy-7α-cyclopropyl-15α,16α-méthylène-19-nor-17α-prégna-4,20(Z)-dién-3-one-21-carboxylique
γ-lactone d'acide 17β-hydroxy-7β-cyclopropyl-15α,16α-méthylène-19-nor-17α-prégna-4,20(Z)-dién-3-one-21-carboxylique
γ-lactone d'acide 7α-cyclopropyl-17β-hydroxy-15α,16β-méthylène-19-nor-17α-prégna-4,20(Z)-dién-3-one-21-carboxylique
γ-lactone d'acide 17β-hydroxy-7β-cyclopropyl-15β,16β-méthylène-19-nor-17α-prégna-4,20(Z)-dién-3-one-21-carboxylique
γ-lactone d'acide 17β-hydroxy-6-méthylène-15α,16α-méthylène-19-nor-17α-prégna-4,20(Z)-dién-3-one-21-carboxylique
γ-lactone d'acide 17β-hydroxy-6-méthylène-15β,16β-méthylène-19-nor-17α-prégna-4,20(Z)-dién-3-one-21-carboxylique
γ-lactone d'acide 17β-hydroxy-6α-hydroxyméthylène-15α,16α-méthylène-19-nor-17a-prégna-4,20(Z)-dién-3-one-21-carboxylique
γ-lactone d'acide 17β-hydroxy-6β-hydroxyméthylène-15α,16α-méthylène-19-nor-17α-prégna-4,20(Z)-dién-3-one-21-carboxylique
γ-lactone d'acide 17β-hydroxy-6α-hydroxyméthylène-15β,16β-méthylène-19-nor-17α-prégna-4,20(Z)-dién-3-one-21-carboxylique
γ-lactone d'acide 17β-hydroxy-6β-hydroxyméthylène-15β,16β-méthylène-19-nor-17α-prégna-4,20(Z)-dién-3-one-21-carboxylique
γ-lactone d'acide 6,6-(1,2-éthanediyl)-17β-hydroxy-15α,16α-méthylène-19-nor-17α-prégna-4,20(Z)-dién-3-one-21-carboxylique
γ-lactone d'acide 6,6-(1,2-éthanediyl)-17β-hydroxy-15β,16β-méthylène-19-nor-17α-prégna-4,20(Z)-dién-3-one-21-carboxylique
γ-lactone d'acide 17β-hydroxy-6α,7α;15α,16α-bisméthylène-19-nor-17α-prégna-4,20(Z)-dién-3-one-21-carboxylique
γ-lactone d'acide 17β-hydroxy-6β,7β;15α,16α-bisméthylène-19-nor-17α-prégna-4,20(Z)-dién-3-one-21-carboxylique
γ-lactone d'acide 17β-hydroxy-6α,7α;15β,16β-bisméthylène-19-nor-17α-prégna-4,20(Z)-dién-3-one-21-carboxylique
γ-lactone d'acide 17β-hydroxy-6β,7β;15β,16β-bisméthylène-19-nor-17a-prégna-4,20(Z)-dién-3-one-21-carboxylique
γ-lactone d'acide 17β-hydroxy-15α,16α-méthylène-19-nor-17α-prégna-4,6,20(Z)-trién-3-one-21-carboxylique γ-lactone d'acide 17β-hydroxy-15β,16β-méthylène-19-nor-17α-prégna-4,6,20(Z)-trién-3-one-21-carboxylique γ-lactone d'acide (E/Z)-3-(hydroxyimino)-17β-hydroxy-15α,16α-méthylène-19-nor-17α-prégna-4,20(Z)diène-21-carboxylique
γ-lactone d'acide (E/Z)-3-(hydroxyimino)-17β-hydroxy-15β,16β-méthylène-19-nor-17α-prégna-4,20(Z)diène-21-carboxylique
γ-lactone d'acide (E/Z)-3-(hydroxyimino)-17β-hydroxy-7α-méthyl-15α,16α-méthylène-19-nor-17α-prégna-4,20(Z)-diène-21-carboxylique
γ-lactone d'acide (E/Z)-3-(hydroxyimino)-17β-hydroxy-7β-méthyl-15α,16α-méthylène-19-nor-17α-prégna-4,20(Z)-diène-21-carboxylique
γ-lactone d'acide (E/Z)-3-(hydroxyimino)-17β-hydroxy-7α-méthyl-15β,16β-méthylène-19-nor-17α-prégna-4,20(Z)-diène-21-carboxylique
γ-lactone d'acide (E/Z)-3-(hydroxyimino)-17β-hydroxy-7β-méthyl-15β,16β-méthylène-19-nor-17α-prégna-4,20(Z)-diène-21-carboxylique
γ-lactone d'acide (E/Z)-3-(hydroxyimino)-17β-hydroxy-7α-éthyl-15α,16α-méthylène-19-nor-17α-prégna-4,20(Z)-diène-21-carboxylique
γ-lactone d'acide (E/Z)-3-(hydroxyimino)-17β-hydroxy-7β-éthyl-15α,16α-méthylène-19-nor-17α-prégna-4,20(Z)-diène-21-carboxylique
γ-lactone d'acide (E/Z)-3-(hydroxyimino)-17β-hydroxy-7α-éthyl-15β,16β-méthylène-19-nor-17α-prégna-4,20(Z)-diène-21-carboxylique
γ-lactone d'acide (E/Z)-3-(hydroxyimino)-17β-hydroxy-7β-éthyl-15β,16β-méthylène-19-nor-17α-prégna-4,20(Z)-diène-21-carboxylique
γ-lactone d'acide (E/Z)-3-(hydroxyimino)-17β-hydroxy-7α-vinyl-15α,16α-méthylène-19-nor-17α-prégna-4,20(Z)-diène-21-carboxylique
γ-lactone d'acide (E/Z)-3-(hydroxyimino)-17β-hydroxy-7β-vinyl-15α,16α-méthylène-19-nor-17α-prégna-4,20(Z)-diène-21-carboxylique
γ-lactone d'acide (E/Z)-3-(hydroxyimino)-17β-hydroxy-7α-vinyl-15β,16β-méthylène-19-nor-17α-prégna-4,20(Z)-diène-21-carboxylique
γ-lactone d'acide (E/Z)-3-(hydroxyimino)-17β-hydroxy-7β-vinyl-15β,16β-méthylène-19-nor-17α-prégna-4,20(Z)-diène-21-carboxylique
γ-lactone d'acide (E/Z)-3-(hydroxyimino)-17β-hydroxy-7α-cyclopropyl-15α,16α-méthylène-19-nor-17α-prégna-4,20(Z)-diène-21-carboxylique
γ-lactone d'acide (E/Z)-3-(hydroxyimino)-17β-hydroxy-7β-cyclopropyl-15a,16a-méthylène-19-nor-17a-prégna-4,20(Z)-diène-21-carboxylique
γ-lactone d'acide (E/Z)-3-(hydroxyimino)-17β-hydroxy-7α-cyclopropyl-15β,16β-méthylène-19-nor-17α-prégna-4,20(Z)-diène-21-carboxylique
γ-lactone d'acide (E/Z)-3-(hydroxyimino)-17β-hydroxy-7β-cyclopropyl-15β,16β-méthylène-19-nor-17α-prégna-4,20(Z)-diène-21-carboxylique
γ-lactone d'acide (E/Z)-3-(hydroxyimino)-17β-hydroxy-6-méthylène-15α,16α-méthylène-19-nor-17α-prégna-4,20(Z)-diène-21-carboxylique
γ-lactone d'acide (E/Z)-3-(hydroxyimino)-17β-hydroxy-6-méthylène-15β,16β-méthylène-19-nor-17α-prégna-4,20(Z)-diène-21-carboxylique
γ-lactone d'acide (E/Z)-3-(hydroxyimino)-17β-hydroxy-6α-hydroxyméthylène-15α,16α-méthylène-19-nor-17α-prégna-4,20(Z)-diène-21-carboxylique
γ-lactone d'acide (E/Z)-3-(hydroxyimino)-17β-hydroxy-6β-hydroxyméthylène-15α,16α-méthylène-19-nor-17α-prégna-4,20(Z)-diène-21-carboxylique
γ-lactone d'acide (E/Z)-3-(hydroxyimino)-17β-hydroxy-6α-hydroxyméthylène-15β,16β-méthylène-19-nor-17α-prégna-4,20(Z)-diène-21-carboxylique
γ-lactone d'acide (E/Z)-3-(hydroxyimino)-17β-hydroxy-6β-hydroxyméthylène-15β,16β-méthylène-19-nor-17α-prégna-4,20(Z)-diène-21-carboxylique
γ-lactone d'acide (E/Z)-3-(hydroxyimino)-6,6-(1,2-éthanediyl)-17β-hydroxy-15α,16α-méthylène-19-nor-17α-prégna-4,20(Z)-diène-21-carboxylique
γ-lactone d'acide (E/Z)-3-(hydroxyimino)-6,6-(1,2-éthanediyl)-17β-hydroxy-15β,16β-méthylène-19-nor-17α-prégna-4,20(Z)-diène-21-carboxylique
γ-lactone d'acide (E/Z)-3-(hydroxyimino)-17β-hydroxy-6α,7α;15α,16α-bisméthylène-19-nor-17α-prégna-4,20(Z)-diène-21-carboxylique
γ-lactone d'acide (E/Z)-3-(hydroxyimino)-17β-hydroxy-6β,7β;15α,16α-bisméthylène-19-nor-17α-prégna-4,20(Z)-diène-21-carboxylique
γ-lactone d'acide (E/Z)-3-(hydroxyimino)-17β-hydroxy-6α,7α;15β,16β-bisméthylène-19-nor-17α-prégna-4,20(Z)-diène-21-carboxylique
γ-lactone d'acide (E/Z)-3-(hydroxyimino)-17β-hydroxy-6β,7β:15β,16β-bisméthylène-19-nor-17α-prégna-4,20 (Z) - diène-21-carboxylique
γ-lactone d'acide (E/Z)-3-(hydroxyimino)-17β-hydroxy-15α,16α-méthylène-19-nor-17α-prégna-4,6,20(Z)-triène-21-carboxylique
γ-lactone d'acide (E/Z)-3-(hydroxyimino)-17β-hydroxy-15β,16β-méthylène-19-nor-17α-prégna-4,6,20(Z)-triène-21-carboxylique
γ-lactone d'acide 17β-hydroxy-18-méthyl-15α,16α-méthylène-19-nor-17α-prégna-4,20(Z)-dién-3-one-21-carboxylique
γ-lactone d'acide 17β-hydroxy-18-méthyl-15β,16β-méthylène-19-nor-17α-prégna-4,20(Z)-dién-3-one-21-carboxylique
γ-lactone d'acide 17β-hydroxy-7α-méthyl-18-méthyl-15α,16α-méthylène-19-nor-17α-prégna-4,20(Z)-dién-3-one-21-carboxylique
γ-lactone d'acide 17β-hydroxy-7β-méthyl-18-méthyl-15α,16α-méthylène-19-nor-17α-pregna-4,20(Z)dién-3-one-21-carboxylique
γ-lactone d'acide 17β-hydroxy-7α-méthyl-18-méthyl-15β,16β-méthyléne-19-nor-17α-prégna-4,20(Z)dién-3-one-21-carboxylique
γ-lactone d'acide 17β-hydroxy-7β-méthyl-18-méthyl-15β,16β-méthylène-19-nor-17α-pregna-4,20(Z)-dién-3-one-21-carboxylique
γ-lactone d'acide 17β-hydroxy-7α-éthyl-18-méthyl-15α,16α-méthylène-19-nor-17α-prégna-4,20(Z)dién-3-one-21-carboxylique
γ-lactone d'acide 17β-hydroxy-7β-éthyl-18-méthyl-15α,16α-méthylène-19-nor-17α-prégna-4,20(Z)-dién-3-one-21-carboxylique
γ-lactone d'acide 17β-hydroxy-7α-éthyl-18-méthyl-15α,16α-méthylène-19-nor-17α-prégna-4,20(Z)dién-3-one-21-carboxylique
γ-lactone d'acide 17β-hydroxy-7β-éthyl-18-méthyl-15β,16β-méthylène-19-nor-17α-prégna-4,20(Z)dién-3-one-21-carboxylique
γ-lactone d'acide 17β-hydroxy-7α-vinyl-18-méthyl-15α,16α-méthylène-19-nor-17α-prégna-4,20(Z)-dién-3-one-21-carboxylique
γ-lactone d'acide 17β-hydroxy-7β-vinyl-18-méthyl-15α,16α-méthylène-19-nor-17α-prégna-4,20(Z)dién-3-one-21-carboxylique
γ-lactone d'acide 17β-hydroxy-7α-vinyl-18-méthyl-15β,16β-méthylène-19-nor-17α-prégna-4,20(Z)-dién-3-one-21-carboxylique
γ-lactone d'acide 17β-hydroxy-7β-vinyl-18-méthyl-15β,16β-méthylène-19-nor-17α-prégna-4,20(Z)-dién-3-one-21-carboxylique
γ-lactone d'acide 17β-hydroxy-7α-cyclopropyl-18-méthyl-15α,16α-méthylène-19-nor-17α-prégna-4,20(Z)-dién-3-one-21-carboxylique
γ-lactone d'acide 17β-hydroxy-7β-cyclopropyl-18-méthyl-15α,16α-méthylène-19-nor-17α-prégna-4,20(Z)-dién-3-one-21-carboxylique
γ-lactone d'acide 7β-hydroxy-7α-cyclopropyl-18-méthyl-15β,16β-méthylène-19-nor-17α-prégna-4,20(Z)-dién-3-one-21-carboxylique
γ-lactone d'acide 17β-hydroxy-7β-cyclopropyl-18-méthyl-15β,16β-méthylène-19-nor-17α-prégna-4,20(Z)-dién-3-one-21-carboxylique
γ-lactone d'acide 17β-hydroxy-6-méthylène-18-méthyl-15α,16α-méthylène-19-nor-17α-prégna-4,20(Z)dién-3-one-21-carboxylique
γ-lactone d'acide 17β-hydroxy-6-méthylène-18-méthyl-15β,16β-méthylène-19-nor-17α-prégna-4,20(Z)dién-3-one-21-carboxylique
γ-lactone d'acide 17β-hydroxy-6α-hydroxyméthylène-18-méthyl-15α,16α-méthylène-19-nor-17α-prégna-4,20(Z)-dién-3-one-21-carboxylique
γ-lactone d'acide 17β-hydroxy-6β-hydroxyméthylène-18-méthyl-15α,16α-méthylène-19-nor-17α-prégna-4,20(Z)-dién-3-one-21-carboxylique
γ-lactone d'acide 17β-hydroxy-6α-hydroxyméthylène-18-méthyl-15β,16β-méthylène-19-nor-17α-prégna-4,20(Z)-dién-3-one-21-carboxylique
γ-lactone d'acide 17β-hydroxy-6β-hydroxyméthylène-18-méthyl-15β,16β-méthylène-19-nor-17α-prégna-4,20(Z)-dién-3-one-21-carboxylique
γ-lactone d'acide 6,6-(1,2-éthanediyl)-17β-hydroxy-18-méthyl-15α,16α-méthylène-19-nor-17α-prégna-4,20(Z)-dién-3-one-21-carboxylique
γ-lactone d'acide 6,6-(1,2-éthanediyl)-17β-hydroxy-18-méthyl-15β,16β-méthylène-19-nor-17α-prégna-4,20(Z)-dién-3-one-21-carboxylique
γ-lactone d'acide 17β-hydroxy-6α,7α;15α,16α-bisméthylène-18-méthyl-19-nor-17α-prégna-4,20(Z)dién-3-one-21-carboxylique
γ-lactone d'acide 17β-hydroxy-6β,7β,15α,16α-bisméthylène-18-méthyl-19-nor-17α-prégna-4,20(Z)-dién-3-one-21-carboxylique
γ-lactone d'acide 17β-hydroxy-6α,7α;15β,16β-bisméthylène-18-méthyl-19-nor-17α-prégna-4,20(Z)dién-3-one-21-carboxylique
γ-lactone d'acide 17β-hydroxy-6β,7β;15β,16β-bisméthylène-18-méthyl-19-nor-17α-prégna-4,20(Z)dién-3-one-21-carboxylique
γ-lactone d'acide 17β-hydroxy-18-méthyl-15α,16α-méthylène-19-nor-17α-prégna-4,6,20(Z)-trién-3-one-21-carboxylique
γ-lactone d'acide 17β-hydroxy-18-méthyl-15β,16β-méthylène-19-nor-17α-prégna-4,6,20(Z)-trién-3-one-21-carboxylique
γ-lactone d'acide (E/Z)-3-(hydroxyimino)-17β-hydroxy-18-méthyl-15α,16α-méthylène-19-nor-17α-prégna-4,20(Z)-diène-21-carboxylique
γ-lactone d'acide (E/Z)-3-(hydroxyimino)-17β-hydroxy-18-méthyl-15β,16β-méthylène-19-nor-17α-prégna-4,20(Z)-diène-21-carboxylique
γ-lactone d'acide (E/Z)-3-(hydroxyimino)-17β-hydroxy-7α-méthyl-18-méthyl-15α,16α-méthylène-19-nor-17α-prégna-4,20(Z)-diène-21-carboxylique
γ-lactone d'acide (E/Z)-3-(hydroxyimino)-17β-hydroxy-7β-méthyl-18-méthyl-15α,16α-méthylène-19-nor-17α-prégna-4,20(Z)-diène-21-carboxylique
γ-lactone d'acide (E/Z)-3-(hydroxyimino)-17β-hydroxy-7α-méthyl-18-méthyl-15β,16β-méthylène-19-nor-17α-prégna-4,20(Z)-diène-21-carboxylique
γ-lactone d'acide (E/Z)-3-(hydroxyimino)-17β-hydroxy-7β-méthyl-18-méthyl-15β,16β-méthylène-19-nor-17α-prégna-4,20(Z)-diène-21-carboxylique
γ-lactone d'acide (E/Z)-3-(hydroxyimino)-17β-hydroxy-7α-éthyl-18-méthyl-15α,16α-méthylène-19-nor-17α-prégna-4,20(Z)-diène-21-carboxylique
γ-lactone d'acide (E/Z)-3-(hydroxyimino)-17β-hydroxy-7β-éthyl-18-méthyl-15α,16α-méthylène-19-nor-17α-prégna-4,20(Z)-diène-21-carboxylique
γ-lactone d'acide (E/Z)-3-(hydroxyimino)-17β-hydroxy-7α-éthyl-18-méthyl-15β,16β-méthylène-19-nor-17α-prégna-4,20(Z)-diène-21-carboxylique
γ-lactone d'acide (E/Z)-3-(hydroxyimino)-17β-hydroxy-7β-éthyl-18-méthyl-15β,16β-méthylène-19-nor-17α-prégna-4,20(Z)-diène-21-carboxylique
γ-lactone d'acide (E/Z)-3-(hydroxyimino)-17β-hydroxy-7α-vinyl-18-méthyl-15α,16α-méthylène-19-nor-17α-prégna-4,20(Z)-diène-21-carboxylique
γ-lactone d'acide (E/Z)-3-(hydroxyimino)-17β-hydroxy-7β-vinyl-18-méthyl-15α,16α-méthylène-19-nor-17α-prégna-4,20(Z)-diène-21-carboxylique
γ-lactone d'acide (E/Z)-3-(hydroxyimino)-17β-hydroxy-7β-vinyl-18-méthyl-15β,16β-méthylène-19-nor-17α-prégna-4,20(Z)-diène-21-carboxylique
γ-lactone d'acide (E/Z)-3-(hydroxyimino)-17β-hydroxy-7β-vinyl-18-méthyl-15β,16β-méthylène-19-nor-17α-prégna-4,20(Z)-diène-21-carboxylique
γ-lactone d'acide (E/Z)-3-(hydroxyimino)-17β-hydroxy-7α-cyclopropyl-18-méthyl-15α,16α-méthylène-19-nor-17α-prégna-4,20(Z)-diène-21-carboxylique
γ-lactone d'acide (E/Z)-3-(hydroxyimino)-17β-hydroxy-7β-cyclopropyl-18-méthyl-15α,16α-méthylène-19-nor-17α-prégna-4,20(Z)-diène-21-carboxylique
γ-lactone d'acide (E/Z)-3-(hydroxyimino)-17β-hydroxy-7α-cyclopropyl-18-méthyl-15β,16β-méthylène-19-nor-17α-prégna-4,20(Z)-diène-21-carboxylique
γ-lactone d'acide (E/Z)-3-(hydroxyimino)-17β-hydroxy-7β-cyclopropyl-18-méthyl-15β,16β-méthylène-19-nor-17α-prégna-4,20(Z)-diène-21-carboxylique
γ-lactone d'acide (E/Z)-3-(hydroxyimino)-17β-hydroxy-6-méthylène-18-méthyl-15a,16a-méthylène-19-nor-17a-prégna-4,20(Z)-diène-21-carboxylique
γ-lactone d'acide (E/Z)-3-(hydroxyimino)-17β-hydroxy-6-méthylène-18-méthyl-15β,16β-méthylène-19-nor-17α-prégna-4,20(Z)-diène-21-carboxylique
γ-lactone d'acide (E/Z)-3-(hydroxyimino)-17β-hydroxy-6α-hydroxyméthylène-18-méthyl-15α,16α-méthylène-19-nor-17α-prégna-4,20(Z)-diène-21-carboxylique
γ-lactone d'acide (E/Z)-3-(hydroxyimino)-17β-hydroxy-6β-hydroxyméthylène-18-méthyl-15α,16α-méthylène-19-nor-17α-prégna-4,20(Z)-diène-21-carboxylique
γ-lactone d'acide (E/Z)-3-(hydroxyimino)-17β-hydroxy-6α-hydroxyméthylène-18-méthyl-15β,16β-méthylène-19-nor-17α-prégna-4,20(Z)-dién-21-carboxylique
γ-lactone d'acide (E/Z)-3-(hydroxyimino)-17β-hydroxy-6β-hydroxyméthylène-18-méthyl-15β,16β-méthylène-19-nor-17α-prégna-4,20(Z)-diène-21-carboxylique
γ-lactone d'acide (E/Z)-3-(hydroxyimino)-6,6-(1,2-éthanediyl)-17β-hydroxy-18-méthyl-15α,16α-méthylène-19-nor-17α-prégna-4,20(Z)-diène-21-carboxylique
γ-lactone d'acide (E/Z)-3-(hydroxyimino)-6,6-(1,2-éthanediyl)-17β-hydroxy-18-méthyl-15β,16β-méthylène-19-nor-17α-prégna-4,20(Z)-diène-21-carboxylique
γ-lactone d'acide (E/Z)-3-(hydroxyimino)-17β-hydroxy-6α,7α;15α,16α-bisméthylène-18-méthyl-19-nor-17α-prégna-4,20(Z)-diène-21-carboxylique
γ-lactone d'acide (E/Z)-3-(hydroxyimino)-17β-hydroxy-6β,7β;15α,16α-bisméthylène-18-méthyl-19-nor-17α-prégna-4,20(Z)-diène-21-carboxylique
γ-lactone d'acide (E/Z)-3-(hydroxyimino)-17β-hydroxy-6α,7α;15β,16α-bisméthylène-18-méthyl-19-nor-17α-prégna-4,20(Z)-diène-21-carboxylique
γ-lactone d'acide (E/Z)-3-(hydroxyimino)-17β-hydroxy-6β,7β;15β,16β-bisméthylène-18-méthyl-19-nor-17α-prégna-4,20(Z)-diène-21-carboxylique
γ-lactone d'acide (E/Z)-3-(hydroxyimino)-17β-hydroxy-18-méthyl-15α,16α-méthylène-19-nor-17α-prégna-4,6,20(Z)-triène-21-carboxylique
γ-lactone d'acide (E/Z)-3-(hydroxyimino)-17β-hydroxy-18-méthyl-15β,16β-méthylène-19-nor-17α-prégna-4,6,20(Z)-triène-21-carboxylique
γ-lactone d'acide 6β,7β;15β,16β-bisméthylène-4-chloro-17β-hydroxy-18-méthyl-19-nor-17α-prégna-4,20(Z)-dién-3-one-21-carboxylique
γ-lactone d'acide 4-chloro-17β-hydroxy-15α,16α-méthylène-19-nor-17α-prégna-4,20(Z)-dién-3-one-21-carboxylique
γ-lactone d'acide 4-chloro-17β-hydroxy-15β,16β-méthylène-19-nor-17α-prégna-4,20(Z)-dién-3-one-21-carboxylique.

11. Dérivé de γ-lactone de 15,16-méthylène-17-hydroxy-19-nor-21-carboxy-stéroïde selon l'une quelconque des revendications précédentes, pour la contraception orale et le traitement de troubles pré-, péri- et postménopausiques.

12. Utilisation du dérivé de γ-lactone de 15,16-méthylène-17-hydroxy-19-nor-21-carboxy-stéroïde selon l'une quelconque des revendications 1 à 11, pour la fabrication d'un médicament destiné à la contraception orale et au traitement de troubles pré-, péri- et postménopausiques.

13. Utilisation selon la revendication 12, **caractérisée en ce que** le médicament a une action progestative, antiminéralocorticoïde et une action androgène neutre à légère.

14. Médicament contenant au moins un dérivé de γ-lactone de 15,16-méthylène-17-hydroxy-19-nor-21-carboxy-stéroïde selon l'une quelconque des revendications 1 à 11 ainsi qu'au moins un additif approprié, pharmaceutiquement acceptable.

15. Médicament selon la revendication 14, contenant en outre au moins un oestrogène.

16. Médicament selon la revendication 15, **caractérisé en ce que** l'oestrogène est l'éthinylestradiol.

17. Médicament selon la revendication 15, **caractérisé en ce que** l'oestrogène naturel est le valérate d'estradiol.

18. Médicament selon la revendication 15, **caractérisé en ce que** l'oestrogène est un oestrogène naturel.

19. Médicament selon la revendication 18, **caractérisé en ce que** l'oestrogène naturel est l'estradiol.

20. Médicament selon la revendication 18, **caractérisé en ce que** l'oestrogène naturel est un oestrogène conjugué.

21. Utilisation du dérivé de γ-lactone de 15,16-méthylène-17-hydroxy-19-nor-21-carboxy-stéroïde selon l'une quelconque des revendications 1 à 9, pour la fabrication d'un médicament destiné à l'usage intra-utérin.

22. Utilisation selon la revendication 21, pour la fabrication d'un système intra-utérin (SIU).

23. Médicament contenant au moins un dérivé de γ-lactone de 15,16-méthylène-17-hydroxy-19-nor-21-carboxy-stéroïde selon l'une quelconque des revendications 1 à 9 ainsi qu'au moins un véhicule pharmaceutiquement acceptable approprié, **caractérisée en ce qu'**il est destiné à l'usage intra-utérin.

24. Médicament selon la revendication 23, **caractérisée en ce qu'**il est un système intra-utérin.
